# EUROPÄISCHE PATENTANMELDUNG

(11) **EP 4 105 865 A1**
(43) Veröffentlichungstag der Anmeldung: **21.12.2022**
(21) Anmeldenummer: 21179652.9
(22) Anmeldetag: 15.06.2021
(51) Int. Cl.: G06Q 10/10, G06Q 50/18, G16H 10/60

(54) **VERFAHREN UND SYSTEM ZUM COMPUTERGESTÜTZTEN NACHVERFOLGEN EINER VON EINER PERSON DURCHZUFÜHRENDEN PROZEDUR**

(71) Anmelder: Pulvis Beteiligungs GmbH, 85250 Altomünster (DE); Gill Beteiligungs GmbH, 71088 Holzgerlingen (DE)
(72) Erfinder: Stauber, Jürgen, 85250 Altomünster (DE); Gill, Bernd, 71088 Holzgerlingen (DE)
(74) Vertreter: Ter Meer Steinmeister & Partner

(57) **Zusammenfassung**

Die Erfindung betrifft ein Verfahren und ein System zum computergestützten Nachverfolgen einer von einer Person durchzuführenden Prozedur, insbesondere einer Fertigungsprozedur oder Testprozedur, beispielsweise für einen Virusnachweistest, bevorzugt zur Durchführung eines Antigentests auf SARS-CoV-2. Die Erfindung betrifft auch ein Verfahren und System zum computergestützten Validieren einer von einer Person durchgeführten und computergestützt nachverfolgten Prozedur. Die Erfindung betrifft zudem ein Verfahren und System zum Einrichten eines Personenprofils zur Verwendung in einem der zuvor genannten Verfahren und System. Die Erfindung betrifft zudem ein Computerprogramm-Produkt.

## Beschreibung

### TECHNISCHES GEBIET DER ERFINDUNG

Die Erfindung bezieht sich auf ein Verfahren und ein System zum computergestützten Nachverfolgen einer von einer Person durchzuführenden Prozedur, insbesondere einer Fertigungsprozedur oder Testprozedur, beispielsweise für einen Virusnachweistest, bevorzugt zur Durchführung eines SARS-CoV-2 Tests. Die Erfindung betrifft auch ein Verfahren und System zum computergestützten Validieren einer von einer Person durchgeführten und computergestützt nachverfolgten Prozedur. Die Erfindung betrifft zudem ein Verfahren und System zum Einrichten eines Personenprofils zur Verwendung in einem der zuvor genannten Verfahren und System. Die Erfindung betrifft zudem ein Computerprogramm-Produkt.

### TECHNISCHER HINTERGRUND

Eine Prozedur kann von einer Person händisch und/oder unter Verwendung von Hilfsmitteln durchgeführt werden. Die Prozedur kann an der Person selbst, einem Tier oder einer Sache durchgeführt werden. Eine ordnungsgemäße Durchführung der Prozedur entscheidet darüber, ob die Prozedur mit dem gewünschten Erfolg durchgeführt wurde. Die Prozedur kann zum Erkennen von Manipulationsversuchen bzw. zum Erkennen von Fehlern während der Durchführung der Prozedur durch eine Validierung, eine Qualitätssicherung und/oder eine Zertifizierung (nachfolgend alles zusammenfassend als Validierung bezeichnet) abgesichert werden.

Dazu kann die durchzuführende Prozedur während der Prozedurdurchführung beispielsweise stichprobenartig von einer weiteren Person (=Supervisor) überprüft werden.

Ein Nachteil, dass die weitere Person physisch anwesend sein muss, um die Prozedur während der Durchführung nachzuverfolgen, kann mittlerweile umgangen werden, indem die weitere Person mittels einer Videokommunikation die Durchführung einer Prozedur auch aus der Ferne beobachten kann und so Manipulationen und Fehler bei der Durchführung der Prozedur erkennen kann und die Prozedur entsprechend validiert oder eben nicht validiert. So ist beispielsweise in der WO 2014 / 114 513 A1 ein derartiges "Fernbeobachten" für ein Identifizieren einer Person beschrieben. Hierbei wird - als Alternative zu einem klassischen Post-Ident-Verfahren - eine Videokommunikation zu einem entfernten Server gestartet und die zu identifizierende Person wird gebeten, sich mittels Identifizierungsdokument vor einem Endgerät mit Kamera zu positionieren. Die Person wird angeleitet, zu bestimmten Zeitpunkten das Identifizierungsdokument in die Kamera zu halten und zu drehen. Die Person wird auch dazu angeleitet, ein Gesicht in die Kamera zu halten und ggf. zu drehen. Die weitere Person vergleicht das Lichtbild des Identifizierungsdokuments mit dem Gesicht der Person vor dem Endgerät und fragt ggf. Daten vom Identifizierungsdokument ab, um die Identität der Person zu verifizieren.

Auch dieses Fernbeobachten löst nicht das Problem, dass die weitere Person zeitlich zum Nachverfolgen einer Prozedur verfügbar sein muss. Die Verfügbarkeit einer derartigen weiteren Person kann eingeschränkt sein, sodass es zu Wartezeiten bei der Validierung der Prozedur kommt. Das Vorhalten einer weiteren Person erhöht somit Personalkosten und schränkt den Validierungsprozess zeitlich stark ein.

Zudem kann diese weitere Person Fehler bei der Durchführung der Prozedur übersehen, sodass die Validierung entweder ungerechtfertigter Weise fehlschlägt (falsch negative Validierung) oder ungerechtfertigter Weise erfolgt (falsch positive Validierung).

### ZUSAMMENFASSUNG DER ERFINDUNG

Der Erfindung liegt die Aufgabe zu Grunde, ein Verfahren und System zum Nachverfolgen einer von einer Person durchzuführenden Prozedur zu schaffen, um Fehler bei der Durchführung der Prozedur zuverlässiger zu vermeiden und bei dem Fehler während der Durchführung zuverlässig erkannt werden, wobei gänzlich auf die Anwesenheit einer weiteren Person verzichtet werden soll. Das Nachverfolgen soll in Echtzeit ermöglicht werden.

Die Aufgabe wird durch die in den unabhängigen Patentansprüchen beschriebenen Merkmale gelöst. Vorteilhafte Ausgestaltungen der Erfindung sind in den abhängigen Ansprüchen angegeben.

Erfindungsgemäß wird ein Verfahren zum computergestützten Nachverfolgen einer von einer Person durchzuführenden Prozedur vorgeschlagen. Das Verfahren umfasst die Schritt: Identifizieren der Person unmittelbar vor einem Durchführen der Prozedur durch die Person mit Erfassen von Biometriedaten der Person durch ein Endgerät; und Vergleichen der erfassten Biometriedaten mit Biometriedaten eines Personendatensatzes durch das Endgerät, wobei der Personendatensatz in einer Speichereinheit des Endgeräts abgelegt ist; Identifizieren eines durch die Prozedur zu verändernden Objekts durch das Endgerät unmittelbar vor der Durchführung der Prozedur; Nachverfolgen einer Position der Person und des zumindest einen Objekts durch das Endgerät mittels Methoden computergestützter Gesten- und/oder Muster- und/oder Bilderkennung während der Durchführung der Prozedur; und Anleiten der Person zum Durchführen der Prozedur durch das Endgerät während der Durchführung der Prozedur.

Die Aufgabe wird zudem durch ein System zum computergestützten Nachverfolgen einer von einer Person durchzuführenden Prozedur gelöst. Das System umfasst eine Identifizierungseinheit eines Endgeräts, die eingerichtet ist zum Identifizieren der Person unmittelbar vor einem Durchführen der Prozedur durch die Person. Die Identifizierungseinheit umfasst eine Erfassungseinheit des Endgeräts, eingerichtet zum Erfassen von Biometriedaten der Person; und eine Auswerteeinheit des Endgeräts, eingerichtet zum Vergleichen der erfassten Biometriedaten mit Biometriedaten eines Personendatensatzes, wobei der Personendatensatz in einer Speichereinheit des Endgeräts abgelegt ist; wobei die Identifizierungseinheit weiter eingerichtet ist zum Identifizieren eines durch die Prozedur zu veränderndem Objekt unmittelbar vor der Durchführung der Prozedur; eine Nachverfolgungseinheit des Endgeräts, eingerichtet zum Nachverfolgen zumindest einer Position der Person und zumindest einer Position des Objekts durch das Endgerät mittels Methoden computergestützter Gesten- und/oder Muster- und/oder Bilderkennung während der Durchführung der Prozedur; und einer Anzeigeeinheit des Endgeräts, eingerichtet zum Anleiten der Person zum Durchführen der Prozedur während der Durchführung der Prozedur.

Alle Verfahrensschritte erfolgen durch ein (das gleiche) Endgerät. Alle Einheiten sind Einheiten des (gleichen) Endgeräts. Ein Endgerät kann ein elektronisches Endgerät umfassend eine Anzeigeeinheit (Display), eine Eingabeeinheit (Touchscreen, Tastatur, Bedienfeld), einer Erfassungseinheit (Kamera, Barcode-Scanner) und eine Auswerteeinheit (Steuereinheit, CPU). Das Endgerät ist beispielsweise ein PC, ein Laptop, ein Smartphone, ein Tablet, ein Terminal und/oder ein sonstiges elektronisches Gerät. Das Endgerät ist dazu eingerichtet, Methoden computergestützter Gesten- und/oder Muster- und/oder Bilderkennung auszuführen.

An das Endgerät kann einer oder mehrere Sensoren angeschlossen sein. Ein oder mehrere Sensoren, insbesondere Beschleunigungs- oder Positionssensoren, können dabei an der Person, beispielsweise einer Extremität, insbesondere einer oder beider Hände, angeordnet sein. Einer oder mehrere Sensoren, insbesondere Beschleunigungs- oder Positionssensoren, können eine oder mehrere Bewegungs- und/oder Positionsinformationen über die zumindest eine Position der Person an das Endgerät bereitstellen. Einer oder mehrere Sensoren können dabei an dem einen oder mehreren Objekten, beispielsweise ein Sensor pro Objekt, angeordnet sein. Dieser eine oder die mehreren Sensoren können eine oder mehrere Bewegungs- und/oder Positionsinformationen über die zumindest eine Position des zumindest einen Objekts an das Endgerät bereitstellen. Das Bereitstellen dieser Signale erfolgt bevorzugt drahtlos, beispielsweise mittels einer Bluetooth-Kommunikation und/oder einer NFC-Kommunikation sein.

An die Person und oder das zumindest eine Objekt kann alternativ oder zusätzlich eine optische Markierung angebracht sein, die es einer Erfassungseinheit erleichtert (ermöglicht) die zumindest eine Position der Person und die zumindest eine Position des zumindest einen Objekts nachzuverfolgen.

Die Auswerteeinheit des Endgeräts ist dazu eingerichtet, mittels der Anzeigeeinheit die Person derart anzuweisen, dass die Person mit einer ausführlichen und schrittweisen Anleitung durch die Testprozedur geleitet wird. Dabei kommen computergestützte Methoden der künstlichen Intelligenz (AI) und der erweiterten Realität (AR) im System zum Einsatz, um die Testprozedur selbst, die Bewegungsabläufe und Objekte ggf. auch Hilfsmittel nachzuverfolgen, um die richtige Durchführung des Tests sicher zu stellen. Zusätzlich ist die Auswerteeinheit des Endgeräts dazu eingerichtet, während der Prozedurdurchführung im Zufallsverfahren Bilder von der Person oder den Objekten ggf. der Hilfsmittel zu erfassen und diese in einer Prozedurdokumentation in einer Speichereinheit des Endgeräts abzuspeichern. Die Person kann zuvor auf den Bildern identifiziert werden.

Jede der Anweisungen kann ein Animationsvideo enthalten. Die Anweisung kann durch "Augmented Reality" unterstützt werden. Während eines Prozessschritts kann der Prozessschritt dokumentiert werden, indem mindestens eine Aufnahme durch eine Kamera des Endgeräts von der Person während des Prozessschritts gemacht wird. Es können auch mehrere, beispielsweise zwei, Aufnahmen durch die Kamera des Endgeräts von der Person während des Prozessschritts gemacht werden. Es kann auch ein oder mehrere Videos durch die Kamera des Endgeräts von der Person während des Prozessschritts gemacht werden. Diese Aufnahmen werden in einer Produktdokumentation im Datensatz in der Speichereinheit des Endgeräts gespeichert.

Jeder Prozessschritt der Testprozedur kann durch das Endgerät nachverfolgt werden. Je nach Prozess wird der Schritt daraufhin geprüft, ob dieser erfolgreich entlang der Anleitung von derselben Testperson umgesetzt wurde.

Die Person kann darüber informiert werden, dass eine Prozedur wegen Verstoß gegen die Prozedurdurchführungsvorschriften oder wegen Abbruch fehlgeschlagen ist.

Die Auswerteeinheit des Endgeräts ist dazu eingerichtet, zu prüfen, ob ein soeben nachverfolgter Prozessschritt ein letzter Prozessschritt der Prozedur ist. Ergibt die Prüfung, dass der soeben nachverfolgte Prozessschritt nicht der letzte Prozessschritt der Prozedur ist, wird ein weiterer (nächster) Prozessschritt über die Anzeigeeinheit des Endgeräts angeleitet.

Bevorzugt ist das Endgerät das Smartphone des Benutzers. Das Smartphone kann einfach transportiert werden und ist ggf. bereits hinreichend personalisiert.

Eine Prozedur kann eine Abfolge von Schritten zum Erzielen eines Ergebnisses sein. Die Abfolge kann fest vorgegeben sein, sodass Schrittreihenfolgen zu beachten sind. Die Abfolge kann alternativ willkürlich sein und nur das Ergebnis der Prozedur entscheidend sein.

Eine Prozedur kann eine Testprozedur, beispielsweise eine humanmedizinische Testprozedur, sein.

Die Prozedur kann eine veterinärmedizinische Testprozedur zur Untersuchung einer Körperflüssigkeit an einem Tier sein. Die veterinärmedizinische Testprozedur wird von einer Person durchgeführt.

Die Testprozedur kann eine Untersuchung einer Körperflüssigkeit sein. Die Testprozedur kann zur Untersuchung einer Körperflüssigkeit einer Person und/oder eines Tieres auf Infektionen, Eigenschaften und/oder Parameter sein. Beispielsweise ist die Testprozedur die Durchführung eines Virusnachweistests, eines Keimtests, eines Bakterientests und/oder eines Pilztests an einer Person oder einem Tier.

Die Körperflüssigkeit kann das Blut, der Abstrich, der Speichel, das Aerosol, die Tränenflüssigkeit, der Schweiß, Sputum, der Urin, und/oder der Stuhl einer Person und/oder eines Tieres sein. Die Körperflüssigkeit kann durch Abnahme von einer Hautoberfläche, beispielsweise von einer Hand oder einer Stirn erfolgen.

Die Prozedur kann dabei das Entnehmen der Körperflüssigkeiten-Probe mit unmittelbar daran anschließendem An- oder Aufbringen einer Körperflüssigkeiten-Probe auf das Objekt sein.

Zwischen dem Entnehmen der Probe und dem An- oder Aufbringen der Probe auf das Objekt können Proben-Vorbereitungs-, Proben-Bearbeitungs- und Proben-Nachbearbeitungsschritte in der Prozedur vorgesehen sein.

Es kann zumindest das eine Objekt oder es können mehrere Objekte bei der Durchführung der Prozedur nachverfolgt werden. Die Nachverfolgung mehrerer Objekte kann gleichzeitig erfolgen.

Das Objekt kann ein Gut, eine Ware, ein Halbzeug, ein Gegenstand, ein Werkstück, ein Bauteil, ein Lebensmittel und/oder ein Rohstoff.

In einer humanmedizinischen oder veterinärmedizinischen Testprozedur kann das Objekt beispielsweise eine Testkassette, ein Teststreifen, ein Messstreifen, ein Reagenz in einem Behälter und/oder ein humanmedizinischer Testsensor (beispielsweise zum Erfassen eines Vitalwertes (Blutdruck, Herzfrequenz, Atemfrequenz, Temperatur) der Person oder des Tieres) sein.

Das Objekt wird durch die Prozedur verändert. Das heißt, das Objekt hat einen ersten Zustand vor der Durchführung der Prozedur und einen - vom ersten Zustand verschiedenen - zweiten Zustand nach der Durchführung der Prozedur. Die Prozedur ist ursächlich für die Überführung des Objekts von dem ersten Zustand in den zweiten Zustand.

Der Zustand des Objekts kann eine Form, eine Kontur, eine Farbe, ein Aggregatzustand und/oder ein Volumen sein.

Die Auswerteeinheit des Endgeräts ist dazu eingerichtet, das Nachfolgen, auch Erfassen, Überwachen, Detektieren und/oder Begleiten, der Prozedur computergestützt bzw. computerbasiert, bevorzugt ausschließlich computergestützt, durchzuführen. Damit erfolgt das Nachfolgen insbesondere ohne weitere Person (ohne menschliches Zutun außer der Durchführung der Prozedur selbst), die Prozedur wird nun nicht mehr von einer weiteren Person begleitet. Damit kann eine Person, die die Prozedur durchführt, mittels eines Endgeräts angeleitet und die korrekte Durchführung der Prozedur durch dieses Verfahren sichergestellt werden. Mit dem erfindungsgemäßen Verfahren können Manipulationen oder Fehler im Ablauf der Prozedur zuverlässig erkannt und durch entsprechende Warnungen bzw. Instruktionen vermieden werden. Mit dem erfindungsgemäßen Verfahren kann bei Erkennen von Manipulationen oder Fehlern im Ablauf der Prozedur ein Prozedurabbruch und die Ungültigkeit der Prozedur zuverlässig erkannt werden.

Eine Person ist ein Mensch. Die Person, nachfolgend auch Benutzer, Testperson, Prozedurdurchführende(r), soll bzw. möchte die Prozedur (selbst) durchführen. Zur Durchführung der Prozedur benötigt die Person zumindest teilweise seinen eigenen Körper, insbesondere seine Körperteile, insbesondere seine Extremitäten. Die Prozedur wird demnach zumindest teilweise "händisch" und damit nicht voll-automatisch durchgeführt. Beispielsweise benötigt der Mensch einen oder beide Arme, eine oder beide Hände, ein oder beide Beine und/oder ein oder beide Füße.

Zudem kann die Prozedur auch an einem Tier durchgeführt werden. Eine Person, die die Prozedur an dem Tier durchführt, ist ein Mensch und soll bzw. möchte die Prozedur (selbst) durchführen. Zur Durchführung der Prozedur benötigt die Person zumindest teilweise den Körper, insbesondere seine Körperteile, insbesondere die Extremitäten, des Tieres. Die Prozedur wird demnach zumindest teilweise "händisch" und damit nicht voll-automatisch durchgeführt.

Beispielsweise benötigt der Mensch ein Körperteil des Tieres, um die Prozedur an dem Tier durchzuführen, beispielsweise um eine Probe zu entnehmen.

Die Prozedur ist personenbezogen, das heißt es ist ein unmittelbares Kriterium des Nachverfolgens der Prozedur, dass eine bestimmte Person die Prozedur durchführt.

Unmittelbar vor der Durchführung der Prozedur, also ohne wesentlichen zeitlichen Abstand zur Durchführung der Prozedur durch die Person (ggf. außer einer Objekt- und/oder Hilfsmittel-Identifizierung und unmittelbaren Prozedur-Vorbereitungsschritten), wird die Person durch das Endgerät identifiziert.

Dieser Schritt zum Identifizieren der Person umfasst das Erfassen von Biometriedaten der Person durch das Endgerät. Die Biometriedaten sind das Ergebnis einer Messung und ggf. Auswertung zum Erkennen der Person basierend auf ihren Verhaltens- und biologischen Eigenschaft. Als biometrische Charakteristika (=Biometriedaten) können u. a. verwendet werden, eine DNA (=mobiler DNA-Test, genetischer Fingerabdruck) der Person, ein Blutbild (Hämogramm) der Person, ein Fingerabdruck (Fingerlinienbild) der Person, ein Gangstil der Person, eine Gesichtsgeometrie der Person, eine Handgeometrie der Person, eine Handlinienstruktur der Person, eine Handvenenstruktur der Person, eine Iris (Regenbogenhaut) der Person bzw. eine Iris-Erkennung der Person, ein Körpergeruch der Person, eine Körpergröße (Anthropometrie) der Person, eine Lippenbewegung der Person, ein Nagelbettmuster der Person, eine Ohrform der Person, eine Retina (Augenhintergrund) der Person, eine Stimme der Person bzw. Sprecherauthentifizierung (ungleich Spracherkennung) der Person bzw. eine Stimmerkennung (Klangfarbe) der Person, eine Unterschrift (statisch, dynamisch, auch Handschrift) der Person, eine Röntgenaufnahme eines Gebisses der Person und/oder ein Zahnabdruck der Person.

Bevorzugt werden einfach erfassbare und technisch einfach (bzw. ausgereift) prüfbare Biometriedaten der Person verwendet, beispielsweise die Gesichtsgeometrie, ein Fingerabdruck und/oder eine Iris- oder Retina-Erkennung.

Dieser Schritt zum Identifizieren der Person umfasst zudem das Vergleichen der erfassten Biometriedaten mit Biometriedaten eines Personendatensatzes durch das Endgerät. Damit werden die erfassten Biometriedaten mit abgespeicherten Biometriedaten abgeglichen und die Identität der Person wird verifiziert.

Der abgespeicherte Personendatensatz (Personenprofil) wird bevorzugt durch ein ebenfalls hier beschriebenes Verfahren und System zum Einrichten eines Personenprofils erhalten und auf dem Endgerät abgespeichert.

Als Ergebnis dieses Identifizieren-Schritts ist die Person, die die Prozedur (unmittelbar nach dem Identifizieren) durchführen möchte, durch das Verfahren identifiziert und anhand des abgespeicherten Personendatensatzes auch für das Verfahren verifiziert.

Ebenfalls unmittelbar vor der Durchführung der Prozedur, also ohne wesentlichen zeitlichen Abstand zur Durchführung der Prozedur durch die Person, wird ein durch die Prozedur zu veränderndem Objekt durch das Endgerät identifiziert. Als Ergebnis dieses Identifizieren-Schritts ist das Objekt, das durch die durchzuführende Prozedur (unmittelbar nach dem Identifizieren) zu verändern ist, durch das Verfahren identifiziert.

Im Nachverfolgen-Schritt des Verfahrens wird zumindest eine Position der Person und zumindest eine Position des Objekts durch das Endgerät mittels Methoden computergestützter Gesten- und/oder Muster- und/oder Bilderkennung und/oder Erfassen/Auswerten von Sensorsignalen der angeschlossenen Sensoren während der Durchführung der Prozedur nachverfolgt.

Eine Gestenerkennung ist die computerbasierte automatische Erkennung von einer oder mehrerer durch die Person ausgeführter Gesten mittels des Endgeräts. Die dazu angewendeten Methoden beschreiben Algorithmen und mathematische Verfahren zur Erkennung der Geste. Dabei kann jede Körperhaltung der Person und jede Körperbewegung der Person prinzipiell eine Geste darstellen. Bevorzugt werden mit der Gestenerkennung Hand- und Kopfgesten der Person erkannt. Eine Geste der Person kann mittels am Körper der Person getragener oder mit der Hand der Person geführter (und an das Endgerät angeschlossener) Sensorik, insbesondere integrierter Beschleunigungs- oder Positionssensoren, durch das Endgerät erkannt werden. Alternativ oder zusätzlich kann eine Geste auch mit (personen-) externer Sensorik erkannt werden, insbesondere einer oder mehrerer Erfassungseinheiten des Endgeräts. Bevorzugt werden Kameras des Endgeräts (oder an das Endgerät anschließbare Kameras) genutzt, um Bilder der Person zu erstellen. Diese kamerabasierte Gestenerkennung verwendet eine 2D- und/oder 3D-Bildanalyse, um die Geste der Person zu erkennen.

Es können zwei Gestenarten unterschieden werden. Bei einer kontinuierlichen Geste (erste Art) besteht eine direkte Verbindung zwischen der durch das Endgerät beobachteten Bewegung und einem Zustand im Endgerät (Beispiel: Zeigen auf den Bildschirm kann einen Zeiger steuern). Eine diskrete Geste (zweite Art) ist Teil einer (beschränkten) Menge von eindeutigen Gesten, wobei mit jeder dieser eindeutigen Gesten in der Regel jeweils eine Aktion verknüpft ist.

Bei der Erkennung einer Geste fließen die Informationen der Sensorik in Algorithmen ein, welche die Rohdaten analysieren, um die Gesten zu erkennen. Dabei können Algorithmen zur Mustererkennung zum Einsatz kommen. Zur Entfernung von Rauschen und zur Datenreduktion erfolgt häufig im ersten Schritt eine Vorbearbeitung der Sensordaten. Anschließend werden Merkmale aus den Sensordaten extrahiert. Diese Merkmale dienen als Eingabe für eine Klassifikation. Hierfür werden bevorzugt Hidden Markov Models, künstliche neuronale Netze oder andere Techniken der künstlichen Intelligenz eingesetzt.

Eine Mustererkennung ist die computerbasierte automatische Erkennung von Regelmäßigkeiten, Wiederholungen, Ähnlichkeiten oder Gesetzmäßigkeiten in einer Datenmenge (Bilder, Video) mittels des Endgeräts. Dabei kann das Erkennen von Mustern, als den Merkmalen, die allen Dingen einer Kategorie gemeinsam sind (und sie deshalb vom Inhalt anderer Kategorien unterscheidet) entscheidend sein. Ein Merkmal für die Mustererkennung kann durch eine automatische Merkmalsynthese oder empirisch durch Einlernen und Erfahrung gewonnen werden. Zur Reduktion der gewonnen Merkmale wird dann geprüft, welche Merkmale für die Klassentrennung relevant sind und welche weggelassen werden können. Abschließend kann eine Klassifizierung der Merkmale in Klassen erfolgen. Es kann eine syntaktische Musterkennung, eine statistische Musterkennung und/oder eine strukturelle Mustererkennung angewendet werden.

Eine Bilderkennung - als Teilgebiet der Mustererkennung und Bildverarbeitung - ist die computerbasierte automatische Segmentierung von Elementen in einem Bild. Dabei kann das zumindest eine Objekt, die Person bzw. darüberhinausgehend ein Ort und/oder eine Aktion in einem erfassten Bild identifiziert werden. Dabei kann tiefgreifendes maschinelles Lernen eingesetzt werden, beispielsweise werden Faltungsprozessoren für künstliche neuronale Netze verwendet. Beispielsweise können Bilderkennungsalgorithmen mithilfe von vergleichenden 3D-Modellen, Auftritten aus verschiedenen Winkeln mithilfe einer Kantenerkennung arbeiten, die auf Trainingsbildern mit maschinellem Lernen trainiert werden können.

Beispielsweise wird für eine computergestützte Gesten- und/oder Muster- und/oder Bilderkennung eine Modellierung mittels Mediapipe basierend auf OpenCV verwendet. Diese Modellierung ist geeignet für eine Körpergestenerkennung- und Nachverfolgung ("Human Pose Detection and Tracking" oder "Hand Tracking", "Holistic Tracking", "Face Detection") einer Person, ein Erzeugen einer Gesichtsgeometrie (Face Mesh), eine Objekterkennung- und Nachverfolgung ("Object Detection and Tracking" oder "Hand Tracking", "Holistic Tracking"). Auf die Mediapipe Veröffentlichungen gemäß Website https://google.github.io/mediapipe/ wird hier explizit Bezug genommen.

Durch den Nachverfolgen-Schritt kann zumindest die Position und/oder auch die Bewegung der identifizierten Person und des identifizierten Objekts rein computergestützt durch das Endgerät erkannt und verfolgt werden. So kann nachverfolgt werden, ob die Person die Prozedur korrekt durchführt, beispielsweise ob das Objekt korrekt verändert wird und/oder ob die Person einen Manipulationsversuch oder einen Fehler vornimmt.

In dem Anleiten-Schritt des Verfahrens wird die Person zum Durchführen der Prozedur durch das Endgerät während der Durchführung der Prozedur angeleitet. Dieses Anleiten der Person kann durch ein Anzeigen sowie akustisches und/oder visuelles Ausgeben von Instruktionen, Warnungen, Hinweisen, Vorführungen, Videos, Bildern, Animationen, Augmented Reality und/oder Beispielen auf einer Anzeigeeinheit (Display, Ausgabeeinheit, Lautsprecher) des Endgeräts erfolgen. Dieses Anleiten kann zu unterschiedlichen Zeitpunkten während der Prozedur erfolgen, beispielsweise zu Beginn einmal oder mehrfach mitten in der Prozedur und/oder zum Ende der Prozedur. Die Anleitung kann die Abfolge der Prozedur abbilden, um die Person gemäß der Schrittabfolge der Prozedur durch die Prozedur zu leiten.

Zum Anleiten kann das Endgerät in Kenntnis von der Prozedur, von einzelnen oder der Gesamtheit der Abfolge der Prozedur, deren Ablauf, deren zu erwartender Zeitdauer, deren zwischen einzelnen oder allen Prozedurschritten zu erwartenden Zeitdauern, und/oder zu einem zu erwartenden Endergebnis und/oder einem oder mehreren Zwischenergebnissen sein.

Auf diese Weise wird einer Person eine Anleitung zur Durchführung gegeben und die Durchführung zudem computergestützt - in Echtzeit - nachverfolgt.

Zum Identifizieren der Person im Erfassenschritt kann ein Gesicht der Person mittels einer Kamera des Endgeräts erfasst werden. Das erfasste Gesicht kann mittels den oder anderen Methoden computergestützter Gesichts- Muster- und/oder Bilderkennung durch eine Auswerteeinheit des Endgeräts analysiert werden, um die (zu erfassenden) Biometriedaten der Person zu erhalten. Das Gesicht als Biometriedaten einer Person zu erfassen kann eine eindeutige Feststellung der Identität einfach ermöglichen, die dafür anzuwendenden Methoden der computergestützten Gesichts- Muster- und/oder Bilderkennung sind zuverlässig und gering rechenintensiv.

Das Identifizieren der Person kann zu einem oder mehreren zufälligen Zeitpunkten während der Durchführung der Prozedur wiederholt werden. Damit kann geprüft werden, ob noch dieselbe Person die Prozedur durchführt oder ob - unerlaubter Weise - die identifizierte Person nicht mehr der die Prozedur durchführende Person ist.

Das zum Identifizieren angewendete Erfassen der Person kann das Aufnehmen eines Bildes und/oder eines oder mehrerer Video(-frames) sein. Diese Aufnahme kann zu Dokumentationszwecken auf dem Endgerät gespeichert werden. Diese Aufnahme kann Teil einer Dokumentation der Durchführung der Prozedur sein.

Das beschriebene Nachverfolgen-Verfahren kann mit einer Fehlermeldung beendet werden, wenn im Vergleichenschritt festgestellt wird, dass die erfassten Biometriedaten nicht mit den Biometriedaten des Personendatensatzes übereinstimmen (fehlende Verifizierung). Auf diese Weise wird sichergestellt, dass eine personengebundene Prozedurdurchführung auch tatsächlich von einer einem Personendatensatz entsprechenden Person durchgeführt wird.

Unmittelbar vor dem Durchführen der Prozedur durch die Person können alle während der Prozedur zu verwendenden Hilfsmittel durch das Endgerät identifiziert werden. Ein Hilfsmittel kann ein beliebiger Gegenstand, ein Messgerät, ein Bauteil, ein Behälter, ein Sensor, ein Werkzeug und/oder der gleichen sein. Das Hilfsmittel hilft (oder ist notwendig), dass die Durchführung der Prozedur erfolgreich durchgeführt werden kann. Der Sensor - hier als Hilfsmittel - einer der zuvor erwähnten Sensoren zum Erfassen der Position oder Bewegung der Person oder des Objekts sein. Der Sensor kann alternativ oder zusätzlich der zuvor beschriebene Testsensor sein.

Jedes verwendete Hilfsmittel kann mittels einer Erfassungseinheit, beispielsweise einer Kamera und/oder der angeschlossenen Sensoren des Endgeräts erfasst werden. Das erfasste Hilfsmittel kann mittels Methoden computergestützter Gesichts- und/oder Muster- und oder Bilderkennung und/oder Erfassen/Auswerten von Sensorsignalen der angeschlossenen Sensoren durch eine Auswerteeinheit des Endgeräts analysiert werden, woraufhin das Hilfsmittel identifiziert werden kann.

Es kann zumindest ein Hilfsmittel oder es können mehrere Hilfsmittel bei der Durchführung der Prozedur nachverfolgt werden.

Das Identifizieren des Objekts kann umfassen: Erfassen eines Objektidentifizierungsdatensatzes durch das Endgerät; und Vergleichen des erfassten Objektidentifizierungsdatensatzes mit einem Objektdatensatz eines Hintergrundsystems. Ein Objektidentifizierungsdatensatz ist ein Datensatz, der im Verfahren eindeutig einem Objekt zugeordnet werden kann und das Objekt eindeutig identifizieren kann. Dieser Objektidentifizierungsdatensatz kann beispielsweise eine Produktkennung, eine Seriennummer, ein Barcode, ein QR-Code, ein RFID-Tag-Code, NFC-Tag-Code, etc. sein, der mittels des Endgeräts ausgelesen werden kann.

In einer Ausgestaltung ist der Objektidentifizierungsdatensatz ein QR-Code auf einer Testkassette (=Testobjekt). Der QR-Code kann kodiert und/oder verschlüsselt sein, sodass das Auslesen keinen Aufschluss über eine Systematik des Objektidentifizierungsdatensatzes ermöglicht.

Zum Identifizieren des Objekts im Erfassenschritt kann ein maschinenlesbarer Datensatz mittels einer Erfassungseinheit, insbesondere einer Kamera des Endgeräts erfasst werden. Ein maschinenlesbarer Datensatz kann eine auf dem Objekt aufgebrachte Seriennummer sein, die mittels der Erfassungseinheit erfasst wird. Die Seriennummer kann eine Fertigungsnummer bei der Herstellung des Objekts sein. Die Seriennummer kann eine vom Objekt unabhängige Seriennummer eines Drittanbieters sein, der das Verfahren und System zum computergestützten Nachverfolgen bereitstellt. Die erfasste Seriennummer kann durch die Auswerteeinheit analysiert werden, beispielsweise kann eine optische Zeichenerkennung (OCR) angewendet werden. Der maschinenlesbare Datensatz kann ein auf dem Objekt aufgebrachter Zeichencode sein, die mittels der Erfassungseinheit erfasst wird. Der Zeichencode, beispielsweise ein Barcode oder ein QR-Code kann durch die Auswerteeinheit analysiert werden. Der maschinenlesbare Datensatz kann ein in einen RFID-Tag oder einen NFC-Tag eincodierter Datensatz sein, der mittels der Erfassungseinheit, insbesondere einem NFC-Leseeinheit oder RFID-Leseeinheit erfasst wird. Der Zeichencode, beispielsweise ein Barcode oder ein QR-Code kann durch die Auswerteeinheit analysiert werden.

Der maschinenlesbare Datensatz des Objekts kann durch Abfragen eines entsprechenden Objektdatensatzes in einem Hintergrundsystem validiert werden. Der maschinenlesbare Datensatz des Objekts ist damit in einem Hintergrundsystem registriert. Der maschinenlesbare Datensatz des Objekts ist damit in einem Hintergrundsystem registriert. Auf diese Weise kann die Echtheit eines Objekts, einen Typ des Objekts, ein Produktionsdatum, eine Hersteller-ID, ein Gültigkeitsdatum, eine Objekteigenschaft und/oder ein Zertifikat abgefragt und/oder nachgewiesen werden. Zur Validierung des Objekts wird der maschinenlesbare Datensatz mit dem Objektdatensatz verglichen und bei Übereinstimmung (und oder auch bei bloßer Existenz im Hintergrundsystem) wird das Objekt als echt validiert und ist für die Prozedur freigegeben.

Nach einer Validierung des maschinenlesbaren Datensatzes kann der Objektdatensatz oder ein Prozeduridentifizierungsdatensatz in der Datenbank des Hintergrundsystems durch Änderung eines Status ungültig werden. Damit wird eine Einmaligkeit des Objekts sichergestellt.

Im Nachverfolgen-Schritt kann die zumindest eine Position und/oder eine Bewegung der Person, bevorzugt auch eine Bewegung eines Körperteils (Extremität) der Person und die zumindest eine Position und/oder eine Bewegung des Objekts mittels einer Kamera als Erfassungseinheit des Endgeräts erfasst werden. Die erfasste Position und Bewegung der Person und des Objekts werden mittels Methoden computergestützter Gesten- und/oder Muster- und/oder Bilderkennung durch eine Auswerteeinheit des Endgeräts analysiert. Damit kann geprüft werden, ob die Person eine der Prozedur entsprechende Bewegung durchführt und/oder ob das Objekt der Prozedur entsprechend verändert wird. Fehl- oder Falschbewegungen der Person oder des Objekts können damit geprüft werden.

Zudem kann ein Bewegungsradius von Person und Objekt nachverfolgt werden. Wird erkannt, dass die Person und/oder das Objekt außerhalb eines vordefinierten Positionsrahmens ist, kann das Verfahren abgebrochen und die Prozedur als ungültig betrachtet werden, beispielsweise beim Verlassen eines von einer Kamera erfassbaren Bereichs oder eines zuvor definierten Bereichs. Die Person wird so gezwungen, in einem vordefinierbaren Bewegungsradius und einem vordefinierbaren Bewegungsmuster zu verbleiben. Auch kann geprüft werden, ob Bewegungsabläufe des Objekts mit der durchzuführenden Prozedur übereinstimmen. Falsche Positionen oder Bewegungen werden so erkannt.

Der zuvor definierte Bereich wird beispielsweise vor dem Durchführen der Prozedur bestimmt und festgelegt. Der von der Kamera erfassbare Bereich wird beispielsweise vor dem Durchführen der Prozedur bestimmt und festgelegt. Die Bereiche können durch darstellbare Hilfslinien auf einer Anzeige des Endgeräts angezeigt werden.

Im Nachverfolgen-Schritt kann eine Objekteigenschaft des Objekts mittels einer Kamera des Endgeräts erfasst werden. Die erfasste Objekteigenschaft des Objekts kann mittels Methoden computergestützter Gesichts- und/oder Muster- und/oder Bilderkennung durch eine Auswerteeinheit des Endgeräts analysiert werden. Die analysierte Objekteigenschaft des Objekts kann mit einer während der Prozedur zu erwartenden Objekteigenschaft, beispielsweise in Echtzeit, verglichen werden. Dazu sollten Zustandsänderungen des Objekts während der Prozedur, also Form- Farb-, und/oder Bedingungsveränderung bzw. Zwischen- und/oder Endergebnisse dem Endgerät bekannt sein.

Das Verfahren kann mit einer Fehlermeldung beendet werden, wenn im Analysierenschritt festgestellt wird, dass die erfasste Position der Person und/oder die erfasste Bewegung der Person und/oder die erfasste Position des Objekts und/oder die erfasste Bewegung des Objekts außerhalb fester Vorgaben der Prozedur sind. Verlässt beispielsweise die Person oder ein Körperteil (eine Hand, beide Hände) einen Erfassungsbereich der Kamera des Endgeräts könnte die Prozedur als ungültig gelten. Verlässt beispielsweise das Objekt einen Erfassungsbereich der Kamera des Endgeräts könnte die Prozedur als ungültig gelten.

Für ein Dokumentieren des Durchführens der Prozedur kann das Durchführen der Prozedur zu einem oder mehreren zufälligen Zeitpunkten während der Durchführung der Prozedur mittels einer Kamera des Endgeräts erfasst und abgespeichert werden. So entsteht eine Prozedurdokumentation in Echtzeit, die im Endgerät in einem Prozeduridentifizierungsdatensatz abgelegt werden kann und jederzeit später abrufbar ist. Eine Validierung der Prozedur kann beispielsweise auch aufgrund dieser Prozedurdokumentation (nach-)geprüft werden.

Die Prozedurdokumentation umfasst beispielsweise eine oder mehrere zufällige Aufnahmen der Person und/oder des Objekts bei der Durchführung der Prozedur und/oder eines Prozedurschritts. Dabei können einzelne Bilder (ein Videoframe) oder mehrere Videoframes (als ein oder mehrere Videos) durch die Kamera des Endgeräts von der Person während des Prozessschritts gemacht werden. Die Prozedurdokumentation umfasst beispielsweise bewusst von der Auswerteeinheit angewiesene Aufnahmen der Person und/oder des Objekts bei der Durchführung der Prozedur und/oder eines Prozedurschritts.

Ein Prozedurergebnis für einen Antigen Test eines Virusnachweistests (als Prozedur) kann das Anzeigen eines Balkens auf einem Teststreifen einer Testkassette (als Objekt) umfassen. Dieser Balken kann von der Erfassungseinheit des Endgeräts erfasst und ausgewertet werden. Das Vorhandensein oder Nichtvorhandensein eines Balkens an einer vordefinierten Position der Testkassette zeigt das Prozedurergebnis an. So kann das Vorhandensein des Balkens bedeuten, dass das Prozedurergebnis "positiv" ist. So kann das Nichtvorhandensein des Balkens bedeuten, dass das Prozedurergebnis "negativ" ist.

Zudem kann ein Kontrollbalken in der Testkassette vorgesehen sein, der als Selbsttest des Teststreifens dient. So kann das Vorhandensein des Kontrollbalkens bedeuten, dass die Testkassette ordnungsgemäß funktioniert. So kann das Nichtvorhandensein des Kontrollbalkens bedeuten, dass die Testkassette fehlerhaft ist.

In einer Ausgestaltung können auf einer Testkassette (Objekt) mehrere Balken zur Darstellung mehrerer Prozedurergebnisse vorhanden sein. Dabei werden mit einer Körperflüssigkeit (der Person oder des Tieres) eine Mehrzahl von Tests parallel oder zeitlich versetzt durchgeführt. Jedes Prozedurergebnis wird mit einem eigenen Balken auf der Testkassette angezeigt. Das Vorhandensein oder Nichtvorhandensein eines Balkens an einer für dieses Prozedurergebnis vordefinierten Position der Testkassette zeigt das Prozedurergebnis an. So können verschiedene Tests durch Entnahme/Abnahme von nur einer Probe an einer Körperflüssigkeit durchgeführt werden.

In einer Ausgestaltung können auf einer Testkassette (Objekt) mehrere Balken zur Darstellung eines Prozedurergebnisses vorhanden sein. So kann über die Anzahl der Balken eine Konzentration der Infektiosität der Körperflüssigkeit abgebildet werden, beispielsweise als prozentuale Angabe, beispielsweise ein Balken für gering, zwei Balken für mittel, drei Balken für stark (Konzentration bezogen auf eine Referenzkonzentration der Infektiosität).

Für das Anleiten können Anleitungshinweise auf einer Anzeige des Endgeräts angezeigt werden, wobei ein Anleitungshinweis in Abhängigkeit der Durchführung der Prozedur und/oder in Abhängigkeit des Nachverfolgens von Positionen und Bewegungen der Person und des Objekts durch das Endgerät während der Durchführung der Prozedur erfolgen können.

Erfindungsgemäß ist zudem ein Verfahren zum computergestützten Validieren einer von einer Person durchgeführten und computergestützt nachverfolgten Prozedur vorgesehen. Das Verfahren umfasst dabei die folgenden Schritte: Anfordern einer Validierung eines Prozedurergebnisses durch eine Personeneingabe an einem Endgerät; Erzeugen und Versenden einer Validierungsanfrage bei einem Hintergrundsystem umfassend einen Streuwert über den Personendatensatz und einen Prozeduridentifizierungsdatensatz des zu validierenden Prozedurergebnisses; Empfangen im Endgerät eines Validierungstoken vom Hintergrundsystem; Anzeigen eines maschinenlesbaren Code auf Basis des Validierungstoken und Anzeigen des Prozedurergebnisses auf einer Anzeigeeinheit des Endgeräts; Prüfen des maschinenlesbaren Codes durch eine Prüfinstanz unter Prüfung des Validierungstokens; Anzeigen eines Validierungsergebnisses bezüglich des Prozedurergebnisses zusammen mit dem Prozedurergebnis und dem Streuwert über den Personendatensatz und bevorzugt Prozedurinformationen auf einer Anzeigeeinheit der Prüfinstanz.

Dieses Validierungsverfahren wird auf eine von der Person durchgeführte Prozedur angewendet. Die Durchführung der Prozedur hat dabei ein Prozedurergebnis erzeugt. Das Prozedurergebnis kann das veränderte Objekt sein (der zweite Zustand des Objekts nach der Durchführung der Prozedur).

Die Person wählt dazu das zu validierende Prozedurergebnis durch Eingabe am Endgerät aus. Daraufhin erzeugt das Endgerät eine Validierungsanfrage. Die Person, die die Validierungsanfrage wählt und deren Prozessergebnis gezeigt werden soll, wird bevorzugt nochmal von dem Endgerät identifiziert. Das ist insbesondere notwendig, wenn die Prüfinstanz keine Person, sondern ein Automat (Barcode-Scanner) ist.

Diese Validierungsanfrage umfasst einen Streuwert des Personendatensatzes, der in der Speichereinheit des Endgeräts abgelegt ist. Der Personendatensatz ist eineindeutig der Person zugeordnet, die die Prozedur durchgeführt hat. Der Streuwert (=Hash) der Validierungsanfrage kann mittels einer kryptografischen Streuwert-Funktion, die von der Auswerteeinheit des Endgeräts auf den Personendatensatz angewendet werden kann, erhalten werden. Eine Hashfunktion oder Streuwertfunktion ist eine Abbildung, die den Personendatensatz auf die Streuwerte (=Hashwerte) abbildet. Eine Hashfunktion ist nicht injektiv.

Diese Validierungsanfrage umfasst auch eine Prozeduridentifizierungsdatensatz, kurz Prozedur-ID. Diese Prozedur-ID ist im Verfahren und System eindeutig einer noch-durchzuführenden und/oder bereits durchgeführten Prozedur zugeordnet. Damit wird das Verfahren fälschungssicherer und manipulationssicherer.

Das Hintergrundsystem empfängt die Anfrage und erzeugt einen Validierungstoken. Der Validierungstoken wird an das Endgerät zurückgesendet.

Auf einer Anzeigeeinheit des Endgeräts wird nun ein vom Endgerät erzeugter maschinenlesbarer Code angezeigt. Die Validierungsbestätigung wurde unter Verwendung des Validierungstokens erstellt. Zudem wird das Prozedurergebnis angezeigt. Dabei kann der maschinenlesbare Code (als eine Art unbestätigte Validierungsbestätigung) mit dem Validierungstoken und einem Link zum Validierungsserver zusammen mit dem Prozedurergebnis auf der Anzeigeeinheit des Endgeräts angezeigt. werden.

Zum Validieren des Prozedurergebnisses liest eine Prüfinstanz, beispielsweise eine Erfassungseinheit (Kamera, QR-Scanner, Barcode-Scanner) eines Endgeräts oder Automaten, den maschinenlesbaren Code (die unbestätigte Validierungsbestätigung) aus und prüft den Validierungstoken auf Gültigkeit. Eine Prüfinstanz, beispielsweise ein Auditor oder ein Automat mit Scanfunktion, liest also den maschinenlesbaren Code aus. Mit dem Einlesen stellt die Prüfinstanz eine Validierungsanfrage unter Prüfens des Validierungstokens. Ist der Validierungstoken gültig, so können vom Hintergrundsystem das Prozedurergebnis, ein Streuwert über den Personendatensatz und bevorzugt Prozedurinformationen abgerufen werden.

Auf einer Anzeigeeinheit der Prüfinstanz wird ein positives Validierungsergebnis zusammen mit dem Prozedurergebnis und Daten aus dem Hintergrundsystem angezeigt, wenn der Validierungstoken dekodiert, zeitlich gültig und überprüft (im Sinne von validiert, also wurde die Prozedur von dieser Person durchgeführt) werden konnte.

Auf einer Anzeigeeinheit der Prüfinstanz wird ein negatives Validierungsergebnis zusammen mit dem Prozedurergebnis und Daten aus dem Hintergrundsystem angezeigt, wenn der Validierungstoken nicht dekodiert werden konnte oder wenn der Token ungültig ist (Zeitablauf) oder wenn die Überprüfung nicht erfolgreich ist.

Die computergestützt nachverfolgte Prozedur ist bevorzugt eine, die mit dem Verfahren (bzw. System) zum computergestützten Nachverfolgen einer von einer Person durchzuführenden Prozedur gemäß der vorhergehenden Art nachverfolgt wurde. Damit schließt sich das Validierungsverfahren an das Nachverfolge-Verfahren an, womit sowohl eine Nachverfolgung der Prozedur als auch eine Validierung der Prozedur computergestützt, manipulationssicher und ohne weitere Person durchführbar ist.

Die Prüfinstanz kann eine Person oder ein System (Endgerät, Automat, Barcodescanner mit Auswerteeinheit) sein. Das System kann eine Einlasskontrolle zu einem Gebäude (Restaurant, Konzertsaal, Theater, etc.) oder einer Anlage sein (Stadion, Konzertfläche, Restaurant, etc.) sein. Das System kann überprüfen, ob der Validierungstoken erfolgreich validiert werden konnte und kann die identifizierte Person bei validiertem Prozedurergebnis passieren.

In einer bevorzugten Ausgestaltung umfasst die Validierungsanfrage einen Prozedurdokumentationsdatensatz, der bei der Durchführung der Prozedur mit erstellt wurde.

Der Prozedurdokumentationsdatensatz umfasst beispielsweise zufällige Aufnahmen der Person und/oder des Objekts bei der Durchführung. Der Prozedurdokumentationsdatensatz umfasst beispielsweise bewusst angewiesene Aufnahmen der Person und/oder des Objekts bei der Durchführung. Dabei können einzelne Bilder (ein Videoframe) oder mehrere Videoframes (als ein oder mehrere Videos) durch die Kamera des Endgeräts von der Person während des Prozessschritts gemacht werden.

Bevorzugt kann der Validierungstoken eine Gültigkeitszeitdauer für das Validierungsergebnis bezüglich des Prozedurergebnisses definieren. Die Gültigkeitsdauer kann beispielsweise 1 Minute, 2 Minuten, 5 Minuten, 10 Minuten, 15 Minuten oder 20 Minuten betragen. Die Gültigkeitsdauer wird in den Validierungstoken kodiert. Ist die Gültigkeitsdauer abgelaufen, ist das Validierungsergebnis negativ. Die Person muss dann eine neue Validierung vornehmen.

Die Anzahl der Validierung gemäß dem o.g. Verfahren ist nicht beschränkt. So kann ein Prozedurergebnis beliebig oft validiert werden. Eine bereits durchgeführte Validierung wird im Hintergrundsystem protokolliert und kann kein zweites Mal aufgerufen werden.

Das Validierungsergebnis kann Prozedurinformationen umfassen. Diese Prozedurinformationen sind Informationen des Hintergrundsystems, die die Prüfinstanz bei Prüfen des maschinenlesbaren Codes vom Hintergrundsystem erhalten hat. Damit erhält die Prüfinstanz Zusatzinformationen über die Prozedurdurchführung. Die Prozedurinformation kann ein Prozedurdatum und/oder eine Prozedurzeit gemäß Hintergrundsystem sein. Ist die Prozedur beispielsweise eine humanmedizinische Testprozedur, dann kann angegeben werden, wann die Prozedur durchgeführt wurde. Beispielsweise kann so bei einem Virusnachweistest darauf geschlossen werden, ob das Prozedurergebnis überhaupt noch aussagekräftig ist. Beispielsweise informieren die Prozedurinformationen (zumindest indirekt) darüber, dass die Testprozedur ungültig ist und eine neue Testprozedur durchzuführen ist.

Der Prozeduridentifizierungsdatensatz wird bevorzugt von dem Endgerät erzeugt und dem Hintergrundsystem mitgeteilt. Alternativ wird der Prozeduridentifizierungsdatensatz vom Hintergrundsystem generiert und dem Endgerät bei Prozedurbeginn mitgeteilt.

Der maschinenlesbare Code ist bevorzugt ein QR-Code. Die Prüfinstanz umfasst eine Erfassungseinheit, um den maschinenlesbaren Code einzulesen.

Das Prozedurergebnis kann vom Hintergrundsystem abgefragt werden.

Die Erfindung umfasst zudem ein computergestütztes Validieren einer von einer Person durchgeführten Prozedur. Das System umfasst eine Eingabeeinheit eines Endgeräts, eingerichtet zum Empfangen einer Personeneingabe zum Anfordern einer Validierung eines Prozedurergebnisses. Das System umfasst zudem eine Steuereinheit, eingerichtet zum Erzeugen eines Streuwerts über einen, in einer Speichereinheit des Endgeräts abgelegten Personendatensatz; Erzeugen einer Validierungsanfrage umfassend den Streuwert und einen Prozeduridentifizierungsdatensatz des zu validierenden Prozedurergebnisses; Senden der Validierungsanfrage an ein Hintergrundsystem; und Empfangen im Endgerät eines Validierungstoken des Hintergrundsystems. Das System umfasst zudem eine Anzeigeeinheit des Endgeräts, eingerichtet zum Anzeigen eines maschinenlesbaren Codes mit dem Validierungstoken und dem Prozedurergebnis. Das System umfasst zudem eine Prüfinstanz, eingerichtet zum Prüfen des maschinenlesbaren Codes unter Prüfung des Validierungstoken und Anzeigen eines Validierungsergebnisses bezüglich des Prozedurergebnisses zusammen mit dem Prozedurergebnis und dem Streuwert über den Personendatensatz und bevorzugt Prozedurinformationen.

Erfindungsgemäß ist zudem ein Verfahren zum Einrichten eines Personenprofils zur Verwendung in einem Verfahren der vorhergehenden Art vorgesehen. Das Verfahren umfasst das Erfassen von Daten eines Identifizierungsdokuments einer Person mittels eines Endgeräts; das Auswerten der Daten des erfassten Identifizierungsdokuments durch eine Auswerteeinheit des Endgeräts zum Erhalten eines Personendatensatzes umfassend Biometriedaten und personenbezogene Daten der Person; und das Abspeichern des erhaltenen Personendatensatzes als Personenprofil in einer Speichereinheit des Endgeräts.

Die personenbezogenen Daten sind insbesondere ein Vorname, ein Nachname, ein Geburtsdatum, ein Geburtsort, eine Anschrift (Adresse), einen akademischen Grad und/oder eine Dokumentennummer.

Ein Identifizierungsdokument ist beispielsweise ein Personalausweis, ein Pass, ein Führerschein und/oder ein Firmenausweis. Das Identifizierungsdokument umfasst insbesondere ein Lichtbild der Person.

Im Erfassenschritt kann ein Lichtbild des Identifizierungsdokuments mittels einer Kamera des Endgeräts erfasst werden. Im Auswertenschritt kann das erfasste Lichtbild mittels Methoden computergestützter Gesichts- und/oder Muster und/oder Bilderkennung durch die Auswerteeinheit des Endgeräts analysiert werden, um die Biometriedaten des Personendatensatzes zu erhalten.

Im Erfassenschritt können personenbezogene Daten des Identifizierungsdokuments mittels einer Kamera des Endgeräts erfasst werden. Im Auswertenschritt können die erfassten personenbezogenen Daten mittels optischer Zeichenerkennung durch die Auswerteeinheit des Endgeräts analysiert werden, um die personenbezogenen Daten des Personendatensatzes zu erhalten.

Im Erfassenschritt kann ein elektronischer Datenspeicher des Identifizierungsdokuments ausgelesen werden. Im Auswertenschritt können die ausgelesenen Daten ausgewertet werden, um den Personendatensatzes zu erhalten. Optional wird das Auslesen der Daten nur nach vorheriger PIN-Verifikation durch Eingabe einer PIN durch die Person ermöglicht.

Im Abspeichernschritt kann der erhaltene Personendatensatz kryptografisch gesichert in einer Speichereinheit des Endgeräts abgespeichert werden.

Erfindungsgemäß ist zudem ein System zum Einrichten eines Personenprofils umfassend ein Endgerät und ein Identifizierungsdokument vorgesehen. Das System umfasst: eine Erfassungseinheit des Endgeräts, eingerichtet zum Erfassen von Daten eines Identifizierungsdokuments einer Person; eine Auswerteeinheit des Endgeräts, eingerichtet zum Auswerten der Daten des erfassten Identifizierungsdokuments zum Erhalten eines Personendatensatzes umfassend Biometriedaten und personenbezogene Daten der Person; und eine Speichereinheit des Endgeräts, eingerichtet zum Abspeichern des erhaltenen Personendatensatzes als Personenprofil.

Die Erfassungseinheit des Endgeräts kann eine Kamera sein, wobei die Kamera eingerichtet ist, ein Lichtbild des Identifizierungsdokuments zu erfassen. Die Auswerteeinheit des Endgeräts kann ein Gesichtserkennungsmodul umfasst, wobei das Gesichtserkennungsmodul eingerichtet ist, das erfasste Lichtbild mittels Methoden computergestützter Gesichts- und/oder Muster- und/oder Bilderkennung zu analysieren, um die Biometriedaten des Personendatensatzes zu erhalten.

Die Erfassungseinheit des Endgeräts kann eine Kamera sein, wobei die Kamera eingerichtet ist, personenbezogene Daten des Identifizierungsdokuments zu erfassen. Die Auswerteeinheit des Endgeräts kann ein Zeichenerkennungsmodul umfasst, wobei das Zeichenerkennungsmodul eingerichtet ist, die erfassten personenbezogenen Daten mittels Methoden computergestützter Gesichts- und/oder Muster- und/oder Bilderkennung zu analysieren, um die personenbezogenen Daten des Personendatensatzes zu erhalten.

Das Identifizierungsdokument kann einen elektronischen Datenspeicher umfassen, wobei die Erfassungseinheit des Endgeräts eine Leseeinheit ist, wobei die Leseeinheit eingerichtet ist, den elektronischen Datenspeicher des Identifizierungsdokuments auszulesen; und wobei die Auswerteeinheit des Endgeräts eingerichtet ist, die ausgelesenen Daten auszuwerten, um den Personendatensatzes zu erhalten.

Die Auswerteeinheit kann ein Verschlüsselungsmodul umfasst, wobei das Verschlüsselungsmodul eingerichtet ist, den erhaltenen Personendatensatz kryptografisch abzusichern. Die Speichereinheit des Endgeräts kann eingerichtet sein, den kryptografisch abgesicherten Personendatensatz abzuspeichern.

Die Kommunikation zwischen Endgerät und Hintergrundsystem erfolgt über TCP/IP Protokoll. Das Hintergrundsystem umfasst eine Serverarchitektur, auf der eine Datenbank läuft. Die Datenbank verwaltet einen Objektdatensatz der eineindeutig einem Objekt zugeordnet wird, eine Prozedur-ID für jede Prozedur und einen Personendatensatz für jede Person.

### KURZE BESCHREIBUNG DER FIGUREN

Nachfolgend wird anhand von Figuren die Erfindung bzw. weitere Ausführungsformen und Vorteile der Erfindung näher erläutert, wobei die Figuren lediglich Ausführungsbeispiele der Erfindung beschreiben. Gleiche Bestandteile in den Figuren werden mit gleichen Bezugszeichen versehen. Die Figuren sind nicht als maßstabsgetreu anzusehen, es können einzelne Elemente der
Figuren übertrieben groß bzw. übertrieben vereinfacht dargestellt sein. Gestrichelt dargestellte Elemente sind optional.
Fig. 1 zeigt ein Ablaufdiagram eines Verfahrens 100;
Fig. 2 zeigt ein System 1000;
Fig. 3 zeigt ein Ablaufdiagrams eines Verfahrens 200;
Fig. 4 zeigt ein System 2000;
Fig. 5 zeigt ein Verfahrens 300;
Fig. 6 zeigt ein System 3000;
Fig. 7a-f zeigt jeweils ein Anzeigeelement eines Endgeräts;
Fig. 8a-k zeigt ein Ablaufdiagram der Verfahren 100, 200, 300;
Fig. 9a-f zeigt beispielhafte Strukturen für verschiedene Datensätze der Verfahren 100, 200, 300; und
Fig. 10a-b zeigt zwei alternative Ausgestaltungen des Verfahrensschritts 2044a

### DETAILLIERTE BESCHREIBUNG VON AUSFÜHRUNGSBEISPIELEN

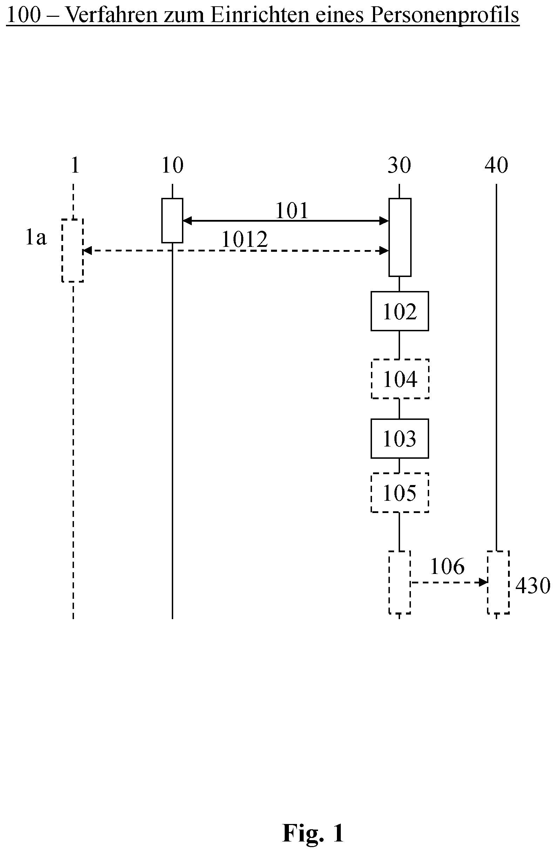

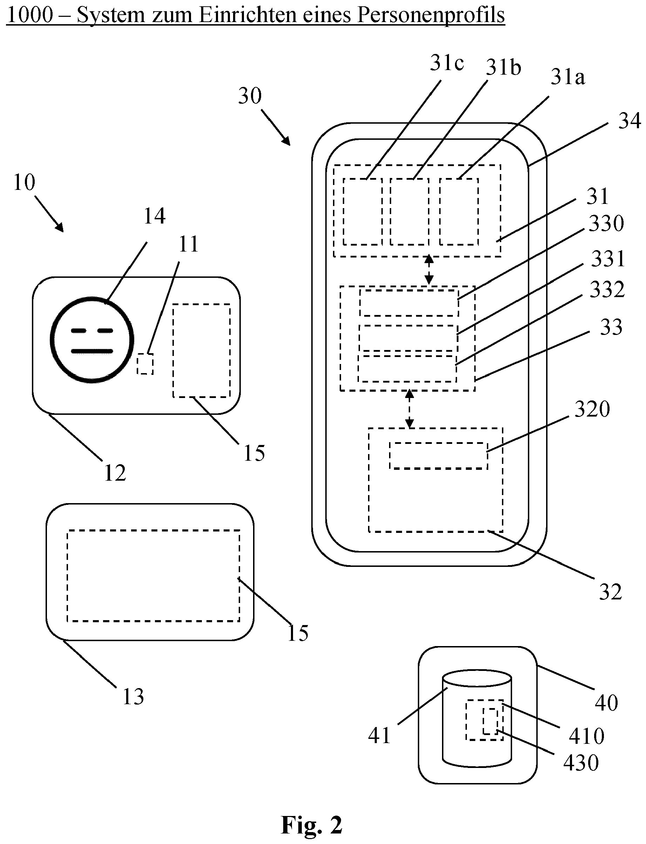

Fig. 1 zeigt ein Ablaufdiagram eines Verfahrens 100 zum Einrichten eines Personendatensatzes (=Personenprofils). Gestrichelt dargestellte Elemente sind optional. Fig. 2 zeigt ein Systems 1000 zum Einrichten eines Personenprofils umfassend ein Endgerät 30 und ein Identifizierungsdokument 10. Bei der Beschreibung der Fig. 1 wird auch auf das System 1000 der Fig. 2 eingegangen.

Das Verfahren 100 der Fig. 1 umfasst in einem ersten Schritt 101 das Erfassen von Daten 11, 14, 15 eines Identifizierungsdokuments 30 der Person 1 mittels eines Endgeräts 30. Das Endgerät 30 weist dazu eine Erfassungseinheit 31 auf.

Das Identifizierungsdokument 30 ist beispielsweise ein Personalausweis, ein Reisepass, ein Führerschein und/oder ein Firmenausweis derjenigen Person 1, deren Personendatensatz 320 im Endgerät 30 eingerichtet werden soll.

Das Identifizierungsdokument 10 kann ein Lichtbild 14 der Person 1 aufweisen. Das Lichtbild 14 kann auf einer Vorderseite 12 des Identifizierungsdokuments 10 angeordnet sein.

Das Identifizierungsdokument 10 kann personenbezogene Daten 15 der Person 1 aufweisen. Die personenbezogenen Daten 15 können auf einer Vorderseite 12 und/oder auf einer Rückseite 13 des Identifizierungsdokuments 10 angeordnet sein. Personenbezogene Daten 15 einer Person 1 können eine oder mehrere der folgenden Angaben umfassen: Vorname der Person 1, Nachname der Person 1, Geburtsname der Person 1, Geburtstag der Person 1, Geburtsort der Person 1, Anschrift der Person 1, Staatsangehörigkeit der Person 1, händische Unterschrift der Person, Augenfarbe der Person 1, Körpergröße der Person 1, Identifizierungsdokumentennummer des Identifizierungsdokuments 10, Identifizierungsdokumententyp des Identifizierungsdokuments 10, Ausstellungsdatum des Identifizierungsdokuments 10, Ausstellungsbehörde des Identifizierungsdokuments 10, Gültigkeitsdatum des Identifizierungsdokuments 10.

Das Identifizierungsdokument 10 kann einen Datenspeicher 11 mit Biometriedaten der Person 1 und personenbezogene Daten 15 der Person 1 aufweisen. Die Biometriedaten sind Merkmale der Person 1, die so eindeutig sind, dass diese Person 1 anhand dieser Merkmale identifiziert werden kann. Die Biometriedaten auf einem deutschen Personalausweis als Beispiel eines Identifizierungsdokuments 10 einer Person 1 sind die Gesichtsgeometrie des Gesichts 1a der Person 1, die Fingerabdrücke der Person 1 und/oder die händische Unterschrift der Person 1.

Weitere mögliche Biometriedaten einer Person 1 (auch mit Blick auf die Verfahren 200 und 300, die nachfolgend beschrieben werden) können sein: eine DNA (=mobiler DNA-Test, genetischer Fingerabdruck) der Person 1, ein Blutbild (Hämogramm) der Person 1, ein Fingerabdruck (Fingerlinienbild) der Person 1, ein Gangstil der Person 1, eine Handgeometrie der Person 1, eine Handlinienstruktur der Person1, eine Handvenenstruktur der Person1, eine Iris (Regenbogenhaut) der Person 1 bzw. eine Iris-Erkennung der Person 1, ein Körpergeruch der Person 1, eine Körpergröße (Anthropometrie) der Person 1, eine Lippenbewegung der Person 1, ein Nagelbettmuster der Person 1, eine Ohrform der Person 1, eine Retina (Augenhintergrund) der Person 1, eine Stimme der Person 1 bzw. Sprecherauthentifizierung (ungleich Spracherkennung) der Person 1 bzw. eine Stimmerkennung (Klangfarbe) der Person 1, eine Unterschrift (statisch, dynamisch, auch Handschrift) der Person 1, eine Röntgenaufnahme eines Gebisses der Person 1 und/oder ein Zahnabdruck der Person 1.

Die Erfassungseinheit kann eine Kamera 31a sein. Die Kamera 31a kann eine Frontkamera des Endgeräts 30 sein, die auf der Seite einer Anzeigeeinheit 34 (Display) des Endgeräts 30 angeordnet ist. Die Kamera 31a kann eine Rückkamera des Endgeräts 30 sein, die auf einer, der Seite der Anzeigeeinheit 34 (Display) des Endgeräts 30 gegenüberliegenden Seite angeordnet ist.

Die Erfassungseinheit 31 kann eine Leseeinheit 31b sein. Die Leseeinheit 31b ist eingerichtet, elektronische Daten aus dem Datenspeicher 11 des Identifizierungsdokuments 30 (oder auch eines Objekts 20 gemäß der Verfahren 200, 300) zu empfangen. Das Empfangen erfolgt beispielsweise mittels Nahfeldkommunikationsmitteln (NFC, RFID) als Leseeinheit 31b. Zum Auslesen der Daten des Datenspeichers 11 des Identifizierungsdokuments 10 kann eine PIN-Abfrage notwendig sein.

Die Erfassungseinheit 31 kann ein Code-Scanner 31c sein. Der Code-Scanner 31c ist eingerichtet, maschinenlesbare Datensätze, wie Barcodes, QR-Codes, etc. vom Identifizierungsdokument 30 (oder auch eines Objekts 20 gemäß der Verfahren 200, 300) zu erfassen.

In einem Folgeschritt der Fig. 1 erfolgt ein Auswerten 102 der Daten 11, 14, 15 des erfassten Identifizierungsdokuments 30 durch eine Auswerteeinheit 33 des Endgeräts 30 zum Erhalten eines Personendatensatzes 320 umfassend Biometriedaten und personenbezogene Daten der Person 1. Die Auswerteeinheit 33 ist beispielsweise eine CPU des Endgeräts 30. Die Auswerteeinheit ist dazu eingerichtet, Methoden computergestützter Gesichts-, Muster-, Zeichen- und/oder Bilderkennung auszuführen. Mit der Ausführung werden die erfassten Daten 11, 14, 15 des Identifizierungsdokuments 10 ausgewertet.

So kann das erfasste Lichtbild 14 des Identifizierungsdokuments 10 mittels eines Gesichtserkennungsmoduls 330 ausgewertet werden. Das Gesichtserkennungsmodul 330 analysiert dazu das erfasste Lichtbild 14 und extrahiert Gesichtsmerkmalsdaten der Person 1 als Biometriedaten des Personendatensatzes 320.

So können die erfassten personenbezogenen Daten 15 des Identifizierungsdokuments 10 mittels einer optischen Zeichenerkennung (englisch optical character recognition, OCR) einer Zeichenerkennungsmoduls 331 analysiert werden. Das Zeichenerkennungsmoduls 331 analysiert dazu die erfassten personenbezogenen Daten 15 und extrahiert diese als personenbezogene Daten des Personendatensatzes 320.

So können die erfassten Daten aus dem Datenspeicher 11 des Identifizierungsdokuments 10 mittels NFC-Kommunikation im Endgerät 30 erhalten werden und diese erfassten Daten umfassen sowohl die Biometriedaten als auch personenbezogene Daten des Personendatensatzes 320.

In einem optionalen Schritt 104 wird der erhaltene Personendatensatz 320 mittels Verschlüsselungsmodul 332 der Auswerteeinheit 33 des Endgeräts 30 kryptografisch verschlüsselt.

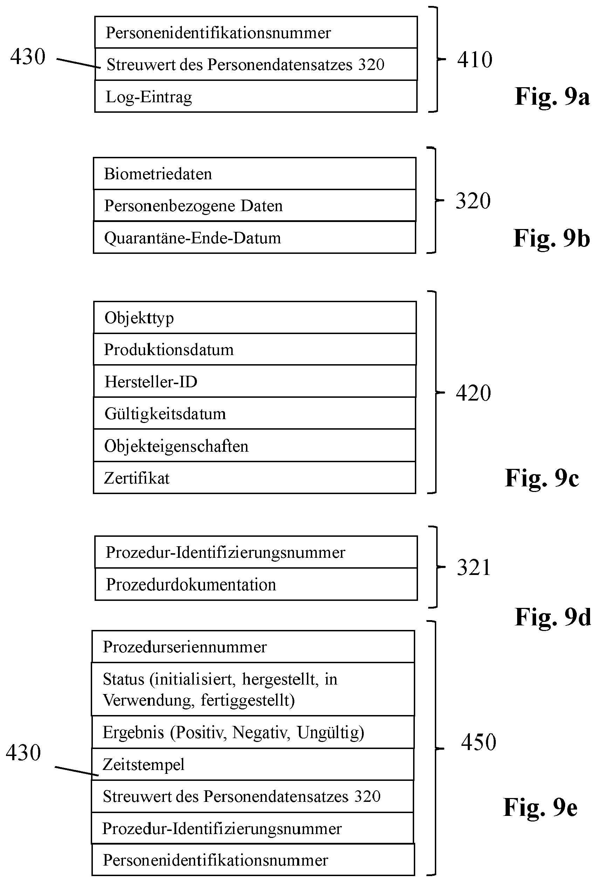

In einem Abspeichern-Schritt 103 wird der erhaltenen Personendatensatzes 320 als Personenprofil in einer Speichereinheit 32 des Endgeräts 30 abgespeichert, ggf. kryptografisch gesichert, wenn Schritt 104 ebenfalls ausgeführt wurde. Eine Struktur eines beispielhaften Personendatensatz 320 ist in Fig. 9b gezeigt.

Im optionalen Schritt 105 wird mittels der Auswerteeinheit 33, beispielsweise ebenfalls mit dem Modul 332 der Auswerteeinheit 33 des Endgeräts 30, ein Streuwert (=Hashwert) des Personendatensatzes 320 erzeugt.

Im optionalen Schritt 106 wird der im Schritt 105 erzeugt Streuwert des Personendatensatzes 320 vom Endgerät 3 an ein Hintergrundsystem 40 gesendet. Im Hintergrundsystem 40 kann dieser Streuwert 430 in einer Datenbank 41 abgelegt werden. Der Streuwert 430 kann dabei ein Teil eines anonymisierten Personendatensatzes 410 in dem Hintergrundsystem 40 sein. Eine beispielhafte Struktur eines anonymisierten Personendatensatzes 410 ist in Fig. 9a gezeigt.

Der anonymisierte Personendatensatzes 410 (="Remote_Testee") kann neben dem Streuwert 430 auch einen Log-Eintrag umfassen, mit dem Prozedurereignisse, insbesondere fehlgeschlagene Objektidentifizierungen, mit Zeitstempel abgelegt werden können.

Der anonymisierte Personendatensatzes 410 (="Remote_Testee") kann zudem eine Personenidentifikationsnummer umfassen, mit der die Person 1 eindeutig am Hintergrundsystem 40 referenziert werden kann.

Alternativ wird mit dem Schritt 106 der gesamte Personendatensatz 320 (Biometriedaten und personenbezogene Daten) in dem Hintergrundsystem 40 abgelegt. Dazu ist das Hintergrundsystem 40 bevorzugt eine vertrauenswürdige Instanz (trusted server), der zur Verwaltung derartiger Personendatensätze 320 eine entsprechende Datenintegrität und Manipulationssicherheit aufweist.

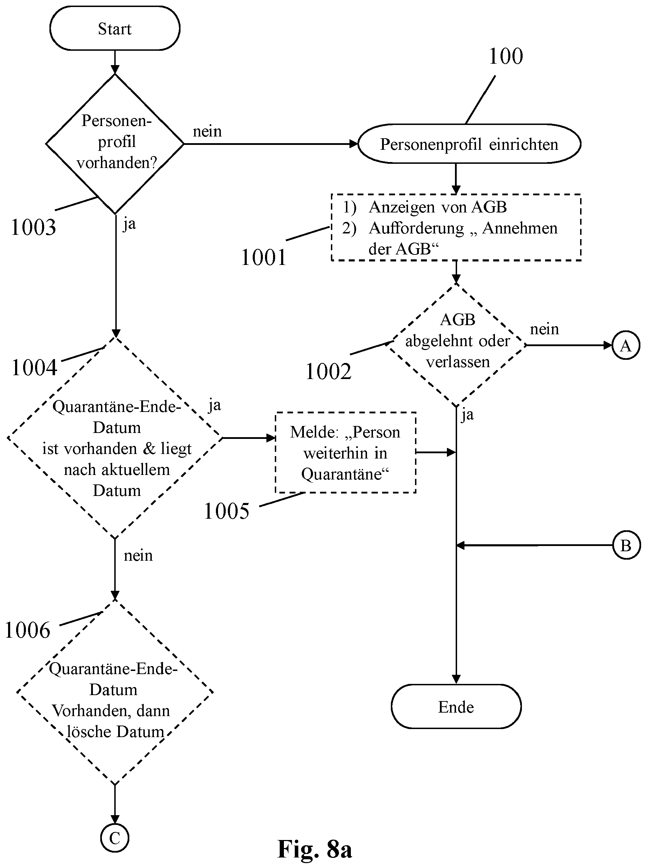

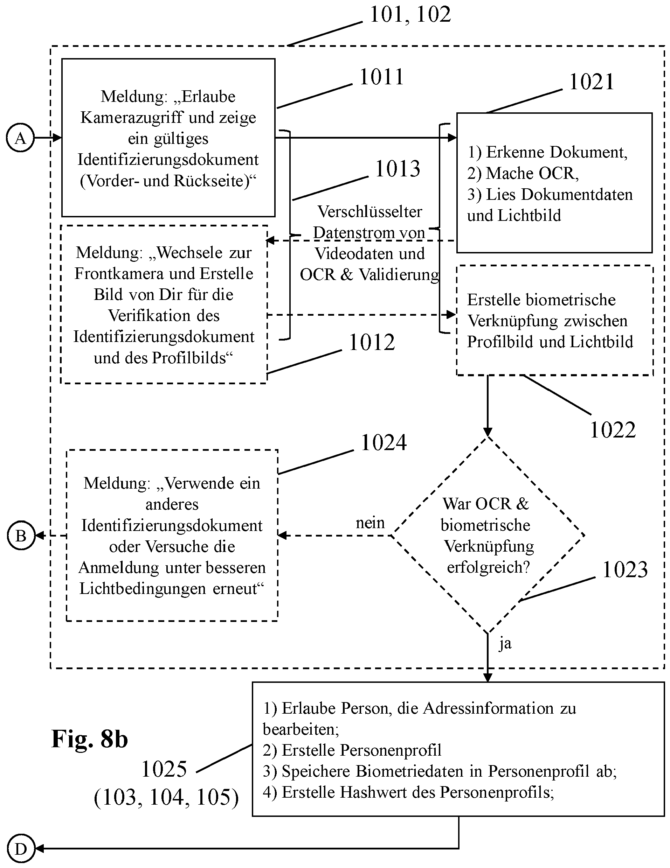

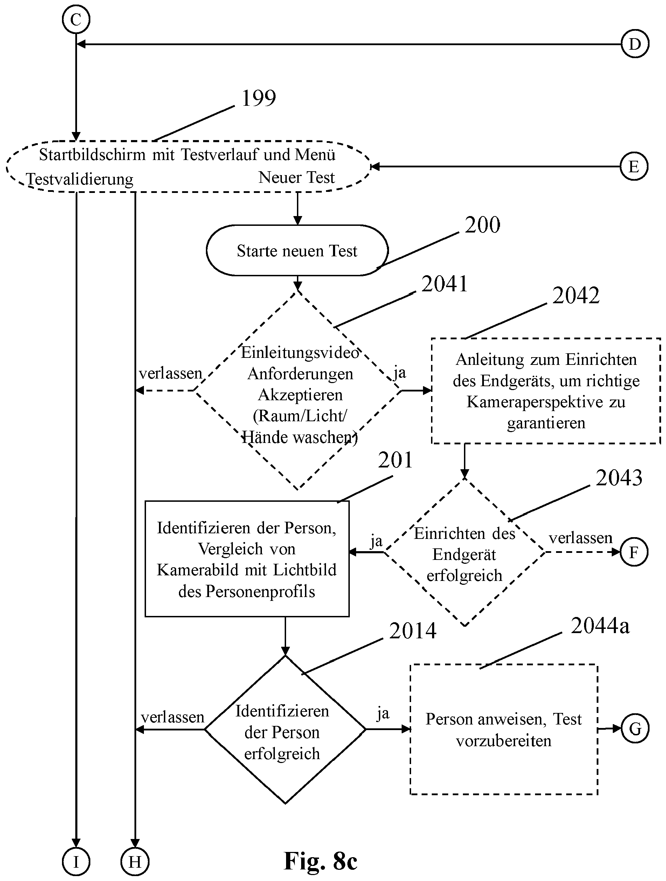

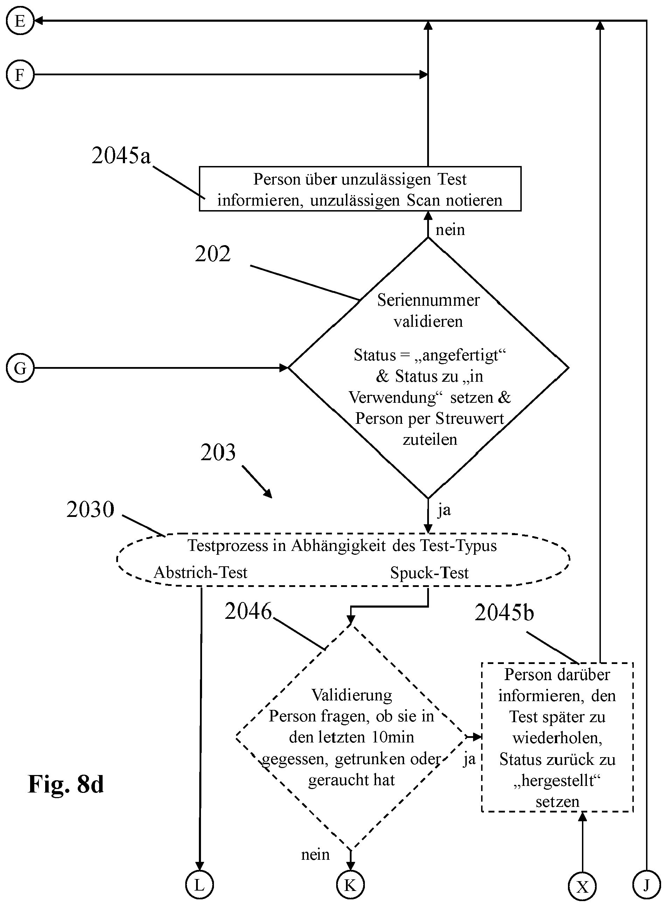

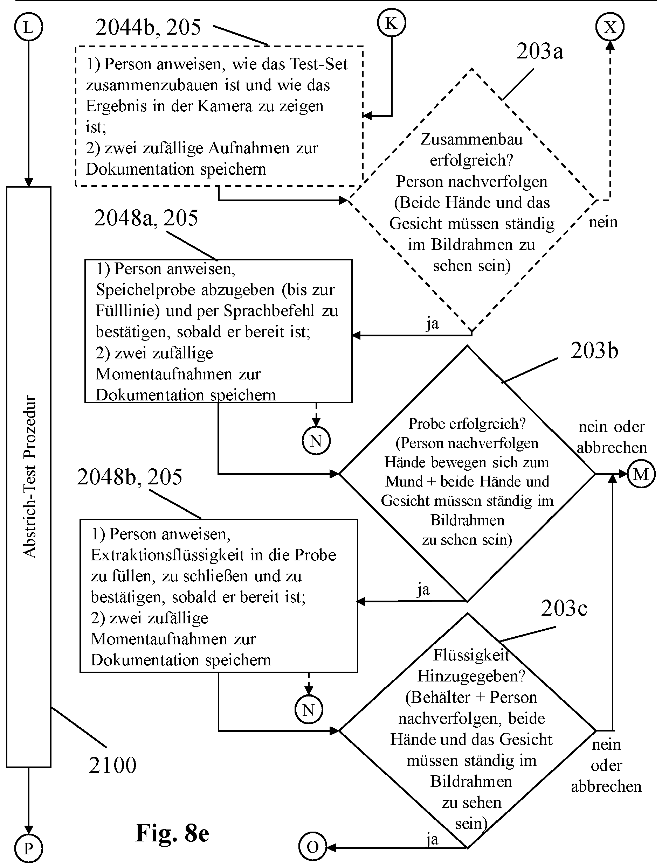

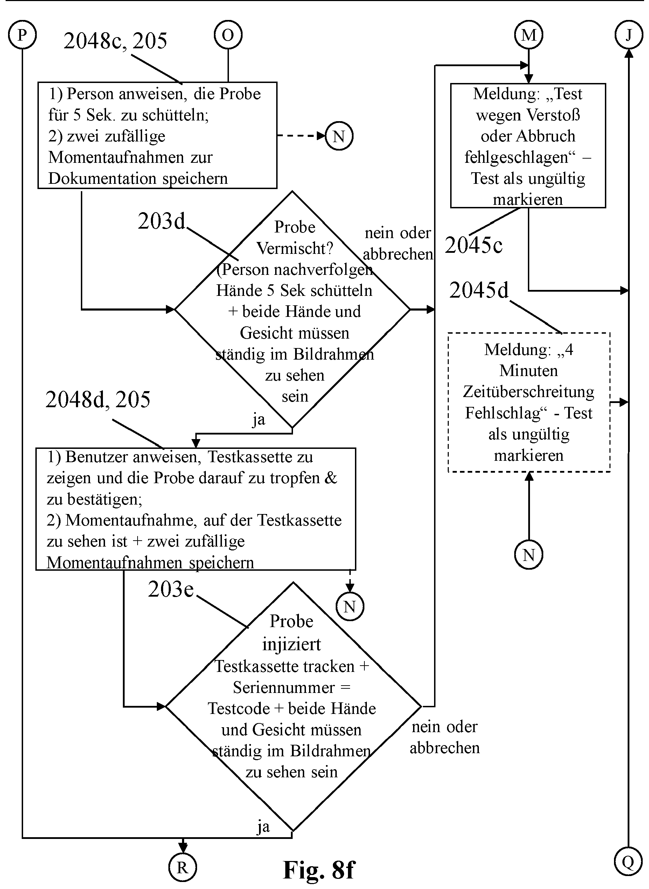

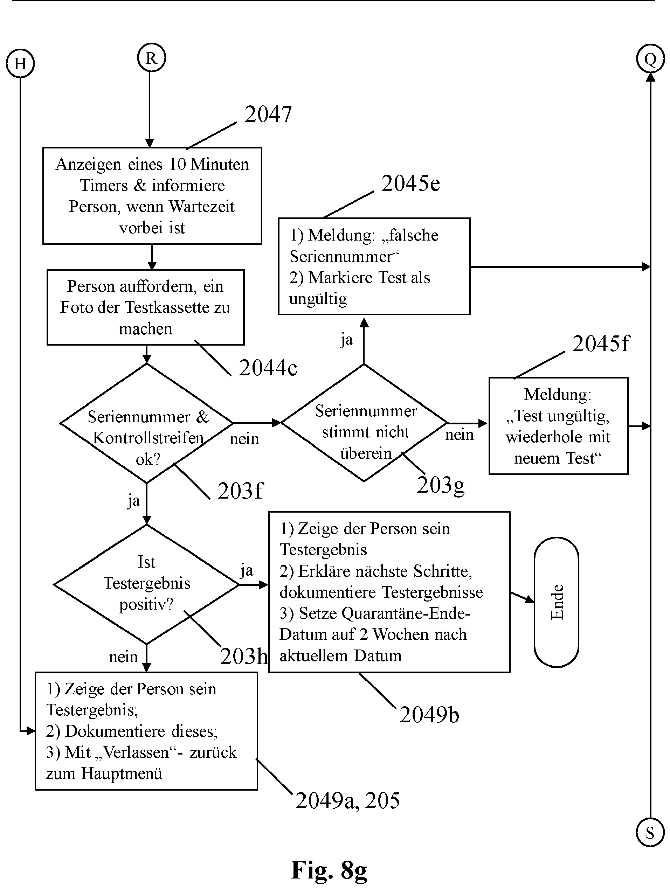

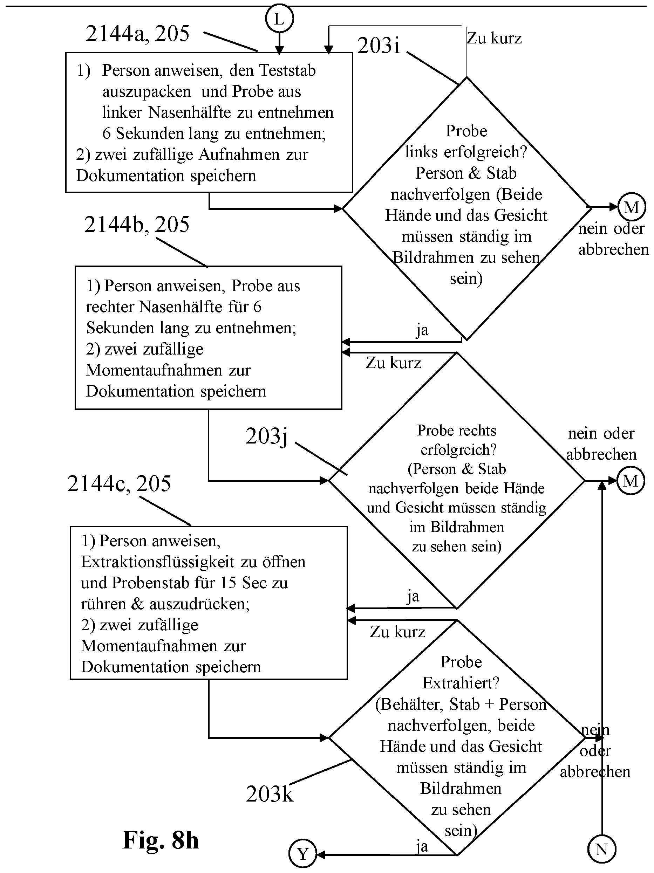

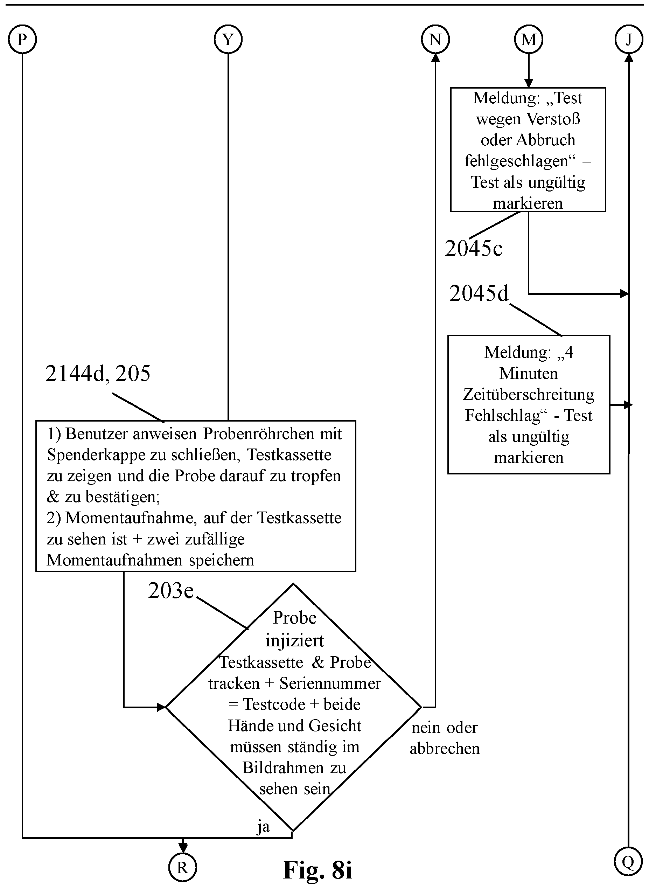

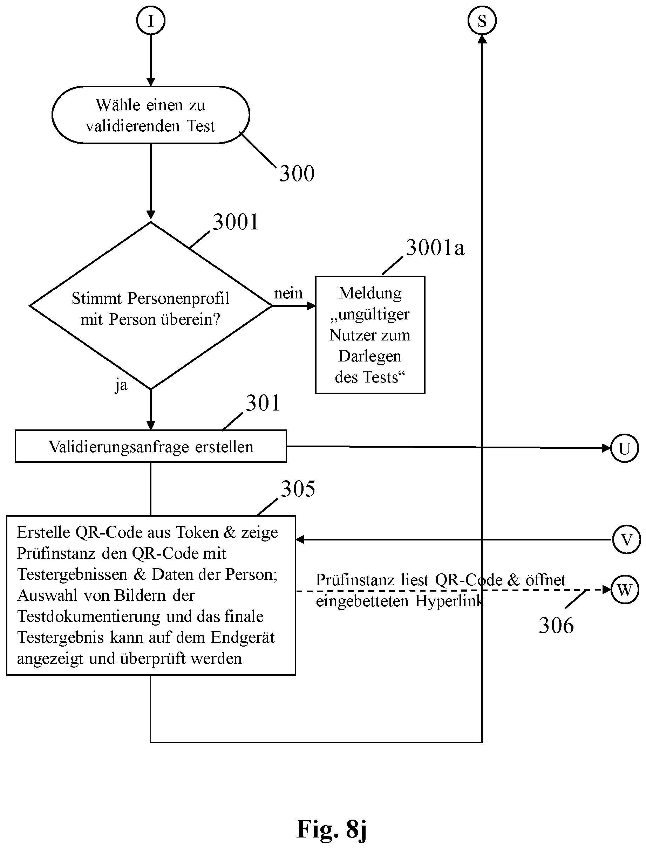

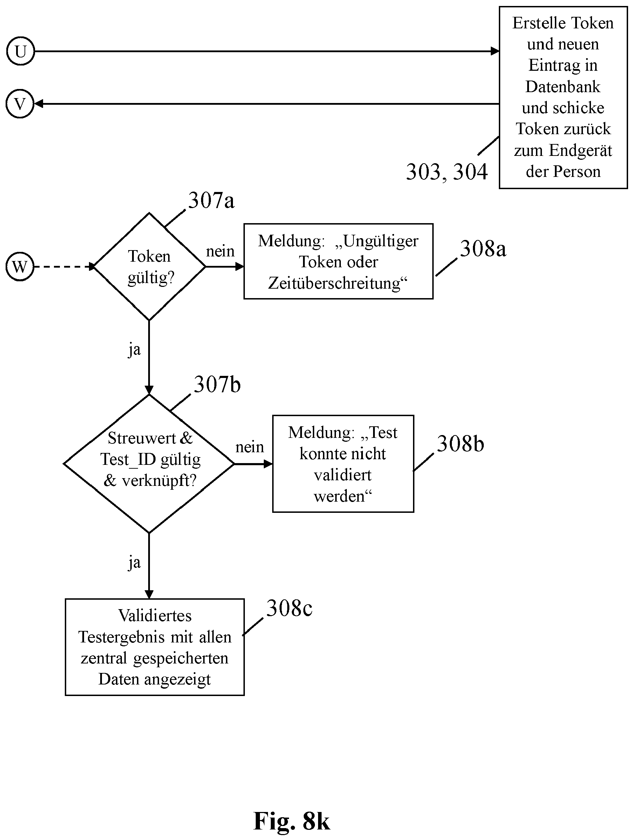

In Fig. 1 gestrichelt dargestellt ist ein optionaler Schritt 1012, der im Ablaufdiagramm der Fig. 8 näher erläutert wird. Im optionalen Schritt 1012 kann nach dem Erfassen der Daten 11, 14, 15 des Identifizierungsdokuments 10 das Endgerät 30 die Person 1 dazu auffordern, ein Bild des Gesichts 1a der Person 1 von der Erfassungseinheit 31 des Endgeräts 30 zu erfassen. Dieses erfasste Gesicht 1a der Person 1 wird vom Endgerät 30 in den Schritten 1022, 1023 der Fig. 8b mit den erhaltenen Biometriedaten aus dem Identifizierungsdokument 10 verglichen, um zu verifizieren, dass die Person 1 auf dem Identifizierungsdokument 10 auch die Person 1 vor dem Endgerät 30 ist. Dieser zusätzliche Verifizierenschritt (Schritte 1012, 1022, 1023 der Fig. 8e) erhöht die Sicherheit am Verfahren 100, 200 und 300 und dient der gesicherten Identitätsfeststellung der Person 1.

Bevorzugt werden die Biometriedaten der Person 1 mit dem Personendatensatz 320 verknüpft anstelle der Biometriedaten des Lichtbildes, um Lichtverhältnisse und ein geändertes optisches Erscheinen der Person 1 gegenüber dem Lichtbild 1 im Verfahren abzubilden und das Erkennen der Person 1 zu erleichtern.

Das Verfahren 100 zum Einrichten eines Personendatensatzes 320 für eine durchzuführende Testprozedur eines Antigentest auf SARS-CoV-2 ist in den Figuren 8a und 8b dargestellt und wird später beschrieben.

Fig. 2 zeigt das Systems 1000 zum Einrichten eines Personenprofils 320 umfassend das Endgerät 30 und das Identifizierungsdokument 10. Auf die Ausführungen zur Fig. 1 wird verwiesen, um unnötige Wiederholungen zu vermeiden.

Das Endgerät 30 kann ein Smartphone der Person 1 sein. Dieses Endgerät 30 wird von der Person 1 ständig mitgeführt und kann zur Prozedurnachverfolgung (Verfahren 200) und Prozedurvalidierung (Verfahren 300) flexibel verwendet werden. Smartphones haben mittlerweile ausreichend Prozessor- und Rechenleistungen in ihren Auswerteeinheiten 33, bspw. einer CPU, einer GPU oder zusätzlicher Signalprozessoren, dass damit eine computergestützte Gesichts- und/oder Muster- und/oder Bilderkennung basierend auf Methoden der künstlichen Intelligenz und des maschinellen Lernens zuverlässig ausgeführt werden können.

Das Endgerät 30 der Systeme 1000, 2000 und 3000 sollte ein Endgerät 30 sein, das ein Betriebssystem ab iOS^{™} der Version 12 und/oder ab Android ^{™} der Version 9 (Pie) oder jünger unterstützt.

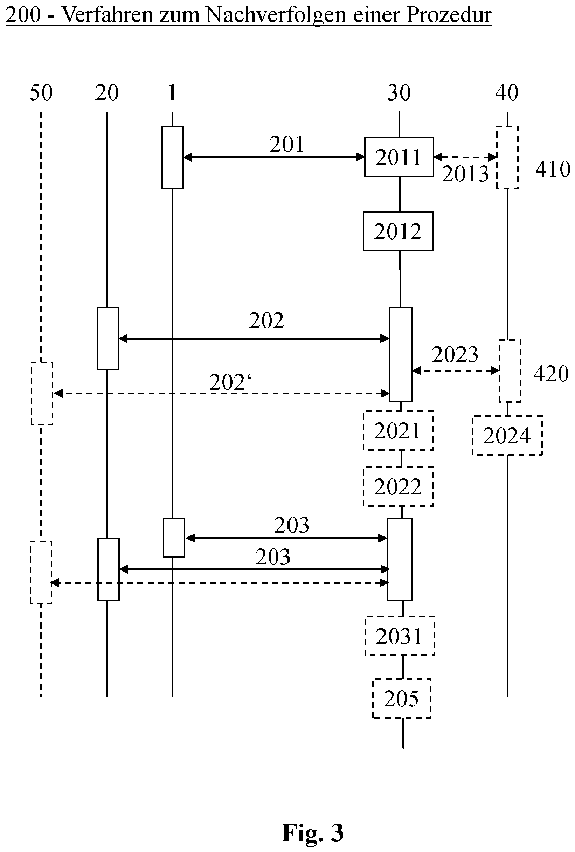

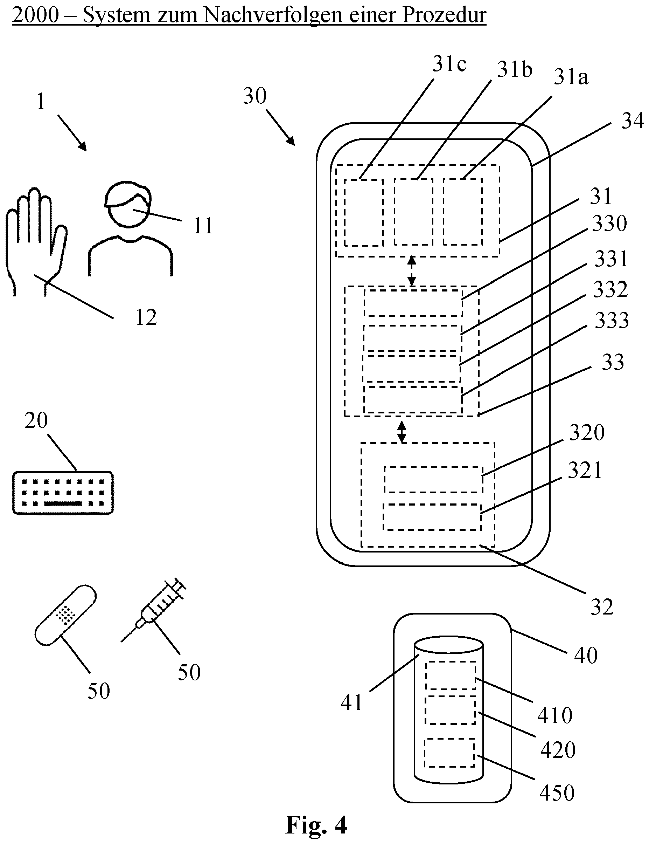

Fig. 3 zeigt ein Ablaufdiagrams eines Verfahrens 200 zum computergestützten Nachverfolgen einer von einer Person 1 durchzuführenden Prozedur. Gestrichelt dargestellte Elemente sind optional. Fig. 4 zeigt ein Systems 2000 zum computergestützten Nachverfolgen einer von der Person 1 durchzuführenden Prozedur. Bei der Beschreibung der Fig. 3 wird auch auf das System 2000 der Fig. 3 eingegangen. Das Verfahren 200 kann sich dem Verfahren 100 der Fig. 1 direkt anschließen. Ein beispielhaftes Anschließen ist in Fig. 8c gezeigt.

In einem Schritt 201 wird durch eine Identifizierungseinheit des Endgeräts 30 unmittelbar vor einem Durchführen der Prozedur durch die Person 1 die Person 1 identifiziert. Dazu werden in einem Erfassenschritt 2011 die Biometriedaten der Person 1 durch das Endgerät 30 erfasst. Dieses Erfassen im Schritt 2011 kann auf Basis der gleichen Technologien erfolgen, wie das Erfassen der Biometriedaten vom Identifizierungsdokument 30, nur mit dem Unterschied, dass nun nicht das Identifizierungsdokument 30, sondern die Person 1 (selbst) von der Erfassungseinheit 31 des Endgeräts 30 erfasst wird.

Zum Identifizieren 201 werden die im Schritt 2011 erfassten Biometriedaten mit den Biometriedaten des Personendatensatzes 320 durch die Identifizierungseinheit des Endgeräts 30 im Schritt 2012 verglichen. Diese Biometriedaten des Personendatensatzes 320 können aus der Speichereinheit 32 des Endgeräts 30 abgerufen werden. Diese Biometriedaten des Personendatensatzes 320 wurden bevorzugt mit dem Verfahren 100 der Fig. 1 (System 1000 der Fig. 2) erhalten. Dazu ist ggf. eine Entschlüsselung des Personendatensatzes 320 notwendig (wenn eine Verschlüsselung im Schritt 104 des Verfahrens 100 erfolgt ist).

Alternativ (oder zusätzlich) können diese Biometriedaten von einem Hintergrundsystem 40 im Schritt 2013 abgerufen werden (bspw. wenn sie dort im Schritt 106 hinversendet wurden). Das Hintergrundsystem 40 könnte eine vertrauliche Instanz zur Verwaltung von Personendatensätzen 320 sein. Das Abrufen des Hintergrundsystems 40 ist weniger bevorzugt, da dadurch Biometriedaten außerhalb des Endgeräts 30 vorliegen müssen. Ein Vergleich 2012 mit lokal gespeicherten Biometriedaten ist aus Sicherheitsgründen bevorzugt.

Zum Identifizieren 202 der Person 1 kann im Erfassenschritt 2011 ein Gesicht 1a der Person 1 mittels der Erfassungseinheit 31, insbesondere einer Kamera 31a des Endgeräts 30 erfasst werden. Das erfasste Gesicht 1a kann mittels Methoden computergestützter Gesichtserkennung durch die Auswerteeinheit 33 des Endgeräts 30 analysiert werden, um die Biometriedaten der Person 1 zu erhalten.

Das Identifizieren 202 der Person 1 kann zu einem oder mehreren zufälligen Zeitpunkten während der Durchführung der Prozedur wiederholt werden. Damit wird sichergestellt, dass die identifizierte (und verifizierte) Person 1 während der gesamten Prozedurdauer die Prozedur durchführt.

In einem Folgeschritt 202 wird zumindest eines durch die Prozedur zu veränderndes Objekt 20 durch das Endgerät 30 unmittelbar vor der Durchführung der Prozedur identifiziert. Das Objekt 20 ist insbesondere ein Gegenstand, der durch die Prozedur von einem ersten Zustand in einen zweiten Zustand verändert wird. Das Objekt 20 kann ein Testobjekt, beispielsweise eine Testkassette eines Virusnachweistests sein. Der erste Zustand dieser Testkassette könnte dann sein, dass die Testkassette einen unverbrauchten Teststreifen hat. Der zweite Zustand (nach der Prozedur) könnte dann sein, dass die Testkassette - durch Applizieren einer Abstrich-, Speichel- oder Spuckprobe - einen verbrauchten Teststreifen hat. Der verbrauchte Teststreifen hat im Vergleich zum unverbrauchten Teststreifen zumindest eine sichtbare Kontrolllinie (=Kontrollbalken), während der unverbrauchte Teststreifen keine sichtbare Kontrolllinie hat.

Zum Identifizieren 202 des Objekts 20 kann eine Abfrage 2023 eines Objektidentifizierungsdatensatzes 420 oder eines Prozedurdatensatzes 45) aus dem (oder einem anderen) Hintergrundsystem 40 erfolgen.

Durch diese Abfrage 2023 kann ein in Fig. 9c dargestellter Objektidentifizierungsdatensatzes 420 abgefragt werden. Der Objektidentifizierungsdatensatzes 420 kann beispielsweise einen Objekttyp umfassen. Dies ist sinnvoll, wenn eine Prozedur mit verschiedenen Typen von Objekten 20 durchgeführt werden kann. Mit dem Objekttyp kann dann das Anleiten und Dokumentieren entsprechend angepasst werden. Der Objektidentifizierungsdatensatzes 420 kann beispielsweise eine Herstellerangabe, ein Produktionsdatum, ein Gültigkeitsdatum, eine Objekteigenschaft und/oder ein Zertifikat umfassen. Damit kann das Objekt 20 bestmöglich im Verfahren 200 identifiziert werden und nützliche Informationen für die Durchführung der Prozedur bereitgestellt werden.

Durch diese Abfrage 2023 kann alternativ oder zusätzlich ein in Fig. 9e dargestellter Prozedurdatensatzes 450 abgefragt werden. Der Prozedurdatensatzes 450 kann beispielsweise eine Prozedurseriennummer umfassen.

Der Prozedurdatensatzes 450 kann beispielsweise einen Status des Objekts 20 umfassen. Ein Status des Objekts 20 kann "initialisiert" sein, beispielsweise wenn das Objekt im Verfahren 200 angelegt wurde. Ein Status des Objekts 20 kann "hergestellt" sein, beispielsweise wenn das Objekt für das Verfahren 200 hergestellt wurde. Ein Status des Objekts 20 kann "in Verwendung" sein, beispielsweise wenn das Objekt im Verfahren 200 verwendet wird. Ein Status des Objekts 20 kann "fertiggestellt" sein, beispielsweise wenn das Objekt im Verfahren 200 fertiggestellt wurde.

Der Prozedurdatensatz 450 kann ein Prozedurergebnis umfassen. Das Prozedurergebnis kann "positiv", "negativ" oder "ungültig" sein. Ein ungültiges Prozedurergebnis wird ausgegeben, wenn die Prozedur falsch angewendet wurde oder Person 1 und/oder Objekt 20 nicht prozedurkonform identifiziert/nachverfolgt werden konnten. Ein positives Prozedurergebnis kann ausgegeben werden, wenn eine Prozedur fehlerfrei und manipulationsfrei durchlaufen wurde. In Anlehnung an einen Virusnachweistest als Prozedur, ist ein positives Testergebnis der Nachweis von Viren durch die Testkassette. In Anlehnung an einen Virusnachweistest als Prozedur, ist ein negatives Testergebnis der fehlende Nachweis von Viren durch die Testkassette.

Der Prozedurdatensatz 450 kann einen Zeitstempel, beispielsweise eine Zeit- oder Datumsangabe umfassen. Der Prozedurdatensatz 450 kann den Streuwert 430 umfassen. Der Prozedurdatensatz 450 kann eine Prozedur-Identifizierungsnummer umfassen. Der Prozedurdatensatz 450 kann eine Personenidentifizierungsnummer umfassen.

Das Identifizieren 202 des Objekts 20 kann das Erfassen 2021 eines Objektidentifizierungsdatensatzes 21 des Objekts durch das Endgerät 30 und ein Vergleichen 2022 des erfassten Objektidentifizierungsdatensatzes 21 mit dem Objektidentifizierungsdatensatz 420 des Hintergrundsystems 40 umfassen. Damit wird eine Manipulationssicherheit erhöht. Der Objektidentifizierungsdatensatz 21 ist beispielsweise ein maschinenlesbarer Datensatz, beispielsweise ein QR-Code 210, ein Barcode oder ein RFID/NFC-Tag. Der maschinenlesbarer Datensatz kann auch eine eindeutige Seriennummer 220 sein, die im Verfahren 200 vergeben wurde. Der maschinenlesbare Datensatz des Objekts 20 kann durch Abfragen 2023 eines entsprechenden Objektidentifizierungsdatensatzes 420 in einem Hintergrundsystem 40 validiert werden, wobei nach einer erfolgreichen Validierung des maschinenlesbaren Datensatzes ein Status des Objektidentifizierungsdatensatzes 420 oder eines Prozedurdatensatzes 450 im Hintergrundsystem 40 auf "in Verwendung" gesetzt wird.

In dem Schritt 202 kann optional auch jedes Hilfsmittel 50 durch das Endgerät 30 unmittelbar vor der Prozedur identifiziert werden. Dabei kann jedes verwendete Hilfsmittel 50 mittels einer Kamera 31a des Endgeräts 30 erfasst werden. Das erfasste Hilfsmittel 50 kann mittels Methoden computergestützter Gesichts- und/oder Muster- und/oder Bilderkennung durch die Auswerteeinheit 33 des Endgeräts 30 analysiert werden, um das Hilfsmittel 50 zu identifizieren.

Nach erfolgreicher Identifizierung in den Schritten 201 und 202 wird die Prozedur von der Person 1 durchgeführt. Dazu wird die Person ggf. dazu aufgefordert, sich vor da Endgerät 30 zu platzieren und die Frontkamera 31a des Endgeräts 3900 zu aktivieren (sogenannter Selfie-Modus). So kann der Benutzer sich selbst während der Durchführung der Prozedur beobachten (Self-Mirroring). Damit kann er sich selbst begutachten und das Endgerät 30 kann darüber hinaus die Person 1, das Objekt 20 und/oder das Hilfsmittel 50 erkennen und erfassen. Während der gesamten Durchführung der Prozedur bleibt die Erfassungseinheit 31 des Endgeräts 30 aktiviert, wodurch das Endgerät 30 Bilder erfassen und auswerten kann.

Dazu wird die Person ggf. auch dazu aufgefordert, einen von der Kamera 31a des Endgeräts erfassbaren Bereich oder einen vordefinierten Bereich nicht mehr zu verlassen.

Der erfassbare Bereich kann auf der Anzeigeeinheit 34 des Endgeräts 30 während der gesamten Prozedur oder nur zu Beginn der Prozedur angezeigt werden. Dieser Bereich kann durch Methoden der "erweiterten Realität (engl. "Augmented Reality") in der Anzeigeeinheit 34 des Endgeräts 30 durch Linien, Schraffuren, etc. angezeigt werden. Er kann durch Entfernungen zur Kamera, Lichtverhältnisse, etc. vom Endgerät 30 selbst bestimmt werden. Er kann durch die Prozedur selbst bzw. das Objekt 20 und/oder die Hilfsmittel 50 vorbestimmt sein.

In einem Schritt 203 wird zumindest eine Position der Person 1 und zumindest eine Position des Objekts 20 und ggf. eine Position des Hilfsmittels 50 durch das Endgerät 30 mittels Methoden computergestützter Gesten- und/oder Muster- und/oder Bilderkennung während der Durchführung der Prozedur nachverfolgt. Dabei können Positionen und Bewegungen erfasst und mittels der Auswerteeinheit 33 des Endgeräts mittels Methoden computergestützter Gesichts- und/oder Muster- und/oder Bilderkennung analysiert werden. Beispielsweise wird die Position und/oder die Bewegung eines Körperteils der Person 1, bevorzugt eine oder beide Hände 12 der Person 1 erfasst und analysiert dazu werden.

Dabei kann eine Nachverfolgungseinheit des Endgeräts 30 zum Nachverfolgen 203 einer oder mehrerer Positionen der Person 1 während der Durchführung der Testprozedur mittels Methoden computergestützter Gesten- und/oder Muster- und/oder Bilderkennung mittels eines Prüfens, ob beide Hände 12 der Person 1 während der gesamten Durchführung der Testprozedur in einem vordefinierten Bereich oder einem von der Kamera 31a des Endgeräts 30 erfassbaren Bereich sind, eingerichtet sein.

Dabei kann eine Nachverfolgungseinheit des Endgeräts 30 zum Nachverfolgen 203 von einer oder mehreren Positionen des Testobjekts 20 anhand der Form des Objekts während der Durchführung der Testprozedur mittels Methoden computergestützter Gesten- und/oder Muster- und/oder Bilderkennung eingerichtet sein.

Das Prüfen durch die Nachverfolgungseinheit des Endgeräts 30 kann dabei auch umfassen, ob das Gesicht 1a und die beiden Hände 1b der Person 1 während einer Abgabe einer Speichelprobe in dem vordefinierten Bereich oder dem von der Kamera 31a des Endgeräts 30 erfassbaren Bereich ist.

Das Prüfen durch die Nachverfolgungseinheit des Endgeräts 30 kann dabei auch umfassen, ob das Gesicht 1a und die beiden Hände 1b der Person 1 während einer Hinzugabe einer Extraktionsflüssigkeit in die Speichelprobe in dem vordefinierten Bereich oder dem von der Kamera 31a des Endgeräts 30 erfassbaren Bereich ist.

Das Prüfen durch die Nachverfolgungseinheit des Endgeräts 30 kann dabei auch umfassen, ob das Gesicht 1a und die beiden Hände 1b der Person 1 während eines Schüttelns der Speichelprobe für eine vorgegebene Zeitdauer in dem vordefinierten Bereich oder dem von der Kamera 31a des Endgeräts 30 erfassbaren Bereich ist

Das Prüfen durch die Nachverfolgungseinheit des Endgeräts 30 kann dabei auch umfassen, ob das Gesicht 1a und die beiden Hände 1b der Person 1 und das Objekt 20 während eines Injizierens der Speichelprobe auf das Objekt 20 in dem vordefinierten Bereich oder dem von der Kamera 31a des Endgeräts 30 erfassbaren Bereich ist.

Während der Prozedurdurchführung wird die Prozedur durch Anweisungen oder Hinweise oder Warnungen angeleitet 204.

Während der Prozedurdurchführung wird die Prozedur dokumentiert 205. Dazu werden beispielsweise in zufällig ausgewählten Zeitpunkten Screenshots von dem Endgerät 30 aufgenommen und in einen Prozedur-Identifizierungsdatensatz 321 in der Speichereinheit 32 des Endgeräts 30 abgelegt. Eine beispielhafte Struktur eines Prozedur-Identifizierungsdatensatzes 321 ist in Fig. 9d dargestellt. Der Prozedur-Identifizierungsdatensatz 321 der Fig. 9d umfasst eine Prozedur-Identifizierungsnummer, die bevorzugt Verfahrens- und Systemweit eindeutig ist. Der Prozedur-Identifizierungsdatensatz 321 umfasst zudem die Prozedurdokumentation, die Ergebnisse und/oder Zwischenergebnisse und/oder Momentaufnahmen (Screenshots) von der Durchführung umfasst.

Fig. 4 zeigt das System 2000, es wird auf die Ausführungen zur Fig. 3 verwiesen. Die Systemkomponenten (Endgerät 30, Hintergrundsystem 40) sind bevorzugt die Systemkomponenten aus der Fig. 2 oder der Fig. 4, es wird zur Vermeidung von Wiederholungen auf die Aussagen der Figuren 1 bis 3 verwiesen.

Die Auswerteeinheit 33 kann ein Mustererkennungsmodul 333 umfassen. Dieses Mustererkennungsmodul 333 ist eingerichtet, Methoden computergestützter Gesichts-, Muster-, Zeichen- und/oder Bilderkennung auszuführen. Beispielsweise wird zum Erfassen und Auswerten der Position oder Bewegungen von Person 1, Objekt 20 und/oder Hilfsmittel 50 ein "Model target tracking" Verfahren aus dem Bereich der künstlichen Intelligenz angewendet.

Beispielsweise werden zum Erfassen und Auswerten der Position oder Bewegungen von Person 1, Objekt 20 und/oder Hilfsmittel 50 Sensoren (nicht gezeigt in Fig. 4) an der Person 1 (bspw. an den Händen 12) angebracht. Die von diesen Sensoren generierten Sensordaten werden dem Endgerät 30 als Positions- oder Bewegungsinformationen bereitgestellt und werden von der Auswerteeinheit 33 bei der Erfassung und Analyse der Position oder Bewegungen von Person 1, Objekt 20 und/oder Hilfsmittel 50 ausgewertet. Zum Einsatz kommen beispielsweise UWB-Sensoren oder Bluetooth-Sensoren. Die Sensoren sind Beschleunigungs- und/oder Positionssensoren.

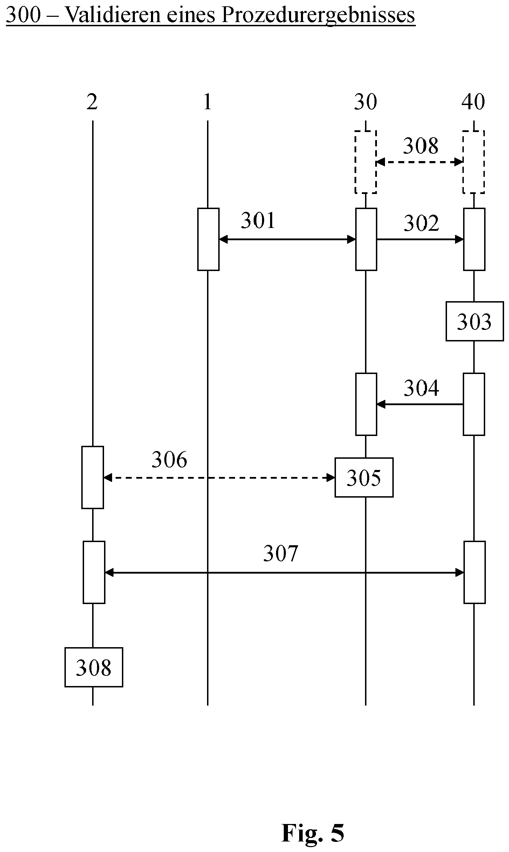

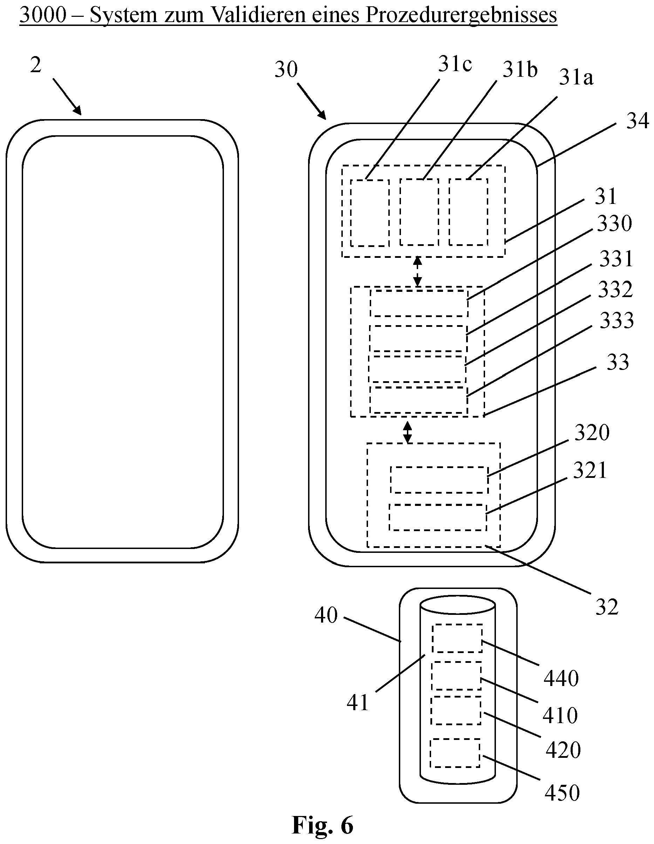

Fig. 5 zeigt ein Ablaufdiagrams eines Verfahrens 300 zum computergestützten Validieren einer von einer Person 1 durchgeführten und computergestützt nachverfolgten Prozedur. Gestrichelt dargestellte Elemente sind optional. Fig. 6 zeigt ein Systems 3000 zum computergestützten Validieren einer von einer Person 1 durchgeführten und computergestützt nachverfolgten Prozedur. Bei der Beschreibung des Verfahrens 300 aus Fig. 5 wird auch auf das System 3000 der Fig. 6 eingegangen. Das Verfahren 300 kann sich dem Verfahren 200 der Fig. 1 direkt anschließen. Ein beispielhaftes Anschließen ist in Fig. 8j gezeigt.

In einem Schritt 301 wird eine Validierung eines Prozedurergebnisses durch eine Personeneingabe an einem Endgerät 30 angefordert. Dazu wählt die Person 1 am Endgerät 30 aus, welches Prozedurergebnis validiert werden soll. Beispielsweise können verschiedene Prozedurergebnisse im Endgerät 30 ausgewählt werden.

Im Schritt 302 wird eine Validierungsanfrage im Endgerät 30 erzeugt und an das Hintergrundsystem 40 gesendet. Die Validierungsanfrage umfasst den Streuwert 430 über den Personendatensatz 320 und einen Prozeduridentifizierungsdatensatz 321 des zu validierenden Prozedurergebnisses. Personendatensatz 320 und einen Prozeduridentifizierungsdatensatz 321 werden entsprechend aus der Speichereinheit 32 des Endgeräts 30 erhalten, ggf. ist eine Entschlüsselung vorzunehmen, wenn Schritt 104 angewendet wurde.

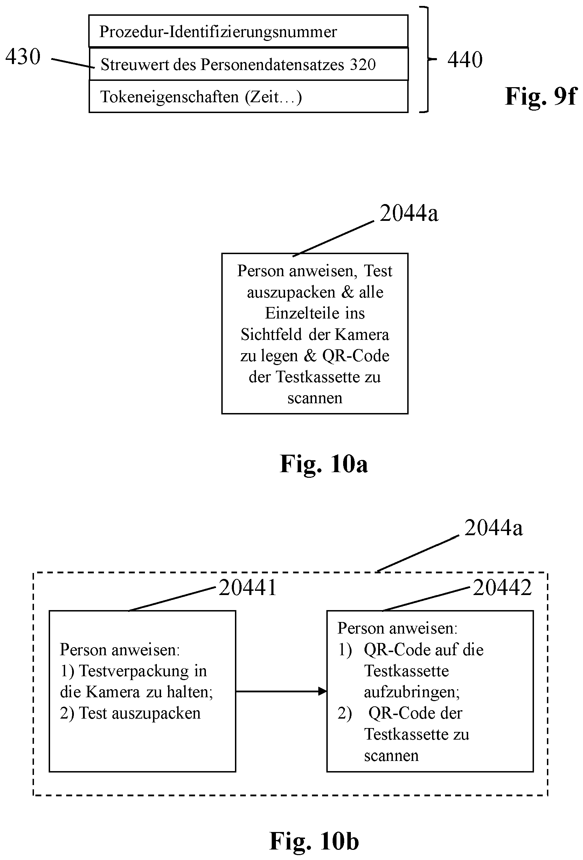

Im Hintergrundsystem 40 wird im Schritt 303 ein Validierungstoken 440 erstellt. Eine beispielhafte Struktur eines Validierungstokens ist in Fig. 9f dargestellt. Der Validierungstoken 440 umfasst beispielsweise auch den Streuwert 430 des Personendatensatzes 320 (gleich Streuwert 430 des Personendatensatzes 320 im Prozedurdatensatz 450 oder zum Streuwert 430 des Personendatensatzes 320 im anonymisierten Personendatensatzes 410). Der Validierungstoken 440 umfasst beispielsweise zudem die Prozeduridentifikationsnummer. Der Validierungstoken 440 umfasst Tokeneigenschaften, beispielsweise eine Zeitdauer, die der Validierungstoken 440 gültig ist und/oder eine Signatur des Hintergrundsystems 40. Der Validierungstoken 440 ist beispielsweise ein jwt-Token. Der Streuwert 430 kann dabei bei jeder Validierungsanfrage neu erstellt werden und mit der Prozedur-Identifizierungsnummer zur Validierung des Prozedurergebnisses (Verfahren 300) an das Hintergrundsystem 40 gesendet werden. Die Validierung findet dann über den Token 440 statt und gleicht die vorher übergebenen Parameter mit dem im Hintergrundsystem 40 dokumentierten Datenbestand ab. Ist der Datenabgleich positiv so ist die Validierung positiv. Ist der Datenabgleich negativ (weichen die übergebenen Daten von den abgelegten Daten ab), so ist die Validierung negativ.

Dieser Validierungstoken 440 wird im Schritt 304 im Endgerät 30 empfangen.

Im Anzeigeschritt 305 wird ein maschinenlesbarer Code mit dem Validierungstoken 440 und einer Information zum Hintergrundsystem 40 (Link zum Validierungsserver) erzeugt und zusammen mit dem Prozedurergebnis auf der Anzeigeeinheit 34 des Endgeräts 30 angezeigt.. Eine Prüfinstanz 2, beispielsweise ein Auditor oder ein Automat mit Scanfunktion liest im Schritt 306 den maschinenlesbaren Code (eine quasi unbestätigte Validierungsbestätigung) aus. Mit dem Einlesen im Schritt 306 stellt die Prüfinstanz 2 eine Validierungsanfrage unter Prüfung des Validierungstokens 440 an das Hintergrundsystem 40. Ist der Validierungstoken 440 gültig, so können vom Hintergrundsystem 40 das Prozedurergebnis, der Streuwert 430 über den Personendatensatz 320 und bevorzugt Prozedurinformationen abgerufen werden. Der maschinenlesbare Code wird zusammen mit dem Prozedurergebnisses auf der Anzeigeeinheit 34 des Endgeräts 30 angezeigt.

Eine Prüfinstanz 2, beispielsweise ein Auditor oder ein Automat mit Scanfunktion liest im Schritt 306 den maschinenlesbaren Code aus Schritt 305 aus. Mit dem Einlesen im Schritt 306 stellt die die Prüfinstanz 2 eine Validierungsanfrage unter Prüfung des Validierungstokens 440. Ist der Validierung des token 440 gültig, so können vom Hintergrundsystem das Prozedurergebnis, der Streuwert 430 über den Personendatensatz 320 und bevorzugt Prozedurinformationen abgerufen werden. In einem Anzeigen-Schritt 308 wird das Validierungsergebnis bezüglich des Prozedurergebnisses zusammen mit dem Prozedurergebnis und dem Streuwert 430 über den Personendatensatz 320 und bevorzugt die Prozedurinformationen auf einer Anzeigeeinheit der Prüfinstanz 2 angezeigt.

Diese computergestützt nachverfolgte Prozedur ist bevorzugt eine nach dem Verfahren 200 zum computergestützten Nachverfolgen der von der Person 1 durchzuführenden Prozedur computergestützt nachverfolgt worden.

Der Validierungstoken 440 hat eine Gültigkeitszeitdauer für das Prozedurergebnis definiert. Diese Gültigkeitsdauer ist beispielsweise zehn Minuten. Damit haben Person 1 und Prüfinstanz 2 diese zehn Minuten nach dem Anfordern-Schritt 301 Zeit, das Prozedurergebnis zu validieren. Danach ist der Validierungstoken ungültig, die Validierung ist nicht mehr möglich.

Das Prozedurergebnis kann beliebig oft validiert werden.

Die Prozedurinformation ist beispielsweise ein Prozedurdatum und/oder eine Prozedurzeit gemäß Hintergrundsystem. Diese Prozedurinformation wird auf der Anzeigeeinheit der Prüfinstanz 2 angezeigt, wodurch ein Benutzer der Prüfinstanz 2 die Informationen gemäß Hintergrundsystem 2 (nicht gemäß Endgerät 30) sieht. Dies gilt auch für das Prozedurergebnis, das vom Hintergrundsystem erhalten und in der Prüfinstanz 2 angezeigt wird.

Der Prozeduridentifizierungsdatensatz 321 wird vom Endgerät 30 erzeugt.

Der maschinenlesbarer Code ist ein QR-Code. Die Prüfinstanz 2 hat damit eine Erfassungseinheit (Kamera, Lesegerät), um den QR-Code im Schritt 306 einzulesen.

Fig. 6 zeigt das System 3000, es wird auf die Ausführungen zur Fig. 5 verwiesen. Die Systemkomponenten (Endgerät 30, Hintergrundsystem 40) sind bevorzugt die Systemkomponenten aus der Fig. 2 oder der Fig. 4, es wird zur Vermeidung von Wiederholungen auf die Aussagen der Figuren 1 bis 5 verwiesen.

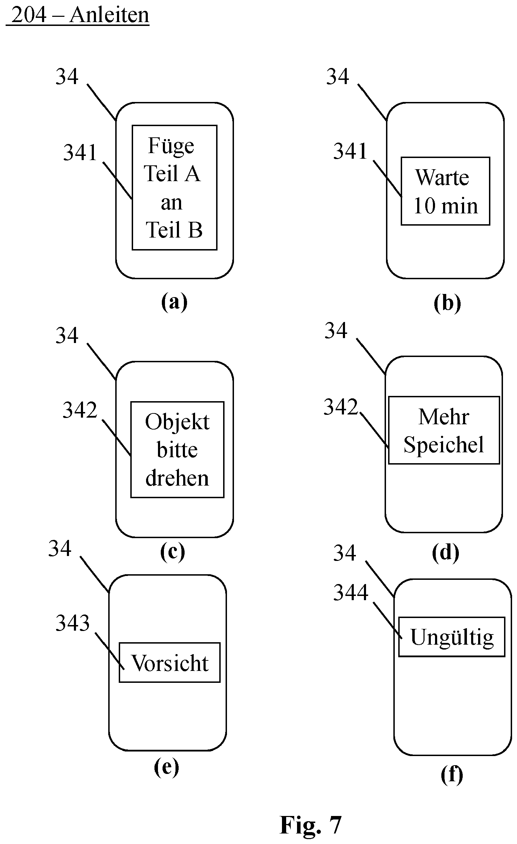

In Fig. 7a-e ist jeweils ein Anzeigeelement 34 eines Endgeräts 30 gezeigt, auf dem Anleitungshinweise während der Durchführung der Prozedur dargestellt sind.

So zeigt Fig. 7a beispielsweise eine Instruktion 341, wie das Objekt 40 behandelt/verändert werden muss, um eine Prozedur erfolgreich durchzuführen. Hier muss ein Teil A an ein Teil B angefügt werden, es könnte sich um das Zusammenbauen einer Testapparatur handeln. Die (korrekte oder fehlerhafte) Durchführung dieser Instruktion 341 wird dann von dem Endgerät 30 nachverfolgt.

So zeigt Fig. 7b beispielsweise eine Instruktion 341, dass nun eine Wartezeit von 10 min einzuhalten ist, um eine Prozedur erfolgreich durchzuführen. Die (korrekte oder fehlerhafte) Durchführung dieser Instruktion 341 wird dann von dem Endgerät 30 nachverfolgt. So könnte es ein Fehler bewertet werden, wenn binnen dieser Wartezeit das Objekt 40 aus dem vordefinierten oder erfassbaren Bereich der Erfassungseinheit des Endgeräts 30 entnommen wird.

So zeigt Fig. 7c beispielsweise eine Instruktion 342, wie eine Position des Objekts 40 korrigiert werden muss, um eine Prozedur erfolgreich durchzuführen. Hier muss das Objekt 40 gedreht werden, möglicherweise erfolgte diese Instruktion 342 nach dem Erkennen einer fehlerhaften Positionierung des Objekts 40. Die (korrekte oder fehlerhafte) Durchführung dieser Instruktion 342 wird dann von dem Endgerät 30 nachverfolgt.

So zeigt Fig. 7d beispielsweise eine Instruktion 342, was am Objekt 40 korrigiert werden muss, um eine Prozedur erfolgreich durchzuführen. Hier muss mehr Speichel auf das Objekt 40 appliziert werden, möglicherweise erfolgte diese Instruktion 342 nach dem Erkennen, dass dem Objekt 40 nicht ausreichend Speichel zugeführt wurde. Die (korrekte oder fehlerhafte) Durchführung dieser Instruktion 342 wird dann von dem Endgerät 30 nachverfolgt.

So zeigt Fig. 7e beispielsweise eine Warnung 343, die beachtet werden muss, um eine Prozedur erfolgreich durchzuführen und/oder um sich nicht zu verletzen. Hier könnte gewarnt werden, dass eine ausreichende Menge an Extraktionsflüssigkeit zu applizieren ist und der diesbezügliche Behälter ggf. ungeeignet ist.

So zeigt Fig. 7d beispielsweise ein (Zwischen-) Ergebnis 344 eines Schritts oder der gesamten Prozedur. Hier gilt ein Ergebnis als "ungültig".

Fig. 8a-k zeigt ein Ablaufdiagram der Verfahren 100, 200, 300 für einen Virentest, hier ein Antigentest auf SARS-CoV-2, als eine beispielhafte Prozedur. Dieses Ablaufdiagram ermöglicht den Test als Spucktest oder als Abstrichtest. Andere Testverfahren sind nicht ausgeschlossen, optionale Schritte werden gestrichelt dargestellt.

Andere Prozeduren, insbesondere humanmedizinische Testprozeduren, zum Untersuchen von Flüssigkeiten (Blut, Abstrich, Speichel, Aerosol, Tränenflüssigkeit, Schweiß, Sputum, Urin, und/oder Stuhl) eines Menschen oder eines Tieres, beispielsweise ein anderer Virusnachweistest, ein Keimtest, ein Bakterientest, ein Pilztest, etc. können durch das erfindungsgemäße Verfahren ebenfalls abgebildet werden. Die Körperflüssigkeit kann durch Abnahme von einer Hautoberfläche, beispielsweise von einer Hand oder einer Stirn erfolgen. Die Körperflüssigkeit einer Person und/oder eines Tieres wird dabei auf Infektionen, Eigenschaften und/oder Parameter untersucht.

Dieses Ablaufdiagramm leitet eine Person 1 an, einen Antigentest auf SARS-CoV-2selbstständig und insbesondere ohne weitere Person (über Videokommunikation oder physisch anwesend) durchzuführen. Dieser Antigentest auf SARS-CoV-2 kann bequem von überall durchgeführt werden. Dieser SARS-CoV-2-Test erfüllt alle Sicherheits- und Zertifizierungsstandards und gilt, als wäre dieser SARS-CoV-2-Test von einer zertifizierten Person (insbesondere humanmedizinisches Personal, wie Arzt, Krankenschwester, Apotheker) begleitet, überwacht oder gar durchgeführt worden. Dazu werden neben dem Anleiten zur korrekten Durchführung zudem auch Positionen/Bewegungen der Person und des Testobjekts (hier eine Testkassette oder ein Teststreifen) nachverfolgt (Schritt 203) und diese Positionen/Bewegungen auf Korrektheit geprüft und deren Durchführung dokumentiert (Schritt 205).

Dieses Ablaufdiagramm beinhaltet zudem auch das Einrichten eines Personenprofils (Verfahren 100, siehe Fig. 1 und 2) und das Validieren eines Prozedurergebnisses (Verfahren 300, siehe Fig. 3 und 4).

Gemäß Fig. 8a wird im Schritt 1003 geprüft, ob ein Personenprofil (=Personendatensatz 320) im Speicher 32 des Endgeräts 30 vorhanden ist.

Wird im Schritt 1003 festgestellt, dass ein Personenprofil vorhanden ist (Ja-Fall), so kann das Verfahren 100 zum Einrichten eines Personenprofils übersprungen/ausgelassen werden und es wird in einem optionalen Folgeschritt 1004 durch das Endgerät 30 geprüft, ob (erstens) ein Quarantäne-Ende-Datum des Personendatensatz 320 vorhanden ist und ob (zweitens) dieses Quarantäne-Ende-Datum nach dem aktuellen Datum liegt. Das aktuelle Datum ist beispielsweise das Datum der Durchführung dieses Verfahrens.

Wird im optionalen Schritt 1004 festgestellt, dass ein Quarantäne-Ende-Datum im Personendatensatz 320 vorhanden ist und dass dieses Quarantäne-Ende-Datum nach dem aktuellen Datum liegt (Ja-Fall), so muss die Person 1 weiter in Quarantäne bleiben. Es wird mit optionalem Schritt 1005 eine Mitteilung auf der Anzeigeeinheit 34 des Endgeräts 30 angezeigt, die die Person 1 darauf hinweist, dass die Person 1 weiterhin in Quarantäne sein muss. Ein Antigentest auf SARS-CoV-2muss bei dieser Person 1 nicht durchgeführt werden, denn egal, wie das Ergebnis dieses Antigentests auf SARS-CoV-2ist, die Person darf sich nicht in der Öffentlichkeit aufhalten. Mit den Schritten 1004 und 1005 kann eine Testkassette (als Testobjekt 20) eingespart werden. Nach der Mitteilung im optionalen Schritt 1005 wird das Verfahren gemäß Fig. 8a-k beendet.

Wird im optionalen Schritt 1004 festgestellt, dass entweder kein Quarantäne-Ende-Datum im Personendatensatz 320 vorhanden ist oder dass ein vorhandenes Quarantäne-Ende-Datum vor dem aktuellen Datum liegt (Nein-Fall), so wird ein Startbildschirm zum Auswählen der Durchführung des Verfahrens 200 (siehe auch Fig. 3) und des Verfahrens 300 (siehe auch Fig. 5) gemäß Fig. 8c durchgeführt.

Wird im Schritt 1003 festgestellt, dass kein Personenprofil vorhanden ist (Nein-Fall), so wird das Verfahren 100 gemäß der Figur 1 (bzw. des Systems 1000) zum Einrichten eines Personenprofils gestartet.

In einem optionalen Schritt 1001 wird der Person 1 (erstens) werden auf der Anzeigeeinheit 34 des Endgeräts 30 die AGB angezeigt und (zweitens) die Person 1 auf der Anzeigeeinheit 34 des Endgeräts 30 angezeigt, dass diese AGB anzunehmen sind.

In einem optionalen Schritt 1002 wird dann geprüft, ob die Person 1 durch eine jeweils entsprechende Benutzereingabe auf der Eingabeeinheit 35 des Endgeräts 30 die AGB ablehnt oder das Verfahren verlassen möchte. Wird im optionalen Schritt 1002 festgestellt, dass die Person 1 durch entsprechende Benutzereingabe auf der Eingabeeinheit 35 des Endgeräts 30 die AGB ablehnt oder das Verfahren verlassen möchte (Ja-Fall), so wird das Verfahren gemäß Fig. 8a-k beendet.

Wird im optionalen Schritt 1002 festgestellt, dass die Person 1 durch eine jeweils entsprechende Benutzereingabe auf der Eingabeeinheit 35 des Endgeräts 30 die AGB nicht ablehnt oder das Verfahren nicht verlassen möchte (Nein-Fall), so wird das Verfahren 100 gemäß Fig. 8b (basierend auf Fig. 2 und Fig. 3) ausgeführt, insbesondere durch die Schritte 101 und 102, siehe gestricheltes Rechteck.

Im Schritt 1011 wird die Person 1 mit einer Mitteilung auf der Anzeigeeinheit 34 des Endgeräts 30 dazu aufgefordert, einen Zugriff auf eine Kamera 31a des Endgeräts 30 zu erlauben und ein gültiges Identifizierungsdokument 10 der Person 1 mit Vorderseite 12 und Rückseite 13 zu zeigen. Diese Mitteilung kann Schritt für Schritt angezeigt werden und vor dem Anzeigen des nächsten Schritts wird von der Person der jeweilige Schritt durchgeführt.

Im Folgeschritt 1021 wird das gezeigte Identifizierungsdokument 10 von der Kamera 31a des Endgeräts 30 erfasst (jeweils Vorder- und Rückseite) und durch die Auswerteeinheit 33 eine OCR-Erkennung durchgeführt, um die personenbezogene Daten 15 des Identifizierungsdokuments 10 für den Personendatensatz 320 zu erhalten. In diesem Schritt 1021 kann auch ein Lichtbild 14 des im Schritt 1011 gezeigten Identifizierungsdokument 10 von der Kamera 31a des Endgeräts 30 erfasst werden, um Biometriedaten für den Personendatensatz 320 zu erhalten.

Im optionalen Schritt 1012 wird die Person 1 mit einer Mitteilung auf der Anzeigeeinheit 34 des Endgeräts 30 dazu aufgefordert, die Frontkamera 31a des Endgeräts 30 zu aktivieren und ein Bild von dem Gesicht 1a der Person 1 zur Verifizierung eines Lichtbilds 14 des Identifizierungsdokuments 10 zu erstellen. Im optionalen Folgeschritt 1022 kann das erstellte Bild des Gesichts 1a der Person von der Kamera 31a des Endgeräts 30 erfasst werden, um Biometriedaten für den Personendatensatz 320 zu erhalten. Diese erhaltenen Biometriedaten des erfassten Gesichts 1a wird zunächst mit den Biometriedaten des Lichtbilds 14 des Identifizierungsdokument 10 verknüpft.

Die Kommunikation in den Schritten 1011, 1021, 1012 und 1022 kann über einen verschlüsselten Datenstrom ("data stream") 1013 in Zusammenarbeit mit einem externen Dienstleister erfolgen. Dabei werden die Videodaten und die OCR-Daten zur Absicherung des so gewonnen Personendatensatz 320 verschlüsselt. Ergibt der Vergleich, dass die Biometriedaten mit ausreichender Genauigkeit übereinstimmen, werden die Biometriedaten von dem Lichtbild 14 akzeptiert und als Teil in dem Benutzerdatensatz 320 vorgesehen.

Im optionalen Schritt 1023 wird dann geprüft, ob die Zeichenerkennung und die Lichtbilderkennung der Schritte 1011, 1021, 1012 und 1022 erfolgreich war (also ob ein gültiger Benutzerdatensatz 320 erhalten wurde). Dazu werden die erhaltenen Biometriedaten des erfassten Gesichts 1a mit den Biometriedaten des Lichtbilds 14 des Identifizierungsdokument 10 durch die Auswerteeinheit 33 des Endgeräts 30 verglichen.

Wird im optionalen Schritt 1023 durch die Auswerteeinheit 33 festgestellt, dass die erhaltenen Biometriedaten des erfassten Gesichts 1a nicht mit ausreichender Genauigkeit mit den Biometriedaten des Lichtbilds 14 des Identifizierungsdokument 10 übereinstimmen oder dass die Zeichenerkennung und die Lichtbilderkennung der Schritte 1011, 1021, 1012 und 1022 nicht erfolgreich waren (Nein-Fall), so wird im optionalen Schritt 1024 eine Aufforderung an die Person 1 generiert und durch die Anzeigeeinheit 34 des Endgeräts 30 angezeigt, dass ein anderes Identifizierungsdokument zu verwenden ist oder dass das Verfahren 100 unter besseren Lichtbedingungen erneut zu versuchen ist. Nach der Mitteilung im optionalen Schritt 1024 wird das Verfahren gemäß Fig. 8a-k beendet.

Wird im optionalen Schritt 1023 durch die Auswerteeinheit 33 festgestellt, dass die erhaltenen Biometriedaten des erfassten Gesichts 1a mit ausreichender Genauigkeit mit den Biometriedaten des Lichtbilds 14 des Identifizierungsdokument 10 übereinstimmen oder dass die Zeichenerkennung und die Lichtbilderkennung der Schritte 1011, 1021, 1012 und 1022 erfolgreich waren (Ja-Fall), so wird im Schritt 1025 der Benutzerdatensatz 320 fertiggestellt. Optional wird der Person 1 in diesem Schritt 1025 gestattet, die Anschrift als personenbezogenes Datum vor dem Abspeichern 103 zu editieren. Der Schritt 1025 entspricht den Schritten 103, 104 und 105 der Fig. 1, auf weiterführende Äußerungen wird verzichtet.

Das Ergebnis der Schritte 1011, 1021, 1012, 1022, 1023 und 1025 ist ein Benutzerdatensatz 320, der Biometriedaten und personenbezogene Daten 15 basierend auf dem Identifizierungsdokument 10 (und ggf. der Verifizierung der Person 1 in Schritt 1012) umfasst. Dieser Benutzerdatensatz 320 kann in einer Speichereinheit 32 des Endgeräts 30 verschlüsselt abgelegt werden.

Nach dem Schritt 1025 wird das Verfahren 200 (siehe auch Fig. 3) und 300 (siehe auch Fig. 5) gemäß Fig. 8c durchgeführt.

Gemäß Fig. 8c wird im optionalen Schritt 199 ein Auswahlbildschirm für die Nachverfolgung des Antigentests auf SARS-CoV-2gemäß Verfahren 200 und für die Validierung des durchgeführten Antigentests auf SARS-CoV-2gemäß Verfahren 300 gezeigt. Mit einer Benutzerauswahl am Endgerät 30 im Schritt 199 kann entweder das Verfahren 200 oder das Verfahren 300 ausgewählt werden. Dieser Auswahlbildschirm im Schritt 199 ist optional, die Verfahren 200 und 300 können auch durch verschiedene Applikationen im Endgerät 30 einzeln und unabhängig voneinander ausgeführt werden.

Wählt die Person 1 im Schritt 199 die Durchführung eines neuen Antigentests auf SARS-CoV-2, so wird das Verfahren 200 gemäß Fig. 3 und 4 ausgeführt. Dazu wird im optionalen Schritt 2041 ein Einleitungsvideo auf der Anzeigeeinheit 34 des Endgeräts 30 angezeigt und es wird von der Auswerteeinheit 33 des Endgeräts 30 geprüft, ob die Umweltbedingungen (Licht, Raum) den Anforderungen an einen Antigentest auf SARS-CoV-2 und den Anforderungen für dieses Verfahren 200 erfüllen.

Wird im Schritt 2041 durch das Endgerät 30 festgestellt, dass die Umweltbedingungen ungeeignet sind, um den Antigentest auf SARS-CoV-2durchzuführen oder um dessen Durchführungen nachverfolgen zu können (bspw. aufgrund schlechter Lichtverhältnisse oder aufgrund großem Bildrauschanteil oder durch viel Bewegung im Kamerabild oder etc.) (Verlassen-Fall), so wird im Folgeschritt 2049a (in Fig. 8g) der Person 1 das Prozedurergebnis "ungültiger Test" angezeigt, dieses Prozedurergebnis wird dokumentiert (bspw. in dem Prozedur-Identifizierungsdatensatz 321) und das Verfahren über eine "Verlassen" Schaltfläche beendet.

Wird im Schritt 2041 (Fig. 8c) durch das Endgerät 30 festgestellt, dass die Umweltbedingungen geeignet sind, um den Antigentest auf SARS-CoV-2durchzuführen und dessen Durchführungen nachverfolgbar sind (Ja-Fall), so wird im optionalen Folgeschritt 2042 der Person 1 eine Anleitung zum Einrichten des Endgeräts 30 auf der Anzeigeeinheit 34 des Endgeräts 30 angezeigt. Mit dieser Anleitung wird die Person 1 dazu angeleitet, die Frontkamera 31a des Endgeräts zu aktivieren, die richtige Kameraperspektive einzustellen und sich so vor das Endgerät 30 zu positionieren, dass die Kamera 31a das Gesicht 1a der Person 1 erfassen kann. Im darauffolgenden Schritt 2043 wird geprüft, ob die Einrichtung des Endgeräts 30 erfolgreich war.

Wird im optionalen Schritt 2043 durch das Endgerät 30 festgestellt, dass die Einrichtung des Endgeräts 30 nicht erfolgreich war, so wird der gemeinsame Auswahlbildschirm im Schritt 199 wieder gezeigt (Fig. 8c).

Wird im optionalen Schritt 2043 durch das Endgerät 30 festgestellt, dass die Einrichtung des Endgeräts 30 erfolgreich war, so erfolgt im Schritt 201 der Versuch die Person 1 zu identifizieren. Dazu wird das aktuelle Kamerabild des Endgeräts 1 erfasst (also das Gesicht 1a der Person 1) und mit den Biometriedaten des Personendatensatzes 320 verglichen (siehe auch Ausführungen zu Fig. 3).

Im folgenden Schritt 2014 wird durch das Endgerät 30 geprüft, ob die Identifizierung der Person 1 erfolgreich war. Wird im Schritt 2014 festgestellt, dass die Identifizierung der Person 1 nicht erfolgreich war (Verlassen-Fall), so wird der gemeinsame Auswahlbildschirm im Schritt 199 wieder gezeigt (Fig. 8c).

Wird im Schritt 2014 durch das Endgerät 30 festgestellt, dass die Identifizierung der Person 1 erfolgreich war (Ja-Fall), so wird im optionalen Folgeschritt 2044a eine Anleitung zum Vorbereiten des Antigentests auf SARS-CoV-2angezeigt. Dieses Vorbereiten kann auf verschiedene Weise erfolgen, wovon zwei in der Fig. 10a und 10b dargestellt sind.

Im darauffolgenden Schritt 202 (Fig. 8d) erfolgt der Versuch die Testkassette 20 (das Testobjekt) zu identifizieren. Dabei wird ein QR-Code 210 der Testkassette 20 erfasst und es wird geprüft, ob eine im QR-Code 210 einkodierte Prozedurseriennummer mit einer Prozedurseriennummer eines Prozedurdatensatzes 450 des Hintergrundsystems 40 übereinstimmt. Zudem kann in dem Schritt 202 geprüft werden, ob ein Status des Prozedurdatensatzes 450 auf "hergestellt" gesetzt ist und ob dieser Status auf den Status "in Verwendung" gesetzt werden kann. Zudem kann in dem Schritt 202 geprüft werden, ob der Person 1 der Streuwert 430 des Personendatensatzes 320 des Prozedurdatensatzes 450 zugeordnet werden kann.

Wird im Schritt 202 durch das Endgerät 30 festgestellt, dass die Identifizierung der Testkassette 20 nicht erfolgreich war (Nein-Fall), so wird im Folgeschritt 2045a die Person durch eine Mitteilung auf der Anzeigeeinheit 34 des Endgerät 30 darüber informiert, dass die Testkassette 20 dieses Antigentests auf SARS-CoV-2 nicht erkannt wurde oder dass die vorliegende Testkassette 20 einen ungültigen/unzulässigen QR-Code aufweist. Darauffolgend wird der Auswahlbildschirm im Schritt 199 gezeigt (Fig. 8c).

Wird im Schritt 202 durch das Endgerät 30 festgestellt, dass die Identifizierung der Testkassette 20 erfolgreich war (Ja-Fall), so kann die Testprozedur durchgeführt werden, denn sowohl die Person 1 als auch das Testobjekt 20 konnten identifiziert werden (Schritte 201, 202). Somit kann jetzt die Nachverfolgung der Testprozedur gemäß Schritt 203 (hier konkret die Schritte 203a-h) erfolgen. In dem optionalen Schritt 2030 der Fig. 8d kann zwischen verschiedenen Testprozeduren für einen Virusnachweistest, hier Antigentest auf SARS-CoV-2, gewählt werden. Hier stellvertretend wird der Abstrich-Test und der Spuck-Test beschrieben, andere Tests zur Untersuchung der Körperflüssigkeit der Person 1 sind ebenfalls denkbar.

Das Nachverfolgen der Testprozedur durch das Endgerät 30 wird zunächst anhand des SpuckTests in den Figuren 8d-8g beschrieben. In den Figuren 8j und 8k wird dann auf den Abstrichtest (=Stäbchentest) gemäß Schritt 2100 eingegangen.

Im optionalen Schritt 2046 wird die Person 1 durch die Anzeigeeinheit 34 des Endgeräts 30 befragt, ob sie in den letzten 10 Minuten gegessen, getrunken oder geraucht hat.

Wird im Schritt 2046 durch das Endgerät 30 festgestellt (durch eine entsprechende Personeneingabe in der Eingabeeinheit 35 des Endgeräts 30), dass die Person 1 in den letzten 10 Minuten gegessen, getrunken oder geraucht hat (Ja-Fall), so wird in einem optionalen Schritt 2045b die Person 1 darüber informiert, dass der Test zu einem späteren Zeitpunkt zu wiederholen ist. Zudem kann der Status des Prozedurdatensatzes 450 in dem Hintergrundsystem 40 von dem Status "in Verwendung" auf den Status "hergestellt" zurückgesetzt werden. Anschließend wird der Auswahlbildschirm in Schritt 199 wieder gezeigt (Fig. 8c).

Wird im optionalen Schritt 2046 durch das Endgerät 30 festgestellt (durch eine entsprechende Personeneingabe in der Eingabeeinheit 35 des Endgeräts 30), dass die Person 1 in den letzten 10 Minuten nicht gegessen, nicht getrunken und auch nicht geraucht hat (Nein-Fall), so wird im optionalen Schritt 2044b (Fig. 8e) die Person 1 angewiesen, (erstens) den Test zusammenzubauen und (zweitens) den zusammengebauten Test in die Kamera 31a des Endgeräts 30 zu halten ist. Das Zusammenbauen kann dann zeitgleich mit der Anweisung erfolgen. Die Anweisung kann ein Animationsvideo enthalten. Die Anweisung kann durch "Augmented Reality" unterstützt werden. Während des Zusammenbauens wird gemäß Schritt 205 der Zusammenbau dokumentiert, indem mindestens eine Aufnahme durch die Kamera 31a des Endgeräts 30 von der Person 1 während des Zusammenbauens gemacht wird. Es können auch mehrere, beispielsweise 2, Aufnahmen oder auch ein oder mehrere Videos durch die Kamera 31a des Endgeräts 30 von der Person 1 während des Zusammenbauens gemacht werden. Diese Aufnahmen werden in einer Produktdokumentation im Datensatz 321 in der Speichereinheit 32 des Endgeräts 30 gespeichert.

Im optionalen Schritt 203a wird durch das Endgerät 30 geprüft, ob das Zusammenbauen im Schritt 2044b erfolgreich war. Dazu müssen sowohl das Gesicht 1a also auch beide Hände 1b der Person 1 ständig im vordefinierten Bereich (bspw. ein Rahmen der Anzeigeeinheit 34 oder ein gezeichneter Bereich ("Augmented Reality") innerhalb des erfassbaren Bereichs der Kamera 31a, auf der Anzeigeeinheit 34 dargestellt) sein und dürfen diesen Bereich nie verlassen haben. Zudem muss der Zusammenbau dem Zusammenbau des Test-Typs (Objektidentifizierungsdatensatz 420) entsprechen, was von der Auswerteeinheit 33 des Endgeräts durch Vergleichen festgestellt wird.

Wird im optionalen Schritt 203a durch das Endgerät 30 festgestellt, dass das Zusammenbauen nicht erfolgreich war (Nein-Fall), so wird im Schritt 2045b (Fig. 8d) die Person 1 darüber informiert, dass der Antigentest auf SARS-CoV-2 zu einem späteren Zeitpunkt zu wiederholen ist. Zudem kann der Status des Prozedurdatensatzes 450 in dem Hintergrundsystem 40 wieder von dem Status "in Verwendung" auf den Status "hergestellt" zurückgesetzt werden. Anschließend wird der Auswahlbildschirm in Schritt 199 wieder gezeigt (Fig. 8c).

Wird im Schritt 203a durch das Endgerät 30 festgestellt, dass das Zusammenbauen erfolgreich war (Ja-Fall), so wird im Schritt 2048a (Fig. 8e) die Person 1 mit der Anzeigeeinheit 34 des Endgeräts 30 angewiesen, eine Speichelprobe in einen Behälter als Hilfsmittel 50 (bis zu einer Fülllinie) abzugeben und mit einer Spracheingabe zu bestätigen, sobald er zur SpeichelprobenAbgabe bereit ist. Ab dem Zeitpunkt der Spracheingabe wird (erstens) die Abgabe der Speichelprobe durch die Person 1 nachverfolgt. Während der Abgabe der Speichelprobe kann gemäß Schritt 205 (zweitens) die Abgabe der Speichelprobe dokumentiert werden, indem mindestens eine Aufnahme durch die Kamera 31a des Endgeräts 30 von der Person 1 während der Speichelprobenabgabe gemacht wird. Es können auch mehrere, beispielsweise 2, Aufnahmen oder auch ein oder mehrere Videos durch die Kamera 31a des Endgeräts 30 von der Person 1 während der Speichelprobenabgabe gemacht werden. Diese Aufnahmen werden in einer Produktdokumentation im Datensatz 321 in der Speichereinheit 32 des Endgeräts 30 gespeichert.

Im Schritt 203b wird durch das Endgerät 30 geprüft, ob die Abgabe der Speichelprobe erfolgreich war. Dazu müssen sowohl das Gesicht 1a also auch beide Hände 1b der Person 1 ständig im vordefinierten Bereich (bspw. im Bilderrahmen der Anzeigeeinheit 34) sein und dürfen diesen Bereich nie verlassen haben, was von der Auswerteeinheit 33 des Endgeräts durch Nachverfolgen der Position des Gesichts 1a und der Hände 1b festgestellt wird. Zudem muss die Abgabe der Speichelprobe bis zur Fülllinie (Objekteigenschaft im Objektidentifizierungsdatensatz 420) in den Behälter erfolgen, was von der Auswerteeinheit 33 des Endgeräts durch Nachverfolgen der Füllmenge der Speichelprobe im Behälter und Erkennen des Erreichens der Fülllinie des Behälters festgestellt wird. Der Behälter muss ständig im vordefinierten Bereich (bspw. im Bilderrahmen der Anzeigeeinheit 34) sein und darf diesen Bereich nie verlassen, was von der Auswerteeinheit 33 des Endgeräts durch Nachverfolgen des Behälters festgestellt wird.

Wird im Schritt 203b durch das Endgerät 30 festgestellt, dass die Abgabe der Speichelprobe nicht erfolgreich war (Nein-Fall), so wird im Schritt 2045c (Fig. 8f) eine Meldung auf der Anzeigeeinheit 34 des Endgeräts 30 erzeugt, mit der die Person 1 darüber informiert, dass der Test wegen Verstoßes gegen die Testvorschriften oder wegen Abbruch fehlgeschlagen ist. Der Test erhält das Prozedurergebnis "ungültig". Dieses Prozedurergebnis wird in den Prozedurdatensatz 450 des Hintergrundsystems 40 eingetragen. Anschließend wird der Auswahlbildschirm in Schritt 199 wieder gezeigt (Fig. 8c).

Wird im Schritt 203b durch das Endgerät 30 festgestellt, dass die Abgabe der Speichelprobe erfolgreich war (Ja-Fall), so wird im Schritt 2048b (Fig. 8e) die Person 1 angewiesen, eine Extraktionsflüssigkeit in den Behälter mit der Speichelprobe einzubringen, den Behälter zu schließen und zu bestätigen, sobald er dazu bereit ist. Mit der Bestätigung (Sprache oder anders) wird (erstens) das Hinzugeben nachverfolgt. Während der Hinzugabe wird gemäß Schritt 205 (zweitens) das Hinzugeben dokumentiert, indem mindestens eine Aufnahme durch die Kamera 31a des Endgeräts 30 von der Person 1, während dem Einbringen der Extraktionsflüssigkeit in den Behälter gemacht wird. Es können auch mehrere, beispielsweise 2, Aufnahmen oder auch ein oder mehrere Videos durch die Kamera 31a des Endgeräts 30 von der Person 1, während dem Einbringen der Extraktionsflüssigkeit in den Behälter gemacht werden. Diese Aufnahmen werden in einer Produktdokumentation im Datensatz 321 in der Speichereinheit 32 des Endgeräts 30 gespeichert.

Im Schritt 203c wird durch das Endgerät 30 geprüft, ob das Einbringen der Extraktionsflüssigkeit in den Behälter erfolgreich war. Dazu müssen sowohl das Gesicht 1a also auch beide Hände 1b der Person 1 ständig im vordefinierten Bereich (bspw. im Bilderrahmen der Anzeigeeinheit 34) sein und dürfen diesen Bereich nie verlassen haben, was von der Auswerteeinheit 33 des Endgeräts durch Nachverfolgen der Position des Gesichts 1a und der Hände 1b festgestellt wird. Der Behälter muss ständig im vordefinierten Bereich (bspw. im Bilderrahmen der Anzeigeeinheit 34) sein und darf diesen Bereich nie verlassen, was von der Auswerteeinheit 33 des Endgeräts durch Nachverfolgen der Position Behälters festgestellt wird.

Wird im Schritt 203c durch das Endgerät 30 festgestellt, dass das Hinzugeben der Extraktionsflüssigkeit nicht erfolgreich war (Nein-Fall), so wird im Schritt 2045c (Fig. 8f) die Person 1 darüber informiert, dass der Test wegen Verstoßes gegen die Testvorschriften oder wegen Abbruch fehlgeschlagen ist. Der Test erhält das Prozedurergebnis "ungültig". Dieses Prozedurergebnis wird in den Prozedurdatensatz 450 des Hintergrundsystems 40 eingetragen. Anschließend wird der Auswahlbildschirm in Schritt 199 wieder gezeigt (Fig. 8c).

Wird im Schritt 203c durch das Endgerät 30 festgestellt, dass das Hinzugeben der Extraktionsflüssigkeit erfolgreich war (Ja-Fall), so wird im Schritt 2048c (Fig. 8f) die Person 1 angewiesen, den Behälter mit der Extraktionsflüssigkeit und der Speichelprobe für 5 Sekunden zu schütteln. Das Schütteln erfolgt zeitgleich mit der Anweisung. Während des Schüttelns wird gemäß Schritt 205 das Schütteln dokumentiert, indem mindestens eine Aufnahme durch die Kamera 31a des Endgeräts 30 von der Person 1 während des Schüttelns gemacht wird. Es können auch mehrere, beispielsweise 2, Aufnahmen oder auch ein oder mehrere Videos durch die Kamera 31a des Endgeräts 30 von der Person 1 während des Schüttelns gemacht werden. Diese Aufnahmen werden in einer Produktdokumentation im Datensatz 321 in der Speichereinheit 32 des Endgeräts 30 gespeichert.

Im Schritt 203d wird durch das Endgerät 30 geprüft, ob das Schütteln erfolgreich war. Dazu müssen sowohl das Gesicht 1a also auch beide Hände 1b der Person 1 ständig im vordefinierten Bereich (bspw. im Bilderrahmen der Anzeigeeinheit 34) sein und dürfen diesen Bereich nie verlassen haben, was von der Auswerteeinheit 33 des Endgeräts durch Nachverfolgen der Position des Gesichts 1a und der Hände 1b festgestellt wird. Das Schütteln des Behälters durch die Person 1 für mindestens 5 Sekunden wird nachverfolgt. Der Behälter muss ständig im vordefinierten Bereich (bspw. im Bilderrahmen der Anzeigeeinheit 34) sein und darf diesen Bereich nie verlassen, was von der Auswerteeinheit 33 des Endgeräts durch Nachverfolgen der Position des Behälters festgestellt wird.

Wird im Schritt 203d durch das Endgerät 30 festgestellt, dass das Schütteln nicht erfolgreich war (Nein-Fall), so wird im Schritt 2045c die Person 1 darüber informiert, dass der Test wegen Verstoßes gegen die Testvorschriften oder wegen Abbruch fehlgeschlagen ist. Der Test erhält das Prozedurergebnis "ungültig". Dieses Prozedurergebnis wird in den Prozedurdatensatz 450 des Hintergrundsystems 40 eingetragen. Anschließend wird der Auswahlbildschirm in Schritt 199 wieder gezeigt (Fig. 8c).

Wird im Schritt 203d durch das Endgerät 30 festgestellt, dass das Schütteln erfolgreich war (Ja-Fall), so wird im Schritt 2048d die Person 1 angewiesen, die Testkassette 20 zu zeigen und den Inhalt des geschüttelten Behälters (die Probe + Extraktionsflüssigkeit) auf die Testkassette 20 zu tropfen. Das Zeigen und das Tropfen erfolgen zeitgleich mit der Anweisung. Während des Zeigens und Tropfens wird gemäß Schritt 205 das Zeigen und das Tropfen dokumentiert, indem mindestens eine Aufnahme durch die Kamera 31a des Endgeräts 30 von der Person 1 während des Zeigens und Tropfens gemacht wird. Es können auch mehrere, beispielsweise 2, Aufnahmen oder auch ein oder mehrere Videos durch die Kamera 31a des Endgeräts 30 von der Person 1 während des Zeigens und Tropfens gemacht werden. Diese Aufnahmen werden in einer Produktdokumentation im Datensatz 321 in der Speichereinheit 32 des Endgeräts 30 gespeichert.

Im Schritt 203e durch das Endgerät 30 wird geprüft, ob das Zeigen und das Tropfen erfolgreich war. Dazu müssen sowohl das Gesicht 1a also auch beide Hände 1b der Person 1 ständig im vordefinierten Bereich (bspw. im Bilderrahmen der Anzeigeeinheit 34) sein und dürfen diesen Bereich nie verlassen haben. Die Testkassette 20 muss erkannt werden und muss ständig im vordefinierten Bereich (bspw. im Bilderrahmen der Anzeigeeinheit 34) sein und darf diesen Bereich nie verlassen.

Wird im Schritt 203e durch das Endgerät 30 festgestellt, dass das Zeigen und das Tropfen nicht erfolgreich war (Nein-Fall), so wird im Schritt 2045c die Person 1 darüber informiert, dass der Test wegen Verstoß gegen die Testvorschriften oder wegen Abbruch fehlgeschlagen ist. Der Test erhält das Prozedurergebnis "ungültig". Dieses Prozedurergebnis wird in den Prozedurdatensatz 450 des Hintergrundsystems 40 eingetragen. Anschließend wird der Auswahlbildschirm in Schritt 199 wieder gezeigt (Fig. 8c).

Wird im Schritt 203e durch das Endgerät 30 festgestellt, dass das Zeigen und das Tropfen erfolgreich war (Ja-Fall), so wird im Schritt 2047 eine Wartezeit von 10 Minuten angezeigt und beispielsweise als Countdown runtergezählt. Die Person 1 wird informiert, sobald die Wartezeit verstrichen ist.

Einer oder jeder der Schritte 2048a-d kann optional einen Countdown aus. Der Countdown startet beispielsweise mit dem Anzeigen der Aufforderungen auf der Anzeigeeinheit 34 des Endgeräts 30. Wird in dieser Zeit die Anweisung nach Schritten 2048a-d nicht oder falsch befolgt, so wird im Schritt 2045d die Person 1 darüber informiert, dass der Test(-Schritt) wegen Zeitüberschreitung fehlgeschlagen ist. Der Test erhält das Prozedurergebnis "ungültig". Dieses Prozedurergebnis wird in den Prozedurdatensatz 450 des Hintergrundsystems 40 eingetragen. Anschließend wird der Auswahlbildschirm in Schritt 199 wieder gezeigt (Fig. 8c).

Im darauffolgenden Schritt 2044c wird die Person 1 aufgefordert, ein Foto von der Testkassette 20 zu machen. Dabei kann die Person 1 dazu aufgefordert werden, auf eine Rückkamera des Endgeräts 30 zu schalten. Diese Rückkamera hat (meist) eine bessere Auflösung als die Frontkamera, mit der die Person 1 nachverfolgt wird (Schritte 203a-h).

Im darauffolgenden Schritt 203f wird durch das Endgerät 30 (erstens) geprüft, ob die Seriennummer der Testkassette 20 mit der im Schritt 202 übereinstimmt. Dazu wird (in Anlehnung an die Schritte 2044a und 202) der QR-Code 210 der Testkassette 20 erfasst und die daraus erhaltene Seriennummer mit der eines Objektdatensatz 450 verglichen. Zudem wird im Schritt 203f (zweitens) geprüft, ob ein oder mehrere Kontrollbalken auf dem Teststreifen der Testkassette 20 zu erkennen ist. Der oder die Kontrollbalken ist ein Sicherheitsmerkmal der Testkassette 20, der immer entstehen muss, ungeachtet der Speichelprobe.

Wird im Schritt 203f durch das Endgerät 30 festgestellt, dass entweder die Seriennummer der Testkassette 20 mit der Seriennummer im Schritt 202 nicht übereinstimmt oder dass der oder die Kontrollbalken des Antigentests auf SARS-CoV-2 auf dem Teststreifen der Testkassette 20 nicht zu erkennen ist (Nein-Fall), dann wird in einem Schritt 203g geprüft, ob die Seriennummer der Testkassette 20 mit der im Schritt 202 übereinstimmt.

Wird im Schritt 203g durch das Endgerät 30 festgestellt, dass die Seriennummer der Testkassette 20 nicht mit der Seriennummer im Schritt 202 übereinstimmt, dann wird im Schritt 2045e die Person 1 darüber informiert, dass die Seriennummer falsch ist. Der Test erhält das Prozedurergebnis "ungültig", der in den Prozedurdatensatz 450 des Hintergrundsystems 40 eingetragen wird. Anschließend wird der Auswahlbildschirm in Schritt 199 wieder gezeigt (Fig. 8c).

Wird im Schritt 203g durch das Endgerät 30 festgestellt, dass die Seriennummer der Testkassette 20 mit der Seriennummer im Schritt 202 übereinstimmt, dann wird im Schritt 2045f die Person 1 darüber informiert, dass der oder die Kontrollbalken des Tests auf dem Teststreifen der Testkassette 20 nicht zu erkennen ist (bspw. keine Farbveränderung im zu erwartenden Bereich des Teststreifens). Der Test erhält das Prozedurergebnis "ungültig", der in den Prozedurdatensatz 450 des Hintergrundsystems 40 eingetragen wird. Anschließend wird der Auswahlbildschirm in Schritt 199 wieder gezeigt (Fig. 8c).

Wird im Schritt 203f durch das Endgerät 30 festgestellt, dass die Seriennummer der Testkassette 20 mit der Seriennummer im Schritt 202 übereinstimmt und dass der oder die Kontrollbalken des Antigentests auf SARS-CoV-2 auf dem Teststreifen der Testkassette 20 zu erkennen ist (Ja-Fall), dann wird im Schritt 203h durch das Endgerät 30 geprüft, ob das Prozedurergebnis "positiv" ist.

Wird im Schritt 203g durch das Endgerät 30 festgestellt, dass das Prozedurergebnis nicht "positiv" ist (Nein-Fall), dann wird im Schritt 2049a durch das Endgerät 30 das Prozedurergebnis "negativ" angezeigt. Dieses Prozedurergebnis wird dokumentiert, beispielsweise durch Setzen des Prozedurergebnis auf "Negativ" im Prozedurdatensatz 450 des Hintergrundsystems 40 ggf. auch durch Dokumentieren in der Prozedurdokumentation des Datensatzes 321, beispielsweise auch durch Abspeichern des Fotos aus dem Schritt 2044c in der Prozedurdokumentation der Speichereinheit 32 des Endgeräts 30.

Wird im Schritt 203g durch das Endgerät 30 festgestellt, dass das Prozedurergebnis "positiv" ist (Ja-Fall), dann wird im Schritt 2049b durch das Endgerät 30 das Prozedurergebnis "positiv" angezeigt. Dieses Prozedurergebnis wird dokumentiert, beispielsweise durch Setzen des Prozedurergebnis auf "Positiv" im Prozedurdatensatz 450 des Hintergrundsystems 40 ggf. auch durch Dokumentieren in der Prozedurdokumentation des Datensatzes 321, beispielsweise auch durch Abspeichern des Fotos aus dem Schritt 2044c in der Prozedurdokumentation der Speichereinheit 32 des Endgeräts 30. Zudem erhält die Person 1 eine Anleitung, wie nun weiter vorzugehen ist. Zudem kann im Personendatensatz 320 ein Quarantäne-Ende-Datum auf ein Datum in zwei Wochen ausgehend von dem aktuellen Datum gesetzt werden. Anschließend ist das Verfahren 200 (Nachverfolgen) beendet.

Gemäß der Fig. 8d ist das Endgerät 30 optional dazu eingerichtet, verschiedene Testprozeduren für einen Virusnachweistest, hier Antigentest auf SARS-CoV-2nachzuverfolgen, wovon im optionalen Schritt 2030 eine Testprozedur auszuwählen ist. Anstelle dem vorhergehend beschriebenen Spucktest könnte im Schritt 20230 auch ein Abstrich-Test gewählt werden. In den Figuren 8j und 8k wird nun das Nachverfolgen des Abstrich-Test (=Stäbchentest) gemäß Schritt 2100 beschrieben. Die Schritte 201, 202, 203e-h, 2044c, 2047, 2045c-f und 2049a-b sind dabei identisch zu den Schritten des Spucktests und werden hier nicht nochmal beschrieben.

Im Schritt 2144a (Fig. 8h) wird die Person 1 angewiesen, (erstens) das Teststäbchen auszupacken und (zweitens) eine Probe aus einem ersten (bspw. dem linken) Nasenloch für eine Zeitspanne von mindestens 5 Sekunden zu entnehmen. Das Auspacken und Entnehmen der Nasenprobe können zeitgleich mit der Anweisung erfolgen. Die Anweisung kann ein Animationsvideo enthalten. Die Anweisung kann durch "Augmented Reality" unterstützt werden. Während des Auspackens und Entnehmen der Nasenprobe wird gemäß Schritt 205 das Auspacken und das Entnehmen der Nasenprobe dokumentiert, indem mindestens eine Aufnahme durch die Kamera 31a des Endgeräts 30 von der Person 1 während des Auspackens und Entnehmen der Nasenprobe gemacht wird. Es können auch mehrere, beispielsweise 2, Aufnahmen oder auch ein oder mehrere Videos durch die Kamera 31a des Endgeräts 30 von der Person 1 während des Auspackens und Entnehmen der Nasenprobe gemacht werden. Diese Aufnahmen werden in einer Produktdokumentation im Datensatz 321 in der Speichereinheit 32 des Endgeräts 30 gespeichert.

Im Schritt 203i wird durch das Endgerät 30 geprüft, ob das Auspacken und Entnehmen der Nasenprobe im Schritt 2144a erfolgreich war. Dazu müssen sowohl das Gesicht 1a also auch beide Hände 1b der Person 1 ständig im vordefinierten Bereich (bspw. ein Rahmen der Anzeigeeinheit 34 oder ein gezeichneter Bereich ("Augmented Reality") innerhalb des erfassbaren Bereichs der Kamera 31a, auf der Anzeigeeinheit 34 dargestellt) sein und dürfen diesen Bereich nie verlassen haben. Der Behälter muss ständig im vordefinierten Bereich (bspw. im Bilderrahmen der Anzeigeeinheit 34) sein und darf diesen Bereich nie verlassen, was von der Auswerteeinheit 33 des Endgeräts durch Nachverfolgen der Position Behälters festgestellt wird.

Wird im Schritt 203i durch das Endgerät 30 festgestellt, dass das Auspacken und Entnehmen der Nasenprobe nicht erfolgreich war (Nein-Fall), so wird im Schritt 2045c (Fig. 8f) eine Meldung auf der Anzeigeeinheit 34 des Endgeräts 30 erzeugt, mit der die Person 1 darüber informiert, dass der Test wegen Verstoßes gegen die Testvorschriften oder wegen Abbruch fehlgeschlagen ist. Der Test erhält das Prozedurergebnis "ungültig". Dieses Prozedurergebnis wird in den Prozedurdatensatz 450 des Hintergrundsystems 40 eingetragen. Anschließend wird der Auswahlbildschirm in Schritt 199 wieder gezeigt (Fig. 8c).

Wird im Schritt 203i durch das Endgerät 30 festgestellt, dass das Auspacken und Entnehmen der Nasenprobe nicht lang genug erfolgt ist (Zu-kurz-Fall), so wird auf der Anzeigeeinheit 34 des Endgeräts 30 eine Mitteilung erzeugt (nicht dargestellt), mit der die Person 1 darüber informiert, dass der Schritt 2144a noch weiter durchzuführen ist.

Wird im Schritt 203i durch das Endgerät 30 festgestellt, dass das Auspacken und Entnehmen der Nasenprobe erfolgreich war (Ja-Fall), so wird im Schritt 2144b (Fig. 8h) die Person 1 angewiesen, eine Probe aus einem zweiten (bspw. dem rechten) Nasenloch für eine Zeitspanne von mindestens 5 Sekunden zu entnehmen. Das Auspacken und Entnehmen der Nasenprobe können zeitgleich mit der Anweisung erfolgen. Die Anweisung kann ein Animationsvideo enthalten. Die Anweisung kann durch "Augmented Reality" unterstützt werden. Während des Entnehmens der Nasenprobe wird gemäß Schritt 205 das Entnehmen der Nasenprobe dokumentiert, indem mindestens eine Aufnahme durch die Kamera 31a des Endgeräts 30 von der Person 1 während des Entnehmens der Nasenprobe gemacht wird. Es können auch mehrere, beispielsweise 2, Aufnahmen oder auch ein oder mehrere Videos durch die Kamera 31a des Endgeräts 30 von der Person 1 während des Entnehmens der Nasenprobe gemacht werden. Diese Aufnahmen werden in einer Produktdokumentation im Datensatz 321 in der Speichereinheit 32 des Endgeräts 30 gespeichert.

Wird im Schritt 203j durch das Endgerät 30 festgestellt, dass Entnehmen der zweiten Nasenprobe nicht erfolgreich war (Nein-Fall), so wird im Schritt 2045c (Fig. 8f) eine Meldung auf der Anzeigeeinheit 34 des Endgeräts 30 erzeugt, mit der die Person 1 darüber informiert, dass der Test wegen Verstoßes gegen die Testvorschriften oder wegen Abbruch fehlgeschlagen ist. Der Test erhält das Prozedurergebnis "ungültig". Dieses Prozedurergebnis wird in den Prozedurdatensatz 450 des Hintergrundsystems 40 eingetragen. Anschließend wird der Auswahlbildschirm in Schritt 199 wieder gezeigt (Fig. 8c).

Wird im Schritt 203j durch das Endgerät 30 festgestellt, dass das Entnehmen der zweiten Nasenprobe nicht lang genug erfolgt ist (Zu-kurz-Fall), so wird auf der Anzeigeeinheit 34 des Endgeräts 30 eine Mitteilung erzeugt (nicht dargestellt), mit der die Person 1 darüber informiert, dass der Schritt 2144b noch weiter durchzuführen ist.

Wird im Schritt 203j durch das Endgerät 30 festgestellt, dass das Entnehmen der zweiten Nasenprobe erfolgreich war (Ja-Fall), so wird im Schritt 2144c die Person 1 angewiesen, einen Behälter mit Extraktionsflüssigkeit zu öffnen und den Nasenstab für mindestens 15 Sekunden zu in dem Behälter zu rühren und darin auszudrücken. Während dem Hinzugeben + Rühren + Ausdrücken wird gemäß Schritt 205 das Hinzugeben + Rühren + Ausdrücken dokumentiert, indem mindestens eine Aufnahme durch die Kamera 31a des Endgeräts 30 von der Person 1 während dem Hinzugeben + Rühren + Ausdrücken gemacht wird. Es können auch mehrere, beispielsweise 2, Aufnahmen oder auch ein oder mehrere Videos durch die Kamera 31a des Endgeräts 30 von der Person 1 während dem Hinzugeben + Rühren + Ausdrücken gemacht werden. Diese Aufnahmen werden in einer Produktdokumentation im Datensatz 321 in der Speichereinheit 32 des Endgeräts 30 gespeichert.

Wird im Schritt 203k durch das Endgerät 30 festgestellt, dass das Hinzugeben + Rühren + Ausdrücken nicht erfolgreich war (Nein-Fall), so wird im Schritt 2045c (Fig. 8f) eine Meldung auf der Anzeigeeinheit 34 des Endgeräts 30 erzeugt, mit der die Person 1 darüber informiert, dass der Test wegen Verstoßes gegen die Testvorschriften oder wegen Abbruch fehlgeschlagen ist. Der Test erhält das Prozedurergebnis "ungültig". Dieses Prozedurergebnis wird in den Prozedurdatensatz 450 des Hintergrundsystems 40 eingetragen. Anschließend wird der Auswahlbildschirm in Schritt 199 wieder gezeigt (Fig. 8c).

Wird im Schritt 203k durch das Endgerät 30 festgestellt, dass das Hinzugeben + Rühren + Ausdrücken nicht lang genug erfolgt ist (Zu-kurz-Fall), so wird auf der Anzeigeeinheit 34 des Endgeräts 30 eine Mitteilung erzeugt (nicht dargestellt), mit der die Person 1 darüber informiert, dass der Schritt 2144c noch weiter durchzuführen ist.

Wird im Schritt 203k durch das Endgerät 30 festgestellt, dass das Hinzugeben + Rühren + Ausdrücken erfolgreich war (Ja-Fall), so wird im Schritt 2144d (Fig. 8i) die Person 1 angewiesen, die Testkassette 20 zu zeigen und den Inhalt des Behälters (die Probe + Extraktionsflüssigkeit) auf die Testkassette 20 zu tropfen. Das Zeigen und das Tropfen erfolgen zeitgleich mit der Anweisung. Während des Zeigens und Tropfens wird gemäß Schritt 205 das Zeigen und das Tropfen dokumentiert, indem mindestens eine Aufnahme durch die Kamera 31a des Endgeräts 30 von der Person 1 während des Zeigens und Tropfens gemacht wird. Es können auch mehrere, beispielsweise 2, Aufnahmen oder auch ein oder mehrere Videos durch die Kamera 31a des Endgeräts 30 von der Person 1 während des Zeigens und Tropfens gemacht werden. Diese Aufnahmen werden in einer Produktdokumentation im Datensatz 321 in der Speichereinheit 32 des Endgeräts 30 gespeichert.

Anschließend wird das Verfahren mit dem Schritt 203e fortgeführt, es wird auf die Ausführungen zum Spuck-Test verwiesen.

Wird im gemeinsamen Auswahlbildschirm gemäß Schritt 199 der Fog. 8c eine Validierung eines bereits durchgeführten Antigentests auf SARS-CoV-2 (Spuck-Test oder Abstrich-Test) ausgewählt, so wird das Verfahren gemäß Fig. 8j und 8k durchlaufen (Schaltfläche 300 in Fig. 8j).

Zunächst wird mit Schritt 3001 der Personendatensatz 320 mit der Person 1 (die sich vor dem Endgerät 1 befindet) verglichen. Ergibt der Vergleich im Schritt 3001, dass der Personendatensatz 320 nicht mit der Person 1 vor dem Endgerät 30 übereinstimmt (Vergleich der Biometriedaten und/oder der benutzerbezogenen Daten) (Nein-Fall), so wird auf der Anzeigeeinheit 34 des Endgeräts 30 eine Mitteilung angezeigt, dass eine nicht autorisierte Person 1 eine Validierung wünscht, was nicht möglich ist. Die Mitteilung könnte lauten "Ungültiger Nutzer zum Darlegen des Tests"

Ergibt der Vergleich im Schritt 3001, dass der Personendatensatz 320 mit der Person 1 vor dem Endgerät 30 übereinstimmt (Vergleich der Biometriedaten und/oder der benutzerbezogenen Daten) (Ja-Fall), so wird von dem Endgerät 30 eine Validierungsanfrage im Schritt 301 erzeugt. Auf Basis dieser Validierungsanfrage wird im Hintergrundsystem 40 ein Validierungstoken 440 im Schritt 303 erzeugt. Zudem wird in der Datenbank 41 des Hintergrundsystems 40 die Eigenschaften des Tokens mit dem Validierungstoken 440 verknüpft. Der Validierungstoken 440 wird im Schritt 304 an das Endgerät 30 übertragen.

Auf Basis des erhaltenen Validierungstoken 440 wird im Schritt 305 ein QR-Code erstellt, der mit den Eigenschaften des Tokens verknüpft ist. Beispielsweise ist dieser QR-Code nur für eine gewisse Zeitdauer gültig (bspw. 10 Minuten). Auf der Anzeigeeinheit 34 des Endgeräts 30 wird das Prozedurergebnis anhand des Datensatzes 321 ggf. mit der Prozedurdokumentation angezeigt. Bei der angezeigten Prozedurdokumentation, die auf der Anzeigeeinheit 34 im Verfahren 300 angezeigt wird, kann die Auswerteeinheit 33 des Endgeräts 30 per Zufallsprinzip eine Auswahl der während des Nachverfolgen-Verfahrens 200 aufgenommenen Bilder und/oder Videos treffen und diese der Prüfinstanz 2 auf Verlangen vorzeigen.

Der QR-Code und das Prozedurergebnis wird einer Prüfinstanz im Schritt 306 angezeigt. Dieses Anzeigen erfolgt optisch. Dazu liest die Prüfinstanz 2 beispielsweise den QR-Code mittels einer Kamera oder eines Lesegeräts der Prüfinstanz aus. Im Schritt 307a wird geprüft, ob der Validierungstoken 440 gültig ist. Ergibt die Prüfung im Schritt 307a, dass der Validierungstoken 440 nicht gültig ist (Nein-Fall), dann wird auf der Anzeigeeinheit der Prüfinstanz 2 eine Meldung im Schritt 308a generiert, die angibt, dass der Validierungstoken 440 ungültig ist oder der Validierungstoken 440 außerhalb der Zeitdauer abgefragt wurde.

Ergibt die Prüfung im Schritt 307a, dass der Validierungstoken 440 gültig ist (Ja-Fall), dann wird von der Prüfinstanz 2 im Schritt 307b geprüft, ob der Streuwert 430 des Personendatensatzes 320 gültig ist und ob die Prozedur-Identifizierungsnummer des Prozedurdatensatzes 450 gültig ist und ob die Prozedur-Identifizierungsnummer des Prozedurdatensatzes 450 mit dem Streuwert 430 verknüpft ist (also ob der Prozedurdatensatzes 450 den Streuwert 430 aufweist.

Ergibt die Prüfung im Schritt 307b, dass der Streuwert 430 des Personendatensatzes 320 gültig ist und dass die Prozedur-Identifizierungsnummer des Prozedurdatensatzes 450 gültig ist und dass der Prozedurdatensatzes 450 den Streuwert 430 aufweist (Ja-Fall), dann wird im Schritt 308c bei der Prüfinstanz 2 angezeigt, dass das Testergebnis validiert ist (Validierungsergebnis des Prozedurergebnisses ist positiv) und der Prozedurdatensatz 450 wird angezeigt, bevorzugt wird auch ein Zeitstempel (Zeit und Datum) des Antigentests auf SARS-CoV-2oder ein Status des Antigentests auf SARS-CoV-2als Prozedurinformation, bevorzugter das Testergebnis des Antigentests auf SARS-CoV-2 gemäß Hintergrundsystem 40 angezeigt.

Entsprechende Einheiten der Systeme 1000, 2000, 3000 der Fig. 2, 4 und 6 sind dazu eingerichtet, diese Verfahrensschritte durchzuführen. Das Zusammenwirken der System 1000, 2000, 3000 gemäß der Fig. 2, 4 und 6 wird nun nochmals anhand von Fig.8a-i beschrieben.

Gemäß Fig. 8a ist die Auswerteeinheit 33 des Endgeräts 30 im Schritt 1003 dazu eingerichtet, zu prüfen, ob ein Personenprofil (=Personendatensatz 320) im Speicher 32 des Endgeräts 30 vorhanden ist.

Stellt die Auswerteeinheit 33 des Endgeräts 30 im Schritt 1003 festgestellt, dass ein Personenprofil im Speicher 32 des Endgeräts 30 vorhanden ist (Ja-Fall), so ist die Auswerteeinheit 33 des Endgeräts 30 dazu eingerichtet, das System 1000 zum Einrichten eines Personenprofils zu überspringen/auszulassen.

Die Auswerteeinheit 33 des Endgeräts 30 ist in einem optionalen Schritt 1004 dazu eingerichtet, zu prüfen, ob (erstens) ein Quarantäne-Ende-Datum im Personendatensatz 320 der Speichereinheit 32 des Endgeräts 30 vorhanden ist und ob (zweitens) dieses Quarantäne-Ende-Datum nach dem aktuellen Datum liegt. Das aktuelle Datum ist beispielsweise das Datum der Durchführung dieses Verfahrens.

Die Auswerteeinheit 33 des Endgeräts 30 ist dazu eingerichtet, im optionalen Schritt 1004 festzustellen, dass ein Quarantäne-Ende-Datum im Personendatensatz 320 vorhanden ist und dass dieses Quarantäne-Ende-Datum nach dem aktuellen Datum liegt (Ja-Fall). In diesem Ja-Fall muss die Person 1 weiter in Quarantäne bleiben. Sodann ist die Anzeigeeinheit 34 des Endgeräts dazu eingerichtet, im optionalen Schritt 1005 eine Mitteilung auf der Anzeigeeinheit 34 des Endgeräts 30 anzeigen. Diese Mitteilung im optionalen Schritt 105 weist die Person 1 daraufhin, dass die Person 1 weiterhin in Quarantäne sein muss. Ein Antigentest auf SARS-CoV-2 muss bei dieser Person 1 nicht durchgeführt werden, denn egal, wie das Ergebnis dieses Antigentests auf SARS-CoV-2 ist, die Person darf sich nicht in der Öffentlichkeit aufhalten. Mit den optionalen Schritten 1004 und 1005 kann eine Testkassette (als Testobjekt 20) eingespart werden. Nachdem die Anzeigeeinheit 34 des Endgeräts die Mitteilung im optionalen Schritt 1005 angezeigt hat, ist die Auswerteeinheit des Endgeräts 30 dazu eingerichtet, das Verfahren gemäß Fig. 8a-k zu beenden.

Die Auswerteeinheit 33 des Endgeräts 30 ist dazu eingerichtet, im optionalen Schritt 1004 festzustellen, dass entweder kein Quarantäne-Ende-Datum im Personendatensatz 320 vorhanden ist oder dass ein vorhandenes Quarantäne-Ende-Datum vor dem aktuellen Datum liegt (Nein-Fall). In diesem Nein-Fall ist die Anzeigeeinheit 34 des Endgeräts dazu eingerichtet, einen Startbildschirm anzuzeigen. Das Endgerät 30 ist dann dazu eingerichtet, eine Benutzereingabe der Person 1 zu erfassen, mit der die Durchführung des Verfahrens 200 (siehe auch Fig. 3) und/oder des Verfahrens 300 (siehe auch Fig. 5) gemäß Fig. 8c ausgewählt wird.

Stellt die Auswerteeinheit 33 des Endgeräts 30 im Schritt 1003 festgestellt, dass kein Personenprofil im Speicher 32 des Endgeräts 30 vorhanden ist (Nein-Fall), so ist die Auswerteeinheit 33 des Endgeräts 30 dazu eingerichtet, das Verfahren 100 durchzuführen. was mit dem System 1000 zum Einrichten eines Personenprofils erfolgt.

Die Anzeigeeinheit 34 des Endgeräts ist in einem optionalen Schritt 1001 dazu eingerichtet, der Person 1 (erstens) auf der Anzeigeeinheit 34 des Endgeräts 30 die AGB anzuzeigen und (zweitens) der Person 1 auf der Anzeigeeinheit 34 des Endgeräts 30 anzuzeigen, dass diese AGB anzunehmen sind.

Die Auswerteeinheit 33 des Endgeräts 30 ist dazu eingerichtet, im optionalen Schritt 1002 zu prüfen, ob die Person 1 durch eine jeweils entsprechende Benutzereingabe auf der Eingabeeinheit 35 des Endgeräts 30 die AGB ablehnt oder das Verfahren verlassen möchte. Die Auswerteeinheit 33 des Endgeräts 30 ist dazu eingerichtet, im optionalen Schritt 1002 festzustellen, dass die Person 1 durch entsprechende Benutzereingabe auf der Eingabeeinheit 35 des Endgeräts 30 die AGB ablehnt oder das Verfahren verlassen möchte (Ja-Fall). Bei dieser festgestellten Benutzereingaben wird das Verfahren gemäß Fig. 8a-k beendet.

Die Auswerteeinheit 33 des Endgeräts 30 ist dazu eingerichtet, im optionalen Schritt 1002 festzustellen, dass die Person 1 durch eine jeweils entsprechende Benutzereingabe auf der Eingabeeinheit 35 des Endgeräts 30 die AGB nicht ablehnt oder das Verfahren nicht verlassen möchte (Nein-Fall). Die Auswerteeinheit 33 des Endgeräts 30 ist dann dazu eingerichtet, das Verfahren 100 basierend auf Fig. 1. bzw. das System 1000 basierend auf Fig. 2 auszuführen, insbesondere durch die Schritte 101 und 102, siehe gestricheltes Rechteck.

Die Auswerteeinheit 33 des Endgeräts 30 ist dazu eingerichtet, im Schritt 1011 eine Mitteilung auf der Anzeigeeinheit 34 des Endgeräts 30 anzuzeigen, mit der die Person 1 dazu aufgefordert wird, einen Zugriff auf eine Kamera 31a des Endgeräts 30 zu erlauben und ein gültiges Identifizierungsdokument 10 der Person 1 mit Vorderseite 12 und Rückseite 13 zu zeigen. Diese Mitteilung kann Schritt für Schritt angezeigt werden und vor dem Anzeigen des nächsten Schritts wird von der Person der jeweilige Schritt durchgeführt.

Die Erfassungseinheit 31 (hier eine Kamera 31a) des Endgeräts 30 ist dazu eingerichtet, im Folgeschritt 1021 das gezeigte Identifizierungsdokument 10 zu erfassen (jeweils Vorder- und Rückseite) und die Auswerteeinheit 33 ist dazu eingerichtet, eine OCR-Erkennung auf das erfasste Identifizierungsdokument 10 durchzuführen, um die personenbezogene Daten 15 des Identifizierungsdokuments 10 für den Personendatensatz 320 zu erhalten. In diesem Schritt 1021 kann die Kamera 31a des Endgeräts 30 auch dazu eingerichtet sein, ein Lichtbild 14 des im Schritt 1011 gezeigten Identifizierungsdokument 10 zu erfassen, um Biometriedaten für den Personendatensatz 320 zu erhalten.

Die Auswerteeinheit 33 des Endgeräts 30 ist dazu eingerichtet, im optionalen Schritt 1012 die Person 1 mit einer Mitteilung auf der Anzeigeeinheit 34 des Endgeräts 30 dazu aufzufordern, die Frontkamera 31a des Endgeräts 30 zu aktivieren und ein Bild von dem Gesicht 1a der Person 1 zur Verifizierung eines Lichtbilds 14 des Identifizierungsdokuments 10 zu erstellen. Die Kamera 31a des Endgeräts 30 ist dazu eingerichtet, im optionalen Folgeschritt 1022 das Gesicht 1a der Person zu erfassen, um Biometriedaten für den Personendatensatz 320 zu erhalten. Die Auswerteeinheit 33 des Endgeräts 30 ist dazu eingerichtet, diese erhaltenen Biometriedaten des erfassten Gesichts 1a zunächst mit den Biometriedaten des Lichtbilds 14 des Identifizierungsdokument 10 zu verknüpfen.

Das Endgerät 30 ist dazu eingerichtet, eine Kommunikation in den Schritten 1011, 1021, 1012 und 1022 über einen verschlüsselten Datenstrom ("data stream") 1013 in Zusammenarbeit mit einem externen Dienstleister durchzuführen. Dabei werden die Videodaten und die OCR-Daten zur Absicherung des so gewonnen Personendatensatz 320 verschlüsselt. Ergibt der Vergleich, dass die Biometriedaten mit ausreichender Genauigkeit übereinstimmen, werden die Biometriedaten von dem Lichtbild 14 akzeptiert und als Teil in dem Benutzerdatensatz 320 vorgesehen.

Die Auswerteeinheit 33 des Endgeräts 30 ist dazu eingerichtet, im optionalen Schritt 1023 zu prüfen, ob die Zeichenerkennung und die Lichtbilderkennung der Schritte 1011, 1021, 1012 und 1022 erfolgreich war (also ob ein gültiger Benutzerdatensatz 320 erhalten wurde). Die Auswerteeinheit 33 des Endgeräts 30 ist dazu eingerichtet, die erhaltenen Biometriedaten des erfassten Gesichts 1a mit den Biometriedaten des Lichtbilds 14 des Identifizierungsdokument 10 zu vergleichen.

Die Auswerteeinheit 33 des Endgeräts 30 ist dazu eingerichtet, im optionalen Schritt 1023 festzustellen, dass die erhaltenen Biometriedaten des erfassten Gesichts 1a nicht mit ausreichender Genauigkeit mit den Biometriedaten des Lichtbilds 14 des Identifizierungsdokument 10 übereinstimmen oder dass die Zeichenerkennung und die Lichtbilderkennung der Schritte 1011, 1021, 1012 und 1022 nicht erfolgreich waren (Nein-Fall), Die Auswerteeinheit 33 des Endgeräts 30 ist in diesem Nein-Fall dazu eingerichtet, im optionalen Schritt 1024 eine Aufforderung an die Person 1 zu erzeugen und diese durch die Anzeigeeinheit 34 des Endgeräts 30 anzuzeigen, dass ein anderes Identifizierungsdokument zu verwenden ist oder dass das Verfahren 100 unter besseren Lichtbedingungen erneut zu versuchen ist (das System 1000 besser ausgeleuchtet werden muss). Nach der Anzeige der Mitteilung im optionalen Schritt 1024 ist das Endgerät 30 dazu eingerichtet, das Verfahren gemäß Fig. 8a-k zu beenden.

Die Auswerteeinheit 33 des Endgeräts 30 ist dazu eingerichtet, im optionalen Schritt 1023 durch festzustellen, dass die erhaltenen Biometriedaten des erfassten Gesichts 1a mit ausreichender Genauigkeit mit den Biometriedaten des Lichtbilds 14 des Identifizierungsdokument 10 übereinstimmen oder dass die Zeichenerkennung und die Lichtbilderkennung der Schritte 1011, 1021, 1012 und 1022 erfolgreich waren (Ja-Fall). Die Auswerteeinheit 33 des Endgeräts 30 ist in diesem Ja-Fall dazu eingerichtet, im Schritt 1025 der Benutzerdatensatz 320 fertigzustellen.

Optional wird der Person 1 in diesem Schritt 1025 gestattet, die Anschrift als personenbezogenes Datum vor dem Abspeichern 103 zu editieren. Der Schritt 1025 entspricht den Schritten 103, 104 und 105 der Fig. 1, auf weiterführende Äußerungen wird verzichtet.

Das Ergebnis der Schritte 1011, 1021, 1012, 1022, 1023 und 1025 ist ein Benutzerdatensatz 320, der Biometriedaten und personenbezogene Daten 15 basierend auf dem Identifizierungsdokument 10 (und ggf. der Verifizierung der Person 1 in Schritt 1012) umfasst. Dieser Benutzerdatensatz 320 kann in einer Speichereinheit 32 des Endgeräts 30 verschlüsselt abgelegt werden.

Gemäß Fig. 8c ist die Auswerteeinheit 33 des Endgeräts 30 im optionalen Schritt 199 dazu eingerichtet, ein Auswahlbildschirm für die Nachverfolgung des Antigentests auf SARS-CoV-2 gemäß Verfahren 200 und für die Validierung des durchgeführten Antigentests auf SARS-CoV-2 gemäß Verfahren 300 auf der Anzeigeeinheit 34 des Endgeräts 30 anzuzeigen. Die Auswerteeinheit 33 des Endgeräts 30 ist im optionalen Schritt 199 dazu eingerichtet, eine Benutzerauswahl an einer Benutzereingabeeinheit 35 des Endgeräts 30 zu erfassen, woraufhin entweder das Verfahren 200 oder das Verfahren 300 ausgewählt wird.

Wählt die Person 1 im Schritt 199 die Durchführung eines neuen Antigentests auf SARS-CoV-2, so wird das Verfahren 200 gemäß Fig. 3 und 4 ausgeführt. Die Auswerteeinheit 33 des Endgeräts 30 ist dann dazu eingerichtet, im optionalen Schritt 2041 ein Einleitungsvideo auf der Anzeigeeinheit 34 des Endgeräts 30 anzuzeigen. Die Auswerteeinheit 33 des Endgeräts 30 ist dazu eingerichtet zu prüfen, ob die Umweltbedingungen (Licht, Raum) den Anforderungen an einen Antigentest auf SARS-CoV-2 und den Anforderungen für dieses Verfahren 200 erfüllen.

Wird im Schritt 2041 durch die Auswerteeinheit 34 des Endgeräts 30 festgestellt, dass die Umweltbedingungen ungeeignet sind, um den Antigentest auf SARS-CoV-2 durchzuführen oder um dessen Durchführungen nachverfolgen zu können (bspw. aufgrund schlechter Lichtverhältnisse oder aufgrund großem Bildrauschanteil oder durch viel Bewegung im Kamerabild oder etc.) (Verlassen-Fall), so ist die Auswerteeinheit 34 des Endgeräts 30 im Folgeschritt 2049a (in Fig. 8g) dazu eingerichtet, der Person 1 das Prozedurergebnis "ungültiger Test" auf der Anzeigeeinheit 35 anzuzeigen, dieses Prozedurergebnis in der Speichereinheit 32 des Endgeräts zu dokumentieren (bspw. in dem Prozedur-Identifizierungsdatensatz 321) und das System 2000 über eine "Verlassen" Schaltfläche zu beenden.

Wird im Schritt 2041 durch die Auswerteeinheit 33 des Endgeräts 30 festgestellt, dass die Umweltbedingungen geeignet sind, um den Test durchzuführen und dessen Durchführungen nachverfolgbar sind (Ja-Fall), so ist die Auswerteeinheit 34 des Endgeräts 30 im optionalen Folgeschritt 2042 dazu eingerichtet, der Person 1 eine Anleitung zum Einrichten des Endgeräts 30 auf der Anzeigeeinheit 34 des Endgeräts 30 anzuzeigen. Dabei wird die Frontkamera 31a des Endgeräts durch eine Benutzereingabe aktiviert, um die richtige Kameraperspektive einzustellen und sich so vor das Endgerät 30 zu positionieren, dass die Kamera 31a das Gesicht 1a der Person 1 erfassen kann. Im darauffolgenden Schritt 2043 ist die Auswerteeinheit 33 des Endgeräts 30 dazu eingerichtet, festzustellen, ob die Einrichtung des Endgeräts 30 erfolgreich war.

Die Auswerteeinheit 33 des Endgeräts 30 ist im optionalen Schritt 2043 dazu eingerichtet, festzustellen, dass die Einrichtung des Endgeräts 30 nicht erfolgreich war. Dann ist die Auswerteeinheit 34 des Endgeräts 30 dazu eingerichtet, den gemeinsamen Auswahlbildschirm im Schritt 199 wieder zu zeigen (Fig. 8c).

Die Auswerteeinheit 33 des Endgeräts 30 ist im optionalen Schritt 2043 dazu eingerichtet, festzustellen, dass die Einrichtung des Endgeräts 30 erfolgreich war. Dann ist die Auswerteeinheit 33 des Endgeräts 30 dazu eingerichtet, im Schritt 201 zu versuchen, die Person 1 zu identifizieren. Die Auswerteeinheit 33 des Endgeräts 30 ist dazu eingerichtet, das aktuelle Kamerabild des Endgeräts 1 zu erfassen (also das Gesicht 1a der Person 1) und mit den Biometriedaten des Personendatensatzes 320 zu verglichen (siehe auch Ausführungen zu Fig. 3).

Im folgenden Schritt 2014 ist die Auswerteeinheit 33 des Endgeräts 30 dazu eingerichtet, zu prüfen, ob die Identifizierung der Person 1 erfolgreich war. Ergibt diese Prüfung durch die Auswerteeinheit im Schritt 2014, dass die Identifizierung der Person 1 nicht erfolgreich war (Verlassen-Fall), so ist die Auswerteeinheit 34 des Endgeräts 30 dazu eingerichtet, den gemeinsamen Auswahlbildschirm im Schritt 199 wieder zu zeigen (Fig. 8c).

Die Auswerteeinheit 33 des Endgeräts 30 ist im Schritt 2014 dazu eingerichtet, festzustellen, dass die Identifizierung der Person 1 erfolgreich war (Ja-Fall). Dann ist die Auswerteeinheit 33 des Endgeräts 30 im optionalen Folgeschritt 2044a dazu eingerichtet, eine Anleitung zum Vorbereiten des Tests auf einer Anzeigeeinheit 33 anzuzeigen. Dieses Vorbereiten kann auf verschiedene Weise erfolgen, wovon zwei in der Fig. 10a und 10b dargestellt sind.

Die Auswerteeinheit 33 des Endgeräts 30 ist im darauffolgenden Schritt 202 (Fig. 8d) dazu eingerichtet, zu versuchen, die Testkassette 20 (das Testobjekt) zu identifizieren. Dabei ist Auswerteeinheit 34 des Endgeräts 30 dazu eingerichtet, einen QR-Code 210 der Testkassette 20 mittels der Erfassungseinheit 31 des Endgeräts 30 zu erfassen und zu prüfen, ob eine im QR-Code 210 einkodierte Prozedurseriennummer mit einer Prozedurseriennummer eines Prozedurdatensatzes 450 des Hintergrundsystems 40 übereinstimmt. Zudem ist die Auswerteeinheit 33 des Endgeräts 30 in dem Schritt 202 dazu eingerichtet, zu prüfen, ob ein Status des Prozedurdatensatzes 450 auf "hergestellt" gesetzt ist und ob dieser Status auf den Status "in Verwendung" gesetzt werden kann. Zudem kann in dem Schritt 202 geprüft werden, ob der Person 1 der Streuwert 430 des Personendatensatzes 320 des Prozedurdatensatzes 450 zugeordnet werden kann.

Die Auswerteeinheit 33 des Endgeräts 30 ist im Schritt 202 dazu eingerichtet, festzustellen, dass die Identifizierung der Testkassette 20 nicht erfolgreich war (Nein-Fall). Dann ist die Auswerteeinheit 34 des Endgeräts 30 dazu eingerichtet, im Folgeschritt 2045a die Person durch eine Mitteilung auf der Anzeigeeinheit 34 des Endgerät 30 darüber zu informieren, dass die Testkassette 20 dieses Antigentests auf SARS-CoV-2 nicht erkannt wurde oder dass die vorliegende Testkassette 20 einen ungültigen/unzulässigen QR-Code aufweist. Darauffolgend wird der Auswahlbildschirm im Schritt 199 gezeigt (Fig. 8c).

Die Auswerteeinheit 33 des Endgeräts 30 ist im Schritt 202 dazu eingerichtet, festzustellen, dass die Identifizierung der Testkassette 20 erfolgreich war (Ja-Fall). Dann kann die Testprozedur durchgeführt werden, denn sowohl die Person 1 als auch das Testobjekt 20 konnten identifiziert werden (Schritte 201, 202). Somit kann jetzt die Nachverfolgung der Testprozedur gemäß Schritt 203 (hier konkret die Schritte 203a-h) erfolgen. In dem optionalen Schritt 2030 der Fig. 8d ist das Endgerät 30 dazu eingerichtet, verschiedene Testprozeduren für einen Virusnachweistest, hier Antigentest auf SARS-CoV-2 nachzuverfolgen, wovon eine Testprozedur auszuwählen ist. Hier stellvertretend wird der Abstrich-Test und der Spuck-Test beschrieben, andere Tests zur Untersuchung der Körperflüssigkeit der Person 1 sind ebenfalls denkbar.

Das Nachverfolgen der Testprozedur durch das Endgerät 30 wird zunächst anhand des Spuck-Tests in den Figuren 8d-8g beschrieben. In den Figuren 8j und 8k wird dann auf den Abstrichtest (=Stäbchentest) gemäß Schritt 2100 eingegangen.

Die Auswerteeinheit 33 des Endgeräts 30 ist im optionalen Schritt 2046 dazu eingerichtet, die Person 1 mittels der Anzeigeeinheit 34 des Endgeräts 30 zu befragen, ob sie in den letzten 10 Minuten gegessen, getrunken oder geraucht hat.

Die Auswerteeinheit 33 des Endgeräts 30 ist im optionalen Schritt 2046 dazu eingerichtet, durch eine entsprechende Personeneingabe in der Eingabeeinheit 35 des Endgeräts 30 festzustellen, dass die Person 1 in den letzten 10 Minuten gegessen, getrunken oder geraucht hat (Ja-Fall). Sodann ist die Auswerteeinheit 33 des Endgeräts 30 im optionalen Schritt 2045b dazu eingerichtet, die Person 1 mittels der Anzeigeeinheit 34 des Endgeräts 30 darüber zu informieren, dass der Test zu einem späteren Zeitpunkt zu wiederholen ist. Zudem kann der Status des Prozedurdatensatzes 450 in dem Hintergrundsystem 40 von dem Status "in Verwendung" auf den Status "hergestellt" zurückgesetzt werden. Anschließend wird der Auswahlbildschirm in Schritt 199 wieder gezeigt (Fig. 8c).

Die Auswerteeinheit 33 des Endgeräts 30 ist im optionalen Schritt 2046 dazu eingerichtet, durch eine entsprechende Personeneingabe in der Eingabeeinheit 35 des Endgeräts 30 festzustellen, dass die Person 1 in den letzten 10 Minuten nicht gegessen, nicht getrunken und auch nicht geraucht hat (Nein-Fall). Sodann ist die Auswerteeinheit 33 des Endgeräts 30 im optionalen Schritt 2044b (Fig. 8e) dazu eingerichtet, die Person 1 anzuweisen, (erstens) den Test zusammenzubauen und (zweitens) den zusammengebauten Test in die Kamera 31a des Endgeräts 30 zu halten ist. Das Zusammenbauen kann dann zeitgleich mit der Anweisung erfolgen. Die Anweisung kann ein Animationsvideo auf der Anzeigeeinheit 34 des Endgeräts 30 sein. Die Anweisung kann durch "Augmented Reality" unterstützt werden. Während des Zusammenbauens ist das Endgerät 30 gemäß Schritt 205 dazu eingerichtet, den Zusammenbau zu dokumentieren, indem die Auswerteeinheit 33 des Endgeräts dazu eingerichtet ist, mindestens eine Aufnahme durch die Kamera 31a des Endgeräts 30 von der Person 1 während des Zusammenbauens zu erfassen. Es können auch mehrere, beispielsweise 2, Aufnahmen oder auch ein oder mehrere Videos durch die Kamera 31a des Endgeräts 30 von der Person 1 während des Zusammenbauens erfasst werden. Die Auswerteeinheit 33 des Endgeräts ist dazu eingerichtet, die Aufnahmen in einer Produktdokumentation im Datensatz 321 in der Speichereinheit 32 des Endgeräts 30 zu speichern.

Die Auswerteeinheit 33 des Endgeräts 30 ist im optionalen Schritt 203a dazu eingerichtet, zu prüfen, ob das Zusammenbauen im Schritt 2044b erfolgreich war. Dazu müssen sowohl das Gesicht 1a also auch beide Hände 1b der Person 1 ständig im vordefinierten Bereich (bspw. ein Rahmen der Anzeigeeinheit 34 oder ein gezeichneter Bereich ("Augmented Reality") innerhalb des erfassbaren Bereichs der Kamera 31a, auf der Anzeigeeinheit 34 dargestellt) sein und dürfen diesen Bereich nie verlassen haben. Zudem muss der Zusammenbau dem Zusammenbau des Test-Typs (Objektidentifizierungsdatensatz 420) entsprechen, was von der Auswerteeinheit 33 des Endgeräts durch Vergleichen festgestellt wird.

Die Auswerteeinheit 33 des Endgeräts 30 ist im optionalen Schritt 203a dazu eingerichtet, festzustellen, dass das Zusammenbauen nicht erfolgreich war (Nein-Fall). Sodann ist die Auswerteeinheit 33 des Endgeräts 30 im Schritt 2045b (Fig. 8d) dazu eingerichtet, die Person 1 mittels der Anzeigeeinheit 34 des Endgeräts 30 darüber zu informieren, dass der Antigentest auf SARS-CoV-2 zu einem späteren Zeitpunkt zu wiederholen ist. Zudem kann der Status des Prozedurdatensatzes 450 in dem Hintergrundsystem 40 wieder von dem Status "in Verwendung" auf den Status "hergestellt" zurückgesetzt werden. Anschließend wird der Auswahlbildschirm in Schritt 199 wieder gezeigt (Fig. 8c).

Die Auswerteeinheit 33 des Endgeräts 30 ist im optionalen Schritt 203a dazu eingerichtet, festzustellen, dass das Zusammenbauen erfolgreich war (Ja-Fall). Sodann ist die Auswerteeinheit 33 des Endgeräts 30 im Schritt 2048a (Fig. 8e) dazu eingerichtet, die Person 1 mit der Anzeigeeinheit 34 des Endgeräts 30 anzuweisen, eine Speichelprobe in einen Behälter als Hilfsmittel 50 (bis zu einer Fülllinie) abzugeben und mit einer Spracheingabe zu bestätigen, sobald er zur Speichelproben-Abgabe bereit ist. Ab dem Zeitpunkt der Spracheingabe wird (erstens) die Abgabe der Speichelprobe durch die Person 1 mittels der Erfassungseinheit erfasst und mit der Auswerteeinheit 33 ausgewerteten (= nachverfolgt). Während der Abgabe der Speichelprobe ist das Endgerät 30 gemäß Schritt 205 dazu eingerichtet, den Zusammenbau zu dokumentieren, indem die Auswerteeinheit 33 des Endgeräts dazu eingerichtet ist, mindestens eine Aufnahme durch die Kamera 31a des Endgeräts 30 von der Person 1 während des Zusammenbauens zu erfassen. Es können auch mehrere, beispielsweise 2, Aufnahmen oder auch ein oder mehrere Videos durch die Kamera 31a des Endgeräts 30 von der Person 1 während der Abgabe der Speichelprobe erfasst werden. Die Auswerteeinheit 33 des Endgeräts ist dazu eingerichtet, die Aufnahmen in einer Produktdokumentation im Datensatz 321 in der Speichereinheit 32 des Endgeräts 30 zu speichern.

Die Auswerteeinheit 33 des Endgeräts 30 ist im Schritt 203b dazu eingerichtet, zu prüfen, ob die Abgabe der Speichelprobe erfolgreich war. Dazu müssen sowohl das Gesicht 1a also auch beide Hände 1b der Person 1 ständig im vordefinierten Bereich (bspw. im Bilderrahmen der Anzeigeeinheit 34) sein und dürfen diesen Bereich nie verlassen haben, was von der Auswerteeinheit 33 des Endgeräts durch Nachverfolgen der Position des Gesichts 1a und der Hände 1b festgestellt wird. Zudem muss die Abgabe der Speichelprobe bis zur Fülllinie (Objekteigenschaft im Objektidentifizierungsdatensatz 420) in den Behälter erfolgen, was von der Auswerteeinheit 33 des Endgeräts durch Nachverfolgen der Füllmenge der Speichelprobe im Behälter und Erkennen des Erreichens der Fülllinie des Behälters festgestellt wird. Der Behälter muss ständig im vordefinierten Bereich (bspw. im Bilderrahmen der Anzeigeeinheit 34) sein und darf diesen Bereich nie verlassen, was von der Auswerteeinheit 33 des Endgeräts durch Nachverfolgen des Behälters festgestellt wird.

Die Auswerteeinheit 33 des Endgeräts 30 ist im Schritt 203b dazu eingerichtet, festzustellen, dass die Abgabe der Speichelprobe nicht erfolgreich war (Nein-Fall). Sodann ist im Schritt 2045c (Fig. 8f) die Auswerteeinheit 33 des Endgeräts 30 dazu eingerichtet, eine Meldung auf der Anzeigeeinheit 34 des Endgeräts 30 zu erzeugen, mit der die Person 1 darüber informiert wird, dass der Test wegen Verstoßes gegen die Testvorschriften oder wegen Abbruch fehlgeschlagen ist. Der Test erhält das Prozedurergebnis "ungültig". Dieses Prozedurergebnis wird in den Prozedurdatensatz 450 des Hintergrundsystems 40 eingetragen. Anschließend wird der Auswahlbildschirm in Schritt 199 wieder gezeigt (Fig. 8c).

Die Auswerteeinheit 33 des Endgeräts 30 ist im Schritt 203b dazu eingerichtet, festzustellen, dass die Abgabe der Speichelprobe erfolgreich war (Ja-Fall). Sodann ist im Schritt 2048b (Fig. 8e) die Auswerteeinheit 33 des Endgeräts 30 dazu eingerichtet, die Person 1 anzuweisen, eine Extraktionsflüssigkeit in den Behälter mit der Speichelprobe einzubringen, den Behälter zu schließen und zu bestätigen, sobald er dazu bereit ist. Mit der Bestätigung (Sprache oder anders) wird (erstens) das Hinzugeben nachverfolgt. Während der Hinzugabe wird gemäß Schritt 205 (zweitens) das Hinzugeben dokumentiert, indem mindestens eine Aufnahme durch die Kamera 31a des Endgeräts 30 von der Person 1 während dem Einbringen der Extraktionsflüssigkeit in den Behälter gemacht wird. Es können auch mehrere, beispielsweise 2, Aufnahmen oder auch ein oder mehrere Videos durch die Kamera 31a des Endgeräts 30 von der Person 1 während dem Einbringen der Extraktionsflüssigkeit in den Behälter gemacht werden. Diese Aufnahmen werden in einer Produktdokumentation im Datensatz 321 in der Speichereinheit 32 des Endgeräts 30 gespeichert.

Die Auswerteeinheit 33 des Endgeräts 30 ist im Schritt 203c dazu eingerichtet, zu prüfen, ob das Einbringen der Extraktionsflüssigkeit in den Behälter erfolgreich war. Dazu müssen sowohl das Gesicht 1a also auch beide Hände 1b der Person 1 ständig im vordefinierten Bereich (bspw. im Bilderrahmen der Anzeigeeinheit 34) sein und dürfen diesen Bereich nie verlassen haben, was von der Auswerteeinheit 33 des Endgeräts durch Nachverfolgen der Position des Gesichts 1a und der Hände 1b festgestellt wird. Der Behälter muss ständig im vordefinierten Bereich (bspw. im Bilderrahmen der Anzeigeeinheit 34) sein und darf diesen Bereich nie verlassen, was von der Auswerteeinheit 33 des Endgeräts durch Nachverfolgen der Position Behälters festgestellt wird.

Die Auswerteeinheit 33 des Endgeräts 30 ist im Schritt 203c dazu eingerichtet, festzustellen, dass das Hinzugeben der Extraktionsflüssigkeit nicht erfolgreich war (Nein-Fall). Sodann ist die Auswerteeinheit 33 des Endgeräts 30 dazu eingerichtet, im Schritt 2045c (Fig. 8f) die Person 1 mittels der Anzeigeeinheit 34 des Endgeräts 30 darüber zu informieren, dass der Test wegen Verstoßes gegen die Testvorschriften oder wegen Abbruch fehlgeschlagen ist. Der Test erhält das Prozedurergebnis "ungültig". Dieses Prozedurergebnis wird in den Prozedurdatensatz 450 des Hintergrundsystems 40 eingetragen. Anschließend wird der Auswahlbildschirm in Schritt 199 wieder gezeigt (Fig. 8c).

Die Auswerteeinheit 33 des Endgeräts 30 ist im Schritt 203c dazu eingerichtet, festzustellen, dass das Hinzugeben der Extraktionsflüssigkeit erfolgreich war (Ja-Fall), Sodann ist die Auswerteeinheit 33 des Endgeräts 30 dazu eingerichtet, im Schritt 2048c (Fig. 8f) die Person 1 anzuweisen, den Behälter mit der Extraktionsflüssigkeit und der Speichelprobe für 5 Sekunden zu schütteln. Das Schütteln erfolgt zeitgleich mit der Anweisung. Während des Schüttelns wird gemäß Schritt 205 das Schütteln dokumentiert, indem mindestens eine Aufnahme durch die Kamera 31a des Endgeräts 30 von der Person 1 während des Schüttelns gemacht wird. Es können auch mehrere, beispielsweise 2, Aufnahmen durch die Kamera 31a des Endgeräts 30 von der Person 1 während des Schüttelns gemacht werden. Diese Aufnahmen werden in einer Produktdokumentation im Datensatz 321 in der Speichereinheit 32 des Endgeräts 30 gespeichert.

Die Auswerteeinheit 33 des Endgeräts 30 ist im Schritt 203d dazu eingerichtet, zu prüfen, ob das Schütteln erfolgreich war. Dazu müssen sowohl das Gesicht 1a also auch beide Hände 1b der Person 1 ständig im vordefinierten Bereich (bspw. im Bilderrahmen der Anzeigeeinheit 34) sein und dürfen diesen Bereich nie verlassen haben, was von der Auswerteeinheit 33 des Endgeräts durch Nachverfolgen der Position des Gesichts 1a und der Hände 1b festgestellt wird. Das Schütteln des Behälters durch die Person 1 für mindestens 5 Sekunden wird nachverfolgt. Der Behälter muss ständig im vordefinierten Bereich (bspw. im Bilderrahmen der Anzeigeeinheit 34) sein und darf diesen Bereich nie verlassen, was von der Auswerteeinheit 33 des Endgeräts durch Nachverfolgen der Position des Behälters festgestellt wird.

Die Auswerteeinheit 33 des Endgeräts 30 ist im Schritt 203d dazu eingerichtet, festzustellen, dass das Schütteln nicht erfolgreich war (Nein-Fall). Sodann ist die Auswerteeinheit 33 im Schritt 2045c dazu eingerichtet, die Person 1 mittels der Anzeigeeinheit 34 darüber zu informieren, dass der Test wegen Verstoßes gegen die Testvorschriften oder wegen Abbruch fehlgeschlagen ist. Der Test erhält das Prozedurergebnis "ungültig". Dieses Prozedurergebnis wird in den Prozedurdatensatz 450 des Hintergrundsystems 40 eingetragen. Anschließend wird der Auswahlbildschirm in Schritt 199 wieder gezeigt (Fig. 8c).

Die Auswerteeinheit 33 des Endgeräts 30 ist im Schritt 203d dazu eingerichtet, festzustellen, dass das Schütteln erfolgreich war (Ja-Fall). Sodann ist die Auswerteeinheit 33 im Schritt 2048d dazu eingerichtet, die Person 1 mittels der Anzeigeeinheit 34 anzuweisen, die Testkassette 20 zu zeigen und den Inhalt des geschüttelten Behälters (die Probe + Extraktionsflüssigkeit) auf die Testkassette 20 zu tropfen. Das Zeigen und das Tropfen erfolgen zeitgleich mit der Anweisung. Während des Zeigens und Tropfens wird gemäß Schritt 205 das Zeigen und das Tropfen dokumentiert, indem mindestens eine Aufnahme durch die Kamera 31a des Endgeräts 30 von der Person 1 während des Zeigens und Tropfens gemacht wird. Es können auch mehrere, beispielsweise 2, Aufnahmen oder auch ein oder mehrere Videos durch die Kamera 31a des Endgeräts 30 von der Person 1 während des Zeigens und Tropfens gemacht werden. Diese Aufnahmen werden in einer Produktdokumentation im Datensatz 321 in der Speichereinheit 32 des Endgeräts 30 gespeichert.

Die Auswerteeinheit 33 des Endgeräts 30 ist im Schritt 203e dazu eingerichtet, zu prüfen, ob das Zeigen und das Tropfen erfolgreich war. Dazu müssen sowohl das Gesicht 1a also auch beide Hände 1b der Person 1 ständig im vordefinierten Bereich (bspw. im Bilderrahmen der Anzeigeeinheit 34) sein und dürfen diesen Bereich nie verlassen haben. Die Testkassette 20 muss erkannt werden und muss ständig im vordefinierten Bereich (bspw. im Bilderrahmen der Anzeigeeinheit 34) sein und darf diesen Bereich nie verlassen.

Die Auswerteeinheit 33 des Endgeräts 30 ist im Schritt 203e dazu eingerichtet, festzustellen, dass das Zeigen und das Tropfen nicht erfolgreich war (Nein-Fall). Sodann ist die Auswerteeinheit 33 im Schritt 2045c dazu eingerichtet, die Person 1 mittels Anzeigeeinheit 34 darüber zu informieren, dass der Test wegen Verstoß gegen die Testvorschriften oder wegen Abbruch fehlgeschlagen ist. Der Test erhält das Prozedurergebnis "ungültig". Dieses Prozedurergebnis wird in den Prozedurdatensatz 450 des Hintergrundsystems 40 eingetragen. Anschließend wird der Auswahlbildschirm in Schritt 199 wieder gezeigt (Fig. 8c).

Die Auswerteeinheit 33 des Endgeräts 30 ist im Schritt 203e dazu eingerichtet, festzustellen, dass das Zeigen und das Tropfen erfolgreich war (Ja-Fall). Sodann ist die Auswerteeinheit 33 dazu eingerichtet im Schritt 2047, eine Wartezeit von 10 Minuten auf der Anzeigeeinheit 34 anzuzeigen und beispielsweise als Countdown runtergezählt. Die Person 1 wird informiert, sobald die Wartezeit verstrichen ist.

Einer oder jeder der Schritte 2048a-d kann optional einen Countdown aus. Der Countdown startet beispielsweise mit dem Anzeigen der Aufforderungen auf der Anzeigeeinheit 34 des Endgeräts 30. Wird in dieser Zeit die Anweisung nach Schritten 2048a-d nicht oder falsch befolgt, so wird im Schritt 2045d die Person 1 darüber informiert, dass der Test(-Schritt) wegen Zeitüberschreitung fehlgeschlagen ist. Der Test erhält das Prozedurergebnis "ungültig". Dieses Prozedurergebnis wird in den Prozedurdatensatz 450 des Hintergrundsystems 40 eingetragen. Anschließend wird der Auswahlbildschirm in Schritt 199 wieder gezeigt (Fig. 8c).

Die Auswerteeinheit 33 des Endgeräts 30 ist im Schritt 2044c dazu eingerichtet, mittels der Anzeigeeinheit 34 die Person 1 aufzufordern, ein Foto von der Testkassette 20 zu machen. Dabei kann die Person 1 dazu aufgefordert werden, auf eine Rückkamera des Endgeräts 30 zu schalten. Diese Rückkamera hat (meist) eine bessere Auflösung als die Frontkamera, mit der die Person 1 nachverfolgt wird (Schritte 203a-h). Die Erfassungseinheit 31 des Endgeräts 30 ist eingerichtet dazu, die Testkassette 20 zu erfassen.

Die Auswerteeinheit 33 des Endgeräts 30 ist im Schritt 203f dazu eingerichtet, erstens zu prüfen, ob die Seriennummer der Testkassette 20 mit der im Schritt 202 übereinstimmt. Dazu wird (in Anlehnung an die Schritte 2044a und 202) der QR-Code 210 der Testkassette 20 erfasst und die daraus erhaltene Seriennummer mit der eines Objektdatensatz 450 verglichen. Zudem wird im Schritt 203f (zweitens) geprüft, ob ein oder mehrere Kontrollbalken auf dem Teststreifen der Testkassette 20 zu erkennen ist. Der oder die Kontrollbalken sind ein oder mehrere Sicherheitsmerkmale der Testkassette 20, die immer entstehen, ungeachtet der Speichelprobe.

Die Auswerteeinheit 33 des Endgeräts 30 ist im Schritt 203f dazu eingerichtet, festzustellen, dass entweder die Seriennummer der Testkassette 20 mit der Seriennummer im Schritt 202 nicht übereinstimmt oder dass der oder die Kontrollbalken des Antigentests auf SARS-CoV-2 auf dem Teststreifen der Testkassette 20 nicht zu erkennen ist (Nein-Fall). Sodann ist die Auswerteeinheit 33 des Endgeräts 33 dazu eingerichtet, im Schritt 203g zu prüfen, ob die Seriennummer der Testkassette 20 mit der im Schritt 202 übereinstimmt.

Wird im Schritt 203g durch das Endgerät 30 festgestellt, dass die Seriennummer der Testkassette 20 nicht mit der Seriennummer im Schritt 202 übereinstimmt, dann wird im Schritt 2045e die Person 1 darüber informiert, dass die Seriennummer falsch ist. Der Test erhält das Prozedurergebnis "ungültig", der in den Prozedurdatensatz 450 des Hintergrundsystems 40 eingetragen wird. Anschließend wird der Auswahlbildschirm in Schritt 199 wieder gezeigt (Fig. 8c).

Die Auswerteeinheit 33 des Endgeräts 30 ist im Schritt 203gdazu eingerichtet, festzustellen, dass die Seriennummer der Testkassette 20 mit der Seriennummer im Schritt 202 übereinstimmt. Sodann ist die Auswerteeinheit 33 des Endgeräts 30 im Schritt 2045f dazu eingerichtet, die Person 1 mittels der Anzeigeeinheit 34 darüber zu informieren, dass der oder die Kontrollbalken des Tests auf dem Teststreifen der Testkassette 20 nicht zu erkennen ist (bspw. keine Farbveränderung im zu erwartenden Bereich des Teststreifens). Der Test erhält das Prozedurergebnis "ungültig", der in den Prozedurdatensatz 450 des Hintergrundsystems 40 eingetragen wird. Anschließend wird der Auswahlbildschirm in Schritt 199 wieder gezeigt (Fig. 8c).

Die Auswerteeinheit 33 des Endgeräts 30 ist im Schritt 203g dazu eingerichtet, festzustellen, dass die Seriennummer der Testkassette 20 mit der Seriennummer im Schritt 202 übereinstimmt und dass der oder die Kontrollbalken des Antigentests auf SARS-CoV-2 auf dem Teststreifen der Testkassette 20 zu erkennen ist (Ja-Fall). Sodann wird im Schritt 203h durch das Endgerät 30 geprüft, ob das Prozedurergebnis "positiv" ist.

Die Auswerteeinheit 33 des Endgeräts 30 ist im Schritt 203h dazu eingerichtet, festzustellen, dass das Prozedurergebnis nicht "positiv" ist (Nein-Fall). Sodann ist die Auswerteeinheit 33 im Schritt 2049a dazu eingerichtet, das Prozedurergebnis "negativ" auf der Anzeigeeinheit 34 anzuzeigen. Dieses Prozedurergebnis wird dokumentiert, beispielsweise durch Setzen des Prozedurergebnis auf "Negativ" im Prozedurdatensatz 450 des Hintergrundsystems 40 ggf. auch durch Dokumentieren in der Prozedurdokumentation des Datensatzes 321, beispielsweise auch durch Abspeichern des Fotos aus dem Schritt 2044c in der Prozedurdokumentation der Speichereinheit 32 des Endgeräts 30.

Die Auswerteeinheit 33 des Endgeräts 30 ist im Schritt 203h dazu eingerichtet, festzustellen, dass das Prozedurergebnis "positiv" ist (Ja-Fall). Sodann ist die Auswerteeinheit 33 im Schritt 2049a dazu eingerichtet, das Prozedurergebnis "positiv" auf der Anzeigeeinheit 34 anzuzeigen. Dieses Prozedurergebnis wird dokumentiert, beispielsweise durch Setzen des Prozedurergebnis auf "Positiv" im Prozedurdatensatz 450 des Hintergrundsystems 40 ggf. auch durch Dokumentieren in der Prozedurdokumentation des Datensatzes 321, beispielsweise auch durch Abspeichern des Fotos aus dem Schritt 2044c in der Prozedurdokumentation der Speichereinheit 32 des Endgeräts 30. Zudem erhält die Person 1 eine Anleitung, wie nun weiter vorzugehen ist. Zudem kann im Personendatensatz 320 ein Quarantäne-Ende-Datum auf ein Datum in zwei Wochen ausgehend von dem aktuellen Datum gesetzt werden. Anschließend ist das Verfahren 200 (Nachverfolgen) beendet.

Die Anzeigeeinheit 34 des Endgeräts 30 kann auf dem gemeinsamen Auswahlbildschirm die Nachverfolgung eines Antigentests auf SARS-CoV-2 gemäß Verfahren 200 (System 2000) und die Validierung eines bereits durchgeführten Antigentests auf SARS-CoV-2 gemäß Verfahren 300 (System 3000) zur Auswahl durch die Person 1 anzeigen. Die Auswerteeinheit 33 des Endgeräts ist dazu eingerichtet, die Auswahl (Schaltfläche 300 in Fig. 8j) der Person mit der Benutzereingabe 35 des Endgeräts zur Durchführung einer Validierung eines bereits durchgeführten Antigentests auf SARS-CoV-2 zu erkennen. Daraufhin ist die Auswerteeinheit 33 des Endgeräts dazu eingerichtet, das Verfahren gemäß Fig. 8j und 8k zu durchlaufen.

Die Auswerteeinheit 33 des Endgeräts ist dazu eingerichtet, mit Schritt 3001 den Personendatensatz 320 mit der Person 1 (die sich vor dem Endgerät 1 befindet) zu vergleichen. Dazu erfolgt das Erhalten von Biometriedaten, wie zuvor mehrfach beschrieben wurde. Die Auswerteeinheit 33 des Endgeräts ist dazu eingerichtet, beim Vergleichen im Schritt 3001 festzustellen, dass der Personendatensatz 320 nicht mit der Person 1 vor dem Endgerät 30 übereinstimmt (Vergleich der Biometriedaten und/oder der benutzerbezogenen Daten) (Nein-Fall). In diesem Nein-Fall ist die Auswerteeinheit 33 des Endgeräts 1 dazu eingerichtet, auf der Anzeigeeinheit 34 des Endgeräts 30 eine Mitteilung anzuzeigen. Die Mitteilung könnte lauten "Ungültiger Nutzer zum Darlegen des Tests", um anzuzeigen, dass eine nicht autorisierte Person 1 eine Validierung wünscht.

Die Auswerteeinheit 33 des Endgeräts ist dazu eingerichtet, beim Vergleichen im Schritt 3001 festzustellen, dass der Personendatensatz 320 mit der Person 1 vor dem Endgerät 30 übereinstimmt (Vergleich der Biometriedaten und/oder der benutzerbezogenen Daten) (Ja-Fall). In diesem Ja-Fall ist die Auswerteeinheit 33 des Endgeräts 1 dazu eingerichtet, eine Validierungsanfrage im Schritt 301 zu erzeugen und an das Hintergrundsystem 40 zu senden. Das Hintergrundsystem 40 ist dazu eingerichtet, die Validierungsanfrage zu empfangen und auf Basis dieser Validierungsanfrage einen Validierungstoken 440 im Schritt 303 zu erzeugen. Dabei verknüpft das Hintergrundsystem 40 die Eigenschaften des Tokens mit dem Validierungstoken 440. Das Hintergrundsystem 40 ist dazu eingerichtet, den Validierungstoken 440 im Schritt 304 an das Endgerät 30 zu übertragen.

Das Endgerät 30 ist eingerichtet, den Validierungstoken 440 vom Hintergrundsystem 40 zu empfangen. Die Auswerteeinheit 33 des Endgeräts 30 ist dazu eingerichtet, auf Basis des erhaltenen Validierungstoken 440 im Schritt 305 einen QR-Code zu erstellen, der mit den Eigenschaften des Tokens verknüpft ist. Beispielsweise ist dieser QR-Code nur für eine gewisse Zeitdauer gültig (bspw. 10 Minuten). Die Auswerteeinheit 33 des Endgeräts 30 ist dazu eingerichtet, auf der Anzeigeeinheit 34 des Endgeräts 30 das Prozedurergebnis anhand des Datensatzes 321 ggf. mit der Prozedurdokumentation anzuzeigen. Bei der angezeigten Prozedurdokumentation, die auf der Anzeigeeinheit 34 im System 300 angezeigt wird, ist die Auswerteeinheit 33 des Endgeräts 30 dazu eingerichtet, per Zufallsprinzip eine Auswahl der während des Nachverfolgen-Verfahrens 200 aufgenommenen Bilder und/oder Videos zu treffen und diese Auswahl der Prüfinstanz 2 auf Verlangen vorzeigen.

Die Auswerteeinheit 33 des Endgeräts 30 ist dazu eingerichtet, auf der Anzeigeeinheit 34 des Endgeräts 30, den QR-Code und das Prozedurergebnis der Prüfinstanz im Schritt 306 anzuzeigen. Dieses Anzeigen erfolgt optisch. Dazu ist die Prüfinstanz 2 eingerichtet, den QR-Code mittels einer Kamera oder eines Lesegeräts der Prüfinstanz einzulesen.

Die Prüfinstanz 2 ist im Schritt 307a dazu eingerichtet, zu prüfen, ob der Validierungstoken 440 gültig ist. Die Prüfinstanz 2 ist im Schritt 307a dazu eingerichtet, während dem Prüfen im Schritt 307a festzustellen, dass der Validierungstoken 440 nicht gültig ist (Nein-Fall), woraufhin die Prüfinstanz 2 dazu eingerichtet ist, auf der Anzeigeeinheit der Prüfinstanz 2 eine Meldung im Schritt 308a zu generieren, die angibt, dass der Validierungstoken 440 ungültig ist oder der Validierungstoken 440 außerhalb der Zeitdauer abgefragt wurde.

Die Prüfinstanz 2 ist im Schritt 307a dazu eingerichtet, während dem Prüfen im Schritt 307a festzustellen, dass der Validierungstoken 440 nicht gültig ist (Nein-Fall), woraufhin, die Prüfinstanz eingerichtet ist, im Schritt 307b zu prüfen, ob der Streuwert 430 des Personendatensatzes 320 gültig ist und ob die Prozedur-Identifizierungsnummer des Prozedurdatensatzes 450 gültig ist und ob die Prozedur-Identifizierungsnummer des Prozedurdatensatzes 450 mit dem Streuwert 430 verknüpft ist (also ob der Prozedurdatensatzes 450 den Streuwert 430 aufweist).

Die Prüfinstanz 2 ist im Schritt 307a dazu eingerichtet, während dem Prüfen im Schritt 307b festzustellen, dass der Streuwert 430 des Personendatensatzes 320 gültig ist und dass die Prozedur-Identifizierungsnummer des Prozedurdatensatzes 450 gültig ist und dass der Prozedurdatensatzes 450 den Streuwert 430 aufweist (Ja-Fall). Daraufhin ist die Prüfinstanz 2 eingerichtet, im Schritt 308c anzuzeigen, dass das Testergebnis validiert ist (Validierungsergebnis des Prozedurergebnisses ist positiv). Dabei kann auch der Prozedurdatensatz 450 angezeigt werden, bevorzugt wird auch ein Zeitstempel (Zeit und Datum) des Antigentests auf SARS-CoV-2 oder ein Status des Antigentests auf SARS-CoV-2 als Prozedurinformation, bevorzugter das Testergebnis des Antigentests auf SARS-CoV-2 gemäß Hintergrundsystem 40 angezeigt.

In Fig. 9a ist eine Struktur eines anonymisierter Personendatensatz in dem Hintergrundsystem 40 dargestellt. Der anonymisierte Personendatensatzes 410 in dem Hintergrundsystem 40 umfasst einen Streuwert 430 des Personendatensatzes 320. Der anonymisierte Personendatensatzes 410 (= "Remote_Testee") kann neben dem Streuwert 430 auch einen Log-Eintrag umfassen, mit dem Prozedurereignisse, insbesondere fehlgeschlagene Objektidentifizierungen, mit Zeitstempel abgelegt werden können.

Der anonymisierte Personendatensatzes 410 (="Remote_Testee") kann zudem eine Personenidentifikationsnummer umfassen, mit der die Person 1 eindeutig am Hintergrundsystem 40 referenziert werden kann.

In Fig. 9b ist eine Struktur eines Personendatensatz 320 der Speichereinheit 32 des Endgerät 30 dargestellt. Der Personendatensatzes 320 umfasst Biometriedaten. Biometriedaten sind insbesondere Gesichtsgeometrie-Daten und/oder Fingerabdruck-Daten. Der Personendatensatzes 320 umfasst auch personenbezogene Daten. Personenbezogene Daten sind insbesondere Vorname, Nachname, Geburtsdatum, Anschrift, Dokumentennummer. Der Personendatensatzes 320 umfasst auch ein Quarantäne-Ende-Datumsfeld, in das ein Ende einer Quarantänezeit (bei SARS-CoV-2 = 2 Wochen) eingetragen werden kann.

In Fig. 9c ist eine Struktur eines Objektidentifizierungsdatensatz 420 in dem Hintergrundsystem 40 dargestellt. Der Objektidentifizierungsdatensatzes 420 kann beispielsweise einen Objekttyp umfassen. Dies ist sinnvoll, wenn eine Prozedur mit verschiedenen Typen von Objekten 20 durchgeführt werden kann. Mit dem Objekttyp kann dann das Anleiten und Dokumentieren entsprechend angepasst werden. Der Objektidentifizierungsdatensatzes 420 kann beispielsweise eine Herstellerangabe, ein Produktionsdatum, ein Gültigkeitsdatum, eine Objekteigenschaft und/oder ein Zertifikat umfassen. Damit kann das Objekt 20 bestmöglich im Verfahren 200 identifiziert werden und nützliche Informationen für die Durchführung der Prozedur bereitgestellt werden.

In Fig. 9d ist eine Struktur eines Prozedur-Identifizierungsdatensatz 321 der Speichereinheit 32 des Endgeräts 30 dargestellt. Der Prozedur-Identifizierungsdatensatz 321 der Fig. 9d umfasst eine Prozedur-Identifizierungsnummer, die bevorzugt verfahrens- und systemweit eindeutig ist. Der Prozedur-Identifizierungsdatensatz 321 umfasst zudem die Prozedurdokumentation, die Ergebnisse und/oder Zwischenergebnisse und/oder Momentaufnahmen (Screenshots) von der Durchführung umfasst.

In Fig. 9e ist eine Struktur eines Prozedurdatensatz 450 in dem Hintergrundsystem 40 dargestellt. Der Prozedurdatensatzes 450 kann beispielsweise eine Prozedurseriennummer umfassen.

Der Prozedurdatensatzes 450 kann beispielsweise einen Status des Objekts 20 umfassen. Ein Status des Objekts 20 kann "initialisiert" sein, beispielsweise wenn das Objekt 20 im Verfahren 200 existent ist, aber noch keine Registrierung im Hintergrundsystem 40 erfolgt ist. Ein Status des Objekts 20 kann "hergestellt" sein, beispielsweise wenn das Objekt 20 im Verfahren 200 existent ist und auch im Hintergrundsystem 40 registriert ist. Ein Status des Objekts 20 kann "in Verwendung" sein, beispielsweise wenn das Objekt im Verfahren 200 im Schritt 202 erfasst wurde und dann verwendet wird. Ein Status des Objekts 20 kann "fertiggestellt" sein, beispielsweise wenn das Objekt 20 im Verfahren 200 verwendet wurde und ein Testergebnis generiert hat.

Der Prozedurdatensatz 450 kann ein Prozedurergebnis umfassen. Das Prozedurergebnis kann "positiv", "negativ" oder "ungültig" sein. Ein ungültiges Prozedurergebnis wird ausgegeben, wenn die Prozedur falsch angewendet wurde oder Person 1 und/oder Objekt 20 nicht prozedurkonform identifiziert/nachverfolgt werden konnten. Ein positives Prozedurergebnis kann ausgegeben werden, wenn eine Prozedur fehlerfrei und manipulationsfrei durchlaufen wurde. In Anlehnung an einen Virusnachweistest als Prozedur, ist ein positives Testergebnis der Nachweis von Viren durch die Testkassette. In Anlehnung an einen Virusnachweistest als Prozedur, ist ein negatives Testergebnis der fehlende Nachweis von Viren durch die Testkassette.

Der Prozedurdatensatz 450 kann einen Zeitstempel, beispielsweise eine Zeit- oder Datumsangabe umfassen. Der Prozedurdatensatz 450 kann den Streuwert 430 umfassen. Der Prozedurdatensatz 450 kann eine Prozedur-Identifizierungsnummer umfassen. Der Prozedurdatensatz 450 kann eine Personenidentifizierungsnummer umfassen.

In Fig. 10a ist eine erste Ausgestaltung zur Vorbereitung eines Tests gemäß Schritt 2044a gezeigt. Dabei wird im Schritt 2044a eine Aufforderung zum Auspacken des Antigentests auf SARS-CoV-2 angezeigt, wobei die Testkassette 20 und auch alle Hilfsmittel 50 in das Sichtfeld (erfassbarer Bereich oder vordefinierter Bereich) der Kamera 31a des Endgeräts 30 platziert werden müssen. Zudem wird die Person im Schritt 2044a angewiesen, den QR-Code 210 (= maschinenlesbarer Objektidentifizierungsdatensatz 21) der Testkassette 20 so zu platzieren, dass die Kamera 31a des Endgeräts 30 diesen QR-Code 210 erfassen kann (Schritt 2021 in Fig. 3).

In dieser Ausgestaltung gemäß Fig. 10a ist der QR-Code 210 bereits während der Produktion des Tests auf die Testkassette 20 appliziert worden. Dabei kann der QR-Code 210 aufgedruckt oder mittels Kleber aufgebracht sein. Die Person hat den Test mit der Information erwerben können, dass der Test bereits vollständig für eine Validierung (gemäß dem Verfahren 300) eingerichtet ist und kein QR-Code 210 zusätzlich erworben werden muss.

In Fig. 10b ist eine zweite Ausgestaltung zur Vorbereitung eines Tests gemäß Schritt 2044a gezeigt. Dabei wird im Schritt 20441 (erstens) eine Anleitung zum Erfassen der Testverpackung (im unausgepackten Zustand) angezeigt und (zweitens) eine Aufforderung zum Auspacken des Antigentests auf SARS-CoV-2 angezeigt. Durch das Erfassen der Testverpackung ist das Verfahren 200/ das System 2000 dazu eingerichtet, die Information über den Testtyp (Objekttyp im Objektidentifizierungsdatensatz 420) zu erfassen.

Danach wird im Schritt 20442 (erstens) eine Aufforderung zum Anbringen eines QR-Codes 210 zur Validierung des Antigentests auf SARS-CoV-2 auf die Testkassette 20 angezeigt. Sodann muss die Person den QR-Code 210 auf die Testkassette 20 aufbringen. Das Aufbringen kann mit einem Kleber erfolgen. Beispielsweise ist der QR-Code 210 als Klebepatch ausgebildet. Eine Klebeschicht an der Unterseite des QR-Code-Patches sorgt bevorzugt für eine mechanisch nicht lösbare Verbindung von QR-Code-Patch und Testkassette 20. Danach wird im Schritt 20442 (zweitens) die Person 1 angewiesen, den aufgebrachten QR-Code 210 (= maschinenlesbarer Objektidentifizierungsdatensatz 21) der Testkassette 20 so zu platzieren, dass die Kamera 31a des Endgeräts 30 diesen QR-Code 210 erfassen kann (Schritt 2021 in Fig. 3).

In dieser Ausgestaltung gemäß Fig. 10b ist der QR-Code 210 nicht während der Produktion des Tests auf die Testkassette 20 appliziert worden. Die Person hat den Test mit der Information erwerben können, dass der Test nicht für eine Validierung (gemäß dem Verfahren 300) eingerichtet ist und keinen QR-Code 210 enthält. Möchte die Person 1 ihr Testergebnis validieren lassen (gemäß Verfahren 300), so muss die Person 1 einen QR-Code 210 händisch aufbringen. Den QR-Code 210 kann die Person 1 parallel zum Test selbst erwerben. In dieser Ausgestaltung gemäß Fig. 10b wird der QR-Code 210 erst durch die Person 1 vor der Durchführung des Tests auf die Testkassette 20 appliziert.

Es folgt eine Liste mit Beispielen:
Beispiel 1: Ein System (2000) zum computergestützten Nachverfolgen einer von einer Person (1) durchzuführenden Testprozedur, insbesondere für einen Virusnachweistest, bevorzugt zur Durchführung eines SARS-CoV-2 Tests, wobei das System (2000) umfasst:
   - eine Identifizierungseinheit eines Endgeräts (30), die eingerichtet ist zum Identifizieren (201) der Person (1) unmittelbar vor einem Durchführen der Testprozedur durch die Person (1);
   - wobei die Identifizierungseinheit des Endgeräts (30) weiter eingerichtet ist zum Identifizieren (202) eines Testobjekts (20), bevorzugt einer Testkassette, insbesondere für einen SARS-CoV-2 Test, mehr bevorzugt eines Teststreifens, weiter bevorzugt ein Reagenz in einem Behälter, weiter bevorzugt eines Testsensors, unmittelbar vor der Durchführung der Testprozedur, die Identifizierungseinheit aufweisend:
      - eine Kamera (31) des Endgeräts (30), eingerichtet zum Erfassen (2021) eines Objektidentifizierungsdatensatzes (21) als maschinenlesbaren Datensatz; und
      - eine Auswerteeinheit (33) des Endgeräts (30), eingerichtet zum Vergleichen (2022) des erfassten Objektidentifizierungsdatensatzes (21) mit einem Objektdatensatz (420) eines Hintergrundsystems (40);
   - eine Nachverfolgungseinheit des Endgeräts (30), die eingerichtet ist zum Nachverfolgen (203) einer oder mehrerer Positionen der Person (1) während der Durchführung der Testprozedur mittels Methoden computergestützter Gesten- und/oder Muster- und/oder Bilderkennung mittels eines Prüfens, ob beide Hände (12) der Person (1) während der gesamten Durchführung der Testprozedur in einem vordefinierten Bereich oder einem von der Kamera (31a) des Endgeräts (30) erfassbaren Bereich sind;
   - die Nachverfolgungseinheit des Endgeräts (30), zudem eingerichtet zum Nachverfolgen (203) von einer oder mehreren Positionen des Testobjekts (20) anhand der Form des Objekts während der Durchführung der Testprozedur mittels Methoden computergestützter Gesten- und/oder Muster- und/oder Bilderkennung; und
   - einer Anzeigeeinheit (34) des Endgeräts, eingerichtet zum Anleiten (204) der Person (1) zum Durchführen der Testprozedur während der Durchführung der Testprozedur.
Beispiel 2. Das System (200) nach Beispiel 1, wobei das Prüfen durch die Nachverfolgungseinheit des Endgeräts (30) auch umfasst, ob das Gesicht (1a) der Person (1) während der gesamten Durchführung der Testprozedur in dem vordefinierten Bereich oder dem von der Kamera (31a) des Endgeräts (30) erfassbaren Bereich ist.
Beispiel 3. Das System (200) nach Beispiel 2, wobei das Prüfen durch die Nachverfolgungseinheit des Endgeräts (30) auch umfasst, ob das Gesicht (1a) und die beiden Hände (1b) der Person (1) während eines Einrichtens eines Test-Sets für die Testprozedur in dem vordefinierten Bereich oder dem von der Kamera (31a) des Endgeräts (30) erfassbaren Bereich ist.
Beispiel 4. Das System (200) nach Beispiel 2 oder 3, wobei das Prüfen durch die Nachverfolgungseinheit des Endgeräts (30) auch umfasst:
   - ob das Gesicht (1a) und die beiden Hände (1b) der Person (1) während einer Abgabe einer Speichelprobe in dem vordefinierten Bereich oder dem von der Kamera (31a) des Endgeräts (30) erfassbaren Bereich ist; und/oder
   - ob das Gesicht (1a) und die beiden Hände (1b) der Person (1) während einer Hinzugabe einer Extraktionsflüssigkeit in die Speichelprobe in dem vordefinierten Bereich oder dem von der Kamera (31a) des Endgeräts (30) erfassbaren Bereich ist; und/oder
   - ob das Gesicht (1a) und die beiden Hände (1b) der Person (1) während eines Schüttelns der Speichelprobe für eine vorgegebene Zeitdauer in dem vordefinierten Bereich oder dem von der Kamera (31a) des Endgeräts (30) erfassbaren Bereich ist; und/oder
   - ob das Gesicht (1a) und die beiden Hände (1b) der Person (1) und das Objekt (20) während eines Injizierens der Speichelprobe auf das Objekt (20) in dem vordefinierten Bereich oder dem von der Kamera (31a) des Endgeräts (30) erfassbaren Bereich ist.
Beispiel 5. Das System (200) nach einem der vorhergehenden Beispiele, wobei die Nachverfolgungseinheit des Endgeräts (30) zudem dazu eingerichtet ist, eine oder mehrere Bewegungen der Person und/oder des Testobjekts anhand der Form des Testobjekts (20) während der Durchführung der Testprozedur mittels Methoden computergestützter Gesten- und/oder Muster- und/oder Bilderkennung zu erkennen.
Beispiel 6. Das System (200) nach Beispiel 5, wobei die Nachverfolgungseinheit des Endgeräts (30) dazu prüft, ob das Testobjekt (20) während der gesamten Durchführung der Testprozedur in einem vordefinierten Bereich ist.
Beispiel 7. Das System (200) nach einem der vorhergehenden Beispiele, wobei die Identifizierungseinheit zum Identifizieren (202) des Testobjekts umfasst:
   - die Kamera (31) des Endgeräts (30), eingerichtet zum Erfassen (2011) eines Gesichts (1a) der Person (1);
   - eine Auswerteeinheit (33) des Endgeräts (30), eingerichtet zum Auswerten des erfassten Gesichts (1a) mittels Methoden computergestützter Gesichts- und/oder Muster- und/oder Bilderkennung zum Erhalten von Biometriedaten und zum Vergleichen (2012) der erhaltenen Biometriedaten mit Biometriedaten eines Personendatensatzes (320), wobei der Personendatensatz (320) in einer Speichereinheit (32) des Endgeräts (30) abgelegt ist.
Beispiel 8. Das System (2000) nach Beispiel 7, wobei die Identifizierungseinheit des Endgeräts (30) weiter eingerichtet ist, das Identifizieren (202) der Person (1) zu einem oder mehreren zufälligen Zeitpunkten während der Durchführung der Testprozedur zu wiederholen.
Beispiel 9. Das System (2000) nach Beispiel 7 oder 8, wobei das Endgerät (2) eine Fehlermeldung auf einer Anzeigeeinheit (35) des Endgeräts (2) anzeigt, wenn die Auswerteeinheit (33) des Endgeräts (30) beim Vergleichen (2012) feststellt, dass die erfassten Biometriedaten nicht mit den Biometriedaten des Personendatensatzes (320) übereinstimmen.
Beispiel 10. Das System (2000) nach einem der vorhergehenden Beispiele, wobei die Identifizierungseinheit des Endgeräts (30) weiter dazu eingerichtet ist, unmittelbar vor dem Durchführen der Testprozedur durch die Person (1) alle während der Testprozedur zu verwendenden Hilfsmittel (50) durch das Endgerät (30) anhand ihrer Form mittels Methoden computergestützter Gesichts- und/oder Muster- und/oder Bilderkennung zu identifizieren.
Beispiel 11. Das System (2000) nach Beispiel 10, wobei das Hilfsmittel (50): ein Stäbchen zur Entnahme einer Nasen- oder Speichelprobe bei der Person (1) ist; und/oder ein Flüssigkeitsbehälter ist.
Beispiel 12. Das System (2000) nach Beispiel 10 oder 11, wobei jedes verwendete Hilfsmittel (50) mittels der Kamera (31) des Endgeräts (30) erfasst wird und das erfasste Hilfsmittel (50) mittels Methoden computergestützter Gesichts- und/oder Muster- und/oder Bilderkennung durch eine Auswerteeinheit (33) des Endgeräts (30) analysiert wird, um das Hilfsmittel (50) zu identifizieren.
Beispiel 13. Das System (2000) nach einem der vorhergehenden Beispiele, wobei der maschinenlesbare Datensatz (210, 220) des Objekts (40) durch Abfragen (2023) eines entsprechenden Objektdatensatzes (420) in einem Hintergrundsystem (40) validierbar ist, wobei nach der Validierung des maschinenlesbaren Datensatzes (210, 220) der Objektdatensatz (420) in der Datenbank (41) des Hintergrundsystems (40) ungültig ist (2024).
Beispiel 14. Das System (2000) nach einem der vorhergehenden Beispiele, wobei die Nachverfolgungseinheit des Endgeräts (30) zudem dazu eingerichtet ist, eine Objekteigenschaft des Objekts (20) mittels der Kamera (31) des Endgeräts (30) zu erfassen (2031), wobei die erfasste Objekteigenschaft des Objekts (20) mittels Methoden computergestützter Gesichts- und/oder Muster- und/oder Bilderkennung durch eine Auswerteeinheit (33) des Endgeräts (30) analysierbar (2032) ist und die analysierte Objekteigenschaft des Objekts (20) mit einer während der Testprozedur zu erwartenden Objekteigenschaft verglichen wird.
Beispiel 15. Das System (2000) nach Beispiel 14, wobei eine Objekteigenschaft das Applizieren einer Abstrich-, Speichel- oder Spuckprobe der Person (1) auf die Testkassette eines Antigentests auf SARS-CoV-2 ist.
Beispiel 16. Das System (2000) nach Beispiel 14 oder 15, wobei eine Objekteigenschaft das Verfärben eines Teststreifens der Testkassette eines SARS-CoV-2 Tests ist.
Beispiel 17. Das System (2000) nach Beispiel 14 bis 16, wobei eine Objekteigenschaft das Erscheinen eines oder mehrerer Teststreifen auf der Testkassette eines SARS-CoV-2 Tests ist.
Beispiel 18. Das System (2000) nach einem der vorhergehenden Beispiel, wobei das Endgerät (2) eine Fehlermeldung auf einer Anzeigeeinheit (35) des Endgeräts (2) anzeigt, wenn die Auswerteeinheit (33) des Endgeräts (30) beim Analysieren (2032) feststellt, dass die erfasste Position der Person (1) und/oder die erfasste Bewegung der Person (1) und/oder die erfasste Position des Objekts (1) und/oder die erfasste Bewegung des Objekts (1) außerhalb des vordefinierten Bereich oder des von der Kamera (31a) des Endgeräts (30) erfassbaren Bereichs ist.
Beispiel 19. Das System (2000) nach einem der vorhergehenden Beispiel, wobei zu einem Dokumentieren des Durchführens der Testprozedur das Durchführen der Testprozedur zu einem oder mehreren zufälligen Zeitpunkten während der Durchführung der Testprozedur mittels der Kamera (31) des Endgeräts (30) erfasst und abgespeichert ist.
Beispiel 20. Das System (2000) nach einem der vorhergehenden Beispiele, wobei auf der Anzeige (34) des Endgeräts (30) Anleitungshinweisen angezeigt werden, wobei ein Anleitungshinweis angezeigt wird:
   - in Abhängigkeit der Durchführung der Testprozedur und/oder
   - in Abhängigkeit des Nachverfolgens (203) von Positionen und Bewegungen der Person (1) und des Objekts (20) durch das Endgerät (30) während der Durchführung der Testprozedur;
   - nach Zeitablauf einer in der Testprozedur vorgeschriebenen Wartezeit.
Beispiel 21. Ein System (3000) zum computergestützten Validieren einer von einer Person (1) durchgeführten Prozedur, wobei das System (3000) umfasst:
   - eine Eingabeeinheit (35) eines Endgeräts (30) eingerichtet zum Empfangen einer Personeneingabe zum Anfordern (301) einer Validierung eines Prozedurergebnisses;
   - eine Steuereinheit (33), eingerichtet zum:
      - Erzeugen eines Streuwerts über einen, in einer Speichereinheit (32) des Endgeräts (30) abgelegten Personendatensatz (320)
      - Erzeugen einer Validierungsanfrage umfassend den Streuwert und einen Prozeduridentifizierungsdatensatz (321) des zu validierenden Prozedurergebnisses;
      - Senden der Validierungsanfrage an ein Hintergrundsystem (40);
      - Empfangen (304) im Endgerät (30) eines Validierungstoken von dem Hintergrundsystems (40);
   - einer Anzeigeeinheit (34) des Endgeräts, eingerichtet zum Anzeigen (305) eines maschinenlesbaren Codes mit dem Validierungstoken (440) und dem Prozedurergebnis;
   - einer Prüfinstanz, eingerichtet zum:
      - Prüfen (306) des maschinenlesbaren Codes unter Prüfung des Validierungstoken;
      - Anzeigen (307) eines Validierungsergebnisses bezüglich des Prozedurergebnisses zusammen mit dem Prozedurergebnis und dem Streuwert über den Personendatensatz und bevorzugt Prozedurinformationen.
Beispiel 22. Das System (3000) nach Beispiel 21, wobei der Validierungstoken eine Gültigkeitszeitdauer für das Prozedurergebnis definiert.
Beispiel 23. Das System (3000) nach einem der vorhergehenden Beispiele 21 oder 22, wobei die Prozedurinformationen ein Prozedurdatum und/oder eine Prozedurzeit gemäß Hintergrundsystem (40) ist.
Beispiel 24. Das System (3000) nach einem der vorhergehenden Beispiele 21 bis 23, wobei der Prozeduridentifizierungsdatensatz von dem Endgerät erzeugt wurde.
Beispiel 25. Das System (3000) nach einem der vorhergehenden Ansprüche 21 bis 24, wobei der maschinenlesbare Code, bevorzugt ein QR-Code ist und wobei die Prüfinstanz (2) eine Erfassungseinheit umfasst, um den maschinenlesbaren Code einzulesen.
Beispiel 26. Das System (3000) nach einem der vorhergehenden Beispiele 21 bis 25, wobei das Prozedurergebnis vom Hintergrundsystem abgefragt (308) wird.
Beispiel 27. Ein System (1000) zum Einrichten eines Personenprofils umfassend ein Endgerät (30) und ein Identifizierungsdokument (10), wobei das System (1000) umfasst:
   - eine Erfassungseinheit (31) des Endgeräts (30), eingerichtet zum Erfassen (101) von Daten (11, 14, 15) eines Identifizierungsdokuments (10) der Person (1);
   - eine Auswerteeinheit (33) des Endgeräts (30), eingerichtet zum:
      - Auswerten (102) der Daten (11, 14, 15) des erfassten Identifizierungsdokuments (10) zum Erhalten eines Personendatensatzes (320) umfassend Biometriedaten und personenbezogene Daten der Person (1);
   - eine Speichereinheit (32) des Endgeräts (30), eingerichtet zum Abspeichern (103) des erhaltenen Personendatensatzes (320) als Personenprofil.
Beispiel 28. Das System (1000) nach Beispiel 27, wobei die Auswerteeinheit (31) des Endgeräts (30) weiter eingerichtet ist zum Erzeugen (105) eines Streuwerts (430) des Personendatensatzes (320).
Beispiel 29. Das System (1000) nach Beispiel 28, weiter umfassend ein Hintergrundsystem (40), eingerichtet zum Empfangen und Abspeichern (106) des Streuwerts (430) in einer Datenbank (41).
Beispiel 30. Das System nach einem der Beispiele 28 oder 29, wobei der Streuwert (430) ein Teil eines anonymisierten Benutzerdatensatzes (410) in dem Hintergrundsystem (40) ist, wobei der anonymisierte Benutzerdatensatz (410) bevorzugt eine Personenidentifizierungsnummer und einen Log-Eintrag umfasst.
Beispiel 31. Das System (1000) nach einem der Beispiele 27 bis 30,
   - wobei die Erfassungseinheit (31) des Endgeräts (30) eine Kamera (31a) ist, wobei die Kamera (31a) eingerichtet ist, ein Lichtbild (14) des Identifizierungsdokuments (10) zu erfassen (101); und
   - wobei die Auswerteeinheit (33) des Endgeräts (30) ein Gesichtserkennungsmodul (330) umfasst, wobei das Gesichtserkennungsmodul (330) eingerichtet ist, das erfasste Lichtbild (14) mittels Methoden computergestützter Gesichtserkennung zu analysieren, um die Biometriedaten des Personendatensatzes (320) zu erhalten.
Beispiel 32. Das System (1000) nach einem der Beispiele 27 bis 31,
   - wobei die Erfassungseinheit (31) des Endgeräts (30) eine Kamera (31a) ist, wobei die Kamera (31a) eingerichtet ist, personenbezogene Daten (15) des Identifizierungsdokuments (10) zu erfassen (101); und
   - wobei die Auswerteeinheit (33) des Endgeräts (30) ein Zeichenerkennungsmodul (331) umfasst, wobei das Zeichenerkennungsmodul (331) eingerichtet ist, die erfassten personenbezogenen Daten (15) mittels Methoden optischer Zeichenerkennung zu analysieren, um die personenbezogenen Daten des Personendatensatzes (320) zu erhalten.
Beispiel 33. Das System (1000) nach einem der Beispiele 27 bis 32,
   - wobei das Identifizierungsdokument (10) einen elektronischen Datenspeicher (11) umfasst;
   - wobei die Erfassungseinheit (31) des Endgeräts (30) eine Leseeinheit (31b) ist, wobei die Leseeinheit (31b) eingerichtet ist, elektronische Daten (11) eines Datenspeichers des Identifizierungsdokuments (10) auszulesen; und
   - wobei die Auswerteeinheit (33) des Endgeräts (30) eingerichtet ist, die ausgelesenen Daten (11) auszuwerten, um den Personendatensatzes (320) zu erhalten.
Beispiel 34. Das System (1000) nach einem der Beispiele 27 bis 33,
   - wobei die Auswerteeinheit (33) ein Verschlüsselungsmodul (332) umfasst, wobei das Verschlüsselungsmodul (332) eingerichtet ist, den erhaltenen Personendatensatz (320) kryptografisch abzusichern; und
   - wobei die Speichereinheit (32) des Endgeräts (30) dazu eingerichtet ist, den kryptografisch abgesicherten Personendatensatz (320) abzuspeichern.
Beispiel 35. Ein Computerprogrammprodukt, umfassend Befehle, die bei der Ausführung des Programms durch einen Computer diesen veranlassen, die Einheiten oder Module des Systems nach einem der vorhergehenden Beispiele 1 bis 34 auszuführen.

Im Rahmen der Erfindung können alle beschriebenen und/oder gezeichneten und/oder beanspruchten Elemente beliebig miteinander kombiniert werden.

### BEZUGSZEICHENLISTE

- 1: Person, Benutzer
1a Gesicht
1b Hand
- 2: Prüfinstanz, ggf. mit Erfassungseinheit,
- 10: Identifizierungsdokument, ID
11 elektronische Daten eines Datenspeichers
12 Vorderseite
13 Rückseite
14 Lichtbild
15 personenbezogene Daten
- 20: Objekt für Prozedur, Testkassette für SARS-CoV-2 Test
21 Obj ektidentifizierungsdatensatz
   210 QR-Code
   220 Seriennummer
22 Form, Kontur
- 30: Endgerät
31 Erfassungseinheit,
   31a Kamera
   31b Leseeinheit
   31c Code-Scanner
32 Speichereinheit
   320 Personendatensatz
   321 Prozedur-Identifizierungsdatensatz
33 Auswerteeinheit, Steuereinheit, CPU
   330 Gesichtserkennungsmodul
   331 Zeichenerkennungsmodul
   332 Verschlüsselungsmodul
   333 Mustererkennungsmodul
34 Anzeigeeinheit
   340 Prozedurergebnis-Validierungsdatensatz
   341 Instruktion bzgl. Prozedur
   342 Instruktion bzgl. Korrektur in der Prozedur
   343 Warnung
   344 (Zwischen-)Ergebnis
35 Eingabeeinheit
- 40: Hintergrundsystem
41 Speichereinheit, Datenbank
   410 anonymisierter Personendatensatz
   420 Obj ektidentifizierungsdatensatz
   430 Hashwert über Personendatensatz 320
   440 Validierungstoken
   450 Prozedurdatensatz
42 Benutzereinrichtungs-System
50 Hilfsmittel (Form, Kontur)
100 Verfahrensschritte zum Einrichten
1001 Anzeige AGB,
1002 Personeneingabe zwecks AGB
1003 Prüfe Existenz von Personenprofil
1004 Prüfe Quarantäne-Ende-Datum
101 Erfassen von Daten
1011 Aufforderung zum Zeigen des Identifizierungsdokuments
1012 Aufforderung zum Zeigen des Gesichts
1013 Verschlüsselter Datenstrom
102 Auswerten der erfassten Daten
1021 Zeichenerkennung am erfassten Identifizierungsdokument
1022 Gesichtserkennung am Lichtbild & am Gesicht
1023 Zeichenerkennung und Gesichtserkennung abgeschlossen
1024 Anzeige eines Fehlers mit Erläuterung
1025 Fertigstellen des Benutzerdatensatzes
103 Abspeichern eines Personendatensatzes als Personenprofil
104 Kryptografisches Absichern des Personendatensatzes
105 Erzeugen eines Streuwerts
106 Senden von Endgeräte-Identifizierungsdatensatz
199 Auswahl
200 Verfahrensschritte zum Nachverfolgen
201 Identifizieren der Person
   2011 Erfassen von Biometriedaten
   2012 Vergleichen mit gespeicherten Personendaten
   2013 Abfragen des Hintergrundsystems
   2014 Identifizierung erfolgreich
202 Identifizieren des Objekts/Hilfsmittel
   2021 Erfassen eines Objektidentifizierungsdatensatzes
   2022 Vergleichen mit Objektdatensatz
   2023 Validieren des Objektdatensatzes durch Abfragen im Hintergrundsystem
   2024 Ungültigmachen des Objektdatensatzes
203a-k Nachverfolgen von Person und Objekt während Prozedurdurchführung
   2030 Auswahl eines Prozedurtyps
   2031 Erfassen von Position, Bewegung von Person, Objekt, Hilfsmittels
   2032 Analysieren mittels computergestützter Erkennung
204 Anleiten/Informieren Prozedurdurchführung
   2041 Einleitungsvideo
   2042 Anleitung zum Einrichten des Endgeräts
   2043 Test der Endgeräte-Einrichtung
   2044a Test vorbereiten für Identifizierung
      20441 Test aufnehmen
      20442 QR-Code und Testkassette verbinden
   2044b Anleitung zum Auspacken/Zusammenbauen/Erfassen des Objekts
   2045a-f Prozedurabbruchinformation
   2046 Bedingungen für Prozedur abfragen
   2047 Wartezeit anzeigen
   2048a-d Anleitung Prozedurschritt und Erfassen des Objekts
   2049a-b Prozedurergebnis anzeigen
205 Dokumentieren der Prozedurdurchführung
2100 Ablaufdiagramm Abstrich-Test
   2144a-c Anleitungen für den Abstrich-Test
206 Prüfung, ob alle Prozessschritte durchgeführt worden sind
300 Verfahrensschritte zum Validieren
3001 Vergleich Personendatensatz mit Person
301 Anfordern einer Validierung eines Prozedurergebnisses
302 Anfrage am Hintergrundsystem
303 Erstelle einen Validierungstoken
304 Übertragen des Validierungstoken
305 Anzeigen einer maschinenlesbaren Code mit Validierungstoken
306 Einlesen des maschinenlesbaren Code
307a,b Prüfen des maschinenlesbaren Code
308a-c Anzeige des Validierungsergebnisses
1000 System zum Einrichten
2000 System zum Nachverfolgen
3000 System zum Validieren

## Patentansprüche

1. Ein Verfahren (200) zum computergestützten Nachverfolgen einer von einer Person (1) durchzuführenden Prozedur, wobei das Verfahren (200) die folgenden Schritte umfasst:
- Identifizieren (201) der Person (1) durch ein Endgerät (30) unmittelbar vor einem Durchführen der Prozedur durch die Person (1) mit:
- Erfassen (2011) von Biometriedaten der Person (1) durch das Endgerät (30);
und
- Vergleichen (2012) der erfassten Biometriedaten mit Biometriedaten eines Personendatensatzes (320) durch das Endgerät (30), wobei der Personendatensatz (320) in einer Speichereinheit (32) des Endgeräts (30) abgelegt ist;
- Identifizieren (202) zumindest eines durch die Prozedur zu verändernden Objekts (20) durch das Endgerät (30) unmittelbar vor der Durchführung der Prozedur;
- Nachverfolgen (203) zumindest einer Position der Person (1) und zumindest einer Position des zumindest einen Objekts (20) durch das Endgerät (30) mittels Methoden computergestützter Gesten- und/oder Muster- und/oder Bilderkennung während der Durchführung der Prozedur; und
- Anleiten (204) der Person (1) zum Durchführen der Prozedur durch das Endgerät (30) während der Durchführung der Prozedur.

2. Das Verfahren (200) nach Anspruch 1, wobei zum Identifizieren (202) der Person (1) im Erfassenschritt (2011) ein Gesicht (1a) der Person (1) mittels einer Erfassungseinheit, insbesondere einer Kamera (31a) des Endgeräts (30) erfasst wird und das erfasste Gesicht (1a) mittels Methoden computergestützter Gesichtserkennung durch eine Auswerteeinheit (33) des Endgeräts (30) analysiert wird, um die Biometriedaten der Person (1) zu erhalten, wobei bevorzugt das Identifizieren (202) der Person zu einem oder mehreren zufälligen Zeitpunkten während der Durchführung der Prozedur wiederholt wird.

3. Das Verfahren (200) nach einem der vorhergehenden Ansprüche, wobei die Prozedur eine humanmedizinischen Untersuchung einer Körperflüssigkeit der Person (1) selbst oder eine veterinärmedizinischen Untersuchung einer Körperflüssigkeit an einem Tier durch die Person (1) ist, wobei die Körperflüssigkeit das Blut, der Abstrich, der Speichel, das Aerosol, die Tränenflüssigkeit, der Schweiß, das Sputum, der Urin, eine Abnahme von einer Hautoberfläche und/oder der Stuhl der Person (1) und/oder des Tieres ist.

4. Das Verfahren nach einem der vorhergehenden Ansprüche, wobei das Identifizieren (202) des Objekts (20) umfasst:
- Erfassen (2021) eines Objektidentifizierungsdatensatzes (21) durch das Endgerät (30); und
- Vergleichen (2022) des erfassten Objektidentifizierungsdatensatzes (21) mit einem Objektidentifizierungsdatensatz (420) oder einem Prozedurdatensatz (450) eines Hintergrundsystems (40).

5. Das Verfahren (200) nach einem der vorhergehenden Ansprüche, wobei unmittelbar vor dem Durchführen der Prozedur durch die Person (1) alle während der Prozedur zu verwendenden Hilfsmittel (50) durch das Endgerät (30) identifiziert werden, wobei bevorzugt jedes verwendete Hilfsmittel (50) mittels einer Kamera (31) des Endgeräts (30) erfasst wird und das erfasste Hilfsmittel (50) mittels Methoden computergestützter Gesichts- und/oder Muster- und/oder Bilderkennung durch eine Auswerteeinheit (33) des Endgeräts (30) analysiert wird, um das Hilfsmittel (50) zu identifizieren.

6. Das Verfahren (200) nach einem der vorhergehenden Ansprüche, wobei zum Identifizieren (203) des Objekts (20) im Erfassenschritt (2021) ein maschinenlesbarer Datensatz (210, 220) mittels einer Erfassungseinheit, insbesondere einer Kamera (31), des Endgeräts (30) erfasst wird und der maschinenlesbarer Datensatz (210, 220) des Objekts (20) durch Abfragen (2023) eines entsprechenden Objektidentifizierungsdatensatzes (420) in einem Hintergrundsystem (40) validiert wird, wobei bevorzugt nach Validierung des maschinenlesbaren Datensatzes (210, 220) der Objektidentifizierungsdatensatz (420) oder der Prozedurdatensatz (450) in der Datenbank (41) des Hintergrundsystems (40) ungültig wird (2024).

7. Das Verfahren (200) nach einem der vorhergehenden Ansprüche, wobei im Nachverfolgenschritt (203) die zumindest eine Position und/oder eine Bewegung der Person (1), bevorzugt auch eine Bewegung eines Körperteils (12) der Person (1), und die zumindest eine Position und/oder eine Bewegung des Objekts (20) mittels einer Kamera (31) des Endgeräts (30) erfasst (2031) wird und die erfasste Position und Bewegung der Person (1) und des Objekts (20) mittels Methoden computergestützter Gesichts- und/oder Muster- und/oder Bilderkennung durch eine Auswerteeinheit (33) des Endgeräts (30) analysiert (2032) wird.

8. Das Verfahren (200) nach einem der vorhergehenden Ansprüche, wobei im Nachverfolgenschritt (203) eine Objekteigenschaft des Objekts (20) mittels einer Kamera (31) des Endgeräts (30) erfasst (2031) wird und die erfasste Objekteigenschaft des Objekts (20) mittels Methoden computergestützter Gesichts- und/oder Muster- und/oder Bilderkennung durch eine Auswerteeinheit (33) des Endgeräts (30) analysiert (2032) wird und die analysierte Objekteigenschaft des Objekts (20) mit einer während der Prozedur zu erwartenden Objekteigenschaft verglichen wird.

9. Das Verfahren (200) nach Anspruch 7 oder 8, wobei das Verfahren (200) mit einer Fehlermeldung beendet wird, wenn im Analysierenschritt (2032) festgestellt wird, dass die erfasste Position der Person (1) und/oder die erfasste Bewegung der Person (1) und/oder die erfasste Position des Objekts (1) und/oder die erfasste Bewegung des Objekts (1) außerhalb fester Vorgaben der Prozedur sind.

10. Das Verfahren (200) nach einem der vorhergehenden Ansprüche, wobei für ein Dokumentieren (204) des Durchführens der Prozedur das Durchführen der Prozedur zu einem oder mehreren zufälligen Zeitpunkten während der Durchführung der Prozedur mittels einer Kamera (31) des Endgeräts (30) erfasst und abgespeichert wird.

11. Das Verfahren (200) nach einem der vorhergehenden Ansprüche, wobei für das Anleiten (204) Anleitungshinweise auf einer Anzeige (34) des Endgeräts (30) angezeigt werden, wobei ein Anleitungshinweis in Abhängigkeit der Durchführung der Prozedur und/oder in Abhängigkeit des Nachverfolgens (203) von Positionen und Bewegungen der Person (1) und des Objekts (20) durch das Endgerät (30) während der Durchführung der Prozedur erfolgen.

12. Ein System (2000) zum computergestützten Nachverfolgen einer von einer Person (1) durchzuführenden Prozedur, wobei das System (2000) umfasst:
- eine Identifizierungseinheit eines Endgeräts (30) eingerichtet zum Identifizieren (201) der Person (1) unmittelbar vor einem Durchführen der Prozedur durch die Person (1), die Identifizierungseinheit umfassend:
- eine Erfassungseinheit (31) des Endgeräts (30), eingerichtet zum Erfassen (2011) von Biometriedaten der Person (1);
- eine Auswerteeinheit (33) des Endgeräts (30), eingerichtet zum Vergleichen (2012) der erfassten Biometriedaten mit Biometriedaten eines Personendatensatzes (320), wobei der Personendatensatz (320) in einer Speichereinheit (32) des Endgeräts (30) abgelegt ist;
- wobei die Identifizierungseinheit weiter eingerichtet ist zum Identifizieren (202) eines durch die Prozedur zu veränderndem Objekt (20) unmittelbar vor der Durchführung der Prozedur;
- eine Nachverfolgungseinheit des Endgeräts (30), eingerichtet zum Nachverfolgen (203) zumindest einer Position der Person (1) und zumindest einer Position des Objekts (20) durch das Endgerät (30) mittels Methoden computergestützter Gesten- und/oder Muster- und/oder Bilderkennung während der Durchführung der Prozedur; und
- einer Anzeigeeinheit (34) des Endgeräts, eingerichtet zum Anleiten (204) der Person (1) zum Durchführen der Prozedur während der Durchführung der Prozedur.

13. Ein Verfahren (300) zum computergestützten Validieren einer von einer Person (1) durchgeführten und computergestützt nachverfolgten Prozedur, wobei das Verfahren (300) die folgenden Schritte umfasst:
- Anfordern (301) einer Validierung eines Prozedurergebnisses durch eine Personeneingabe an einem Endgerät (30);
- Erzeugen und Versenden (302) einer Validierungsanfrage bei einem Hintergrundsystem (40) umfassend einen Streuwert über den Personendatensatz und einen Prozeduridentifizierungsdatensatz des zu validierenden Prozedurergebnisses;
- Empfangen (304) im Endgerät (30) eines Validierungstoken vom Hintergrundsystem (40);
- Anzeigen (305) eines maschinenlesbaren Code unter Verwendung des Validierungstokens (440) und Anzeigen des Prozedurergebnisses auf einer Anzeigeeinheit (34) des Endgeräts (30);
- Einlesen (306) des maschinenlesbaren Codes durch eine Prüfinstanz (2) und Prüfen (307) des Validierungstokens (440);
- Anzeigen (308) eines Validierungsergebnisses bezüglich des Prozedurergebnisses zusammen mit dem Prozedurergebnis und dem Streuwert (430) über den Personendatensatz (320) und bevorzugt Prozedurinformationen des Hintergrundsystems (40) auf einer Anzeigeeinheit der Prüfinstanz (2).

14. Das Verfahren nach Anspruch 13, wobei die computergestützt nachverfolgte Prozedur nach einem Verfahren (200) zum computergestützten Nachverfolgen einer von einem Person durchzuführenden Prozedur gemäß einem der Ansprüche 1 bis 11 computergestützt nachverfolgt wurde.

15. Ein Computerprogrammprodukt, umfassend Befehle, die bei der Ausführung des Programms durch einen Computer diesen veranlassen, die Schritte des Verfahrens nach einem der vorhergehenden Ansprüche 1 bis 11 und/oder 13 und/oder 14 auszuführen.
